(19) Europäisches Patentamt / European Patent Office / Office européen des brevets

(11) **EP 3 967 241 B1**

(12) **FASCICULE DE BREVET EUROPÉEN**

(45) Date de publication et mention de la délivrance du brevet:
**25.09.2024 Bulletin 2024/39**

(21) Numéro de dépôt: **21190940.3**

(22) Date de dépôt: **12.08.2021**

(51) Classification Internationale des Brevets (IPC):
**A61B 8/13** *(2006.01)*     **G01N 29/06** *(2006.01)*
**G01S 15/89** *(2006.01)*

(52) Classification Coopérative des Brevets (CPC):
**G01N 29/262; A61B 8/13; G01N 29/032;**
**G01N 29/0654; G01N 29/46; G01S 7/52026;**
**G01S 7/52036; G01S 7/52046; G01S 7/52049;**
**G01S 15/8915; G10K 11/34;** G01N 2291/02466;
G01N 2291/106

(54) **PROCÉDÉ ET SYSTÈME DE CARACTÉRISATION ULTRASONORE D'UN MILIEU**

VERFAHREN UND SYSTEM ZUR CHARAKTERISIERUNG EINES MEDIUMS MITTELS ULTRASCHALL

METHOD AND SYSTEM FOR ULTRASONIC CHARACTERISATION OF A MEDIUM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.09.2020 FR 2009314**

(43) Date de publication de la demande:
**16.03.2022 Bulletin 2022/11**

(73) Titulaires:
• **SUPERSONIC IMAGINE**
  **13290 Aix-en-Provence (FR)**
• **CENTRE NATIONAL DE LA RECHERCHE SCIENTIFIQUE (CNRS)**
  **75016 Paris (FR)**
• **Ecole Supérieure de Physique et de Chimie Industrielles de la Ville de Paris**
  **75005 Paris (FR)**

(72) Inventeurs:
• **LAMBERT, William**
  **94200 IVRY SUR SEINE (FR)**
• **AUBRY, Alexandre**
  **94200 IVRY SUR SEINE (FR)**
• **FINK, Mathias**
  **92190 MEUDON (FR)**
• **FRAPPART, Thomas**
  **13090 AIX EN PROVENCE (FR)**

(74) Mandataire: **Plasseraud IP**
  **104 Rue de Richelieu**
  **CS92104**
  **75080 Paris Cedex 02 (FR)**

(56) Documents cités:
**WO-A1-2020/016250     FR-A1- 2 815 717**

• **WILLIAM LAMBERT ET AL: "Reflection matrix approach for quantitative imaging of scattering media", ARXIV.ORG, CORNELL UNIVERSITY LIBRARY, 201 OLIN LIBRARY CORNELL UNIVERSITY ITHACA, NY 14853, 4 June 2020 (2020-06-04), XP081680768, DOI: 10.1103/PHYSREVX.10.021048**

**Description**

**DOMAINE** TECHNIQUE

La présente description concerne des procédés et systèmes de caractérisation ultrasonore d'un milieu, et s'applique notamment à l'imagerie médicale ou au contrôle non destructif et plus généralement à tous les domaines dans lesquels l'imagerie ultrasonore peut être utilisée.

ETAT DE L'ART

Dans le domaine de l'imagerie acoustique, on cherche à caractériser un milieu totalement ou partiellement inconnu en le sondant de manière active au moyen d'ondes ultrasonores. C'est notamment le principe de l'échographe utilisé en imagerie médicale.

La résolution d'un système d'imagerie acoustique peut être définie comme la capacité à discerner les petits détails d'un objet. En principe, un système d'imagerie acoustique est limité par la diffraction et la résolution théorique est donnée par $\lambda / 2$ (où $\lambda$ est la longueur d'onde du son dans le milieu), ou par l'ouverture angulaire finie du détecteur. En pratique, la résolution est cependant souvent détériorée par les variations de vitesses du son lorsque le milieu de propagation est hétérogène.

En effet, la plupart du temps en imagerie acoustique, le milieu est considéré comme homogène, avec une vitesse du son $c_0$ constante. Or l'hypothèse d'un milieu homogène n'est pas toujours respectée. Par exemple, dans le cas de l'échographie du foie, la sonde est placée entre les côtes du patient. Les ondes acoustiques traversent une succession de couches de graisse et de muscle avant d'atteindre l'organe visé. Les tissus mous possèdent chacun des propriétés mécaniques différentes. La vitesse du son est donc loin d'être homogène, et elle peut varier par exemple entre 1450 mis pour les tissus adipeux et 1600 mis pour le foie. Les variations de vitesse du son entraînent un déphasage différent des ondes suivant les endroits au travers desquels elles se propagent. Il en résulte une aberration du front d'onde acoustique qui conduit à une distorsion de l'image échographique résultante, et donc à une dégradation de sa résolution et de son contraste. Ces aberrations peuvent être telles qu'elles ne permettent pas de reconstruire une image fiable, compromettant les résultats, par exemple lors d'un examen médical.

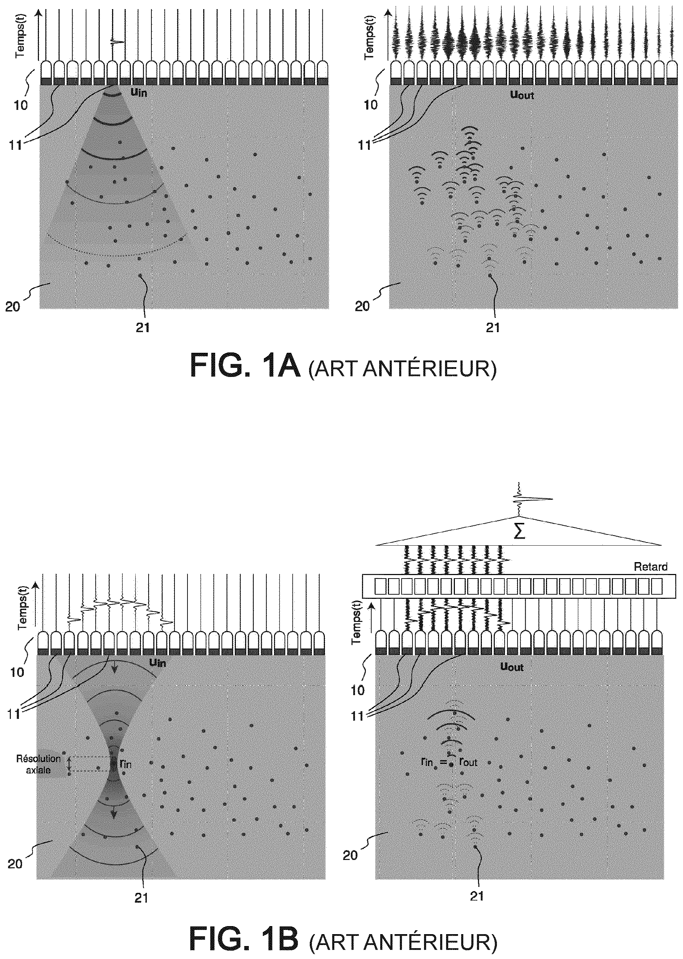

Comme illustré sur les **figures 1A à 1C,** les méthodes d'échographie conventionnelle utilisent un réseau 10 de transducteurs piézo-électriques 11 qui peuvent émettre et/ou recevoir des impulsions ultrasonores de manière indépendante. On repère la position de chacun des transducteurs par le vecteur **u.** Lorsqu'un tel réseau est placé en vis-à-vis d'un milieu 20 que l'on cherche à étudier, celui-ci peut être insonifié et imagé de différentes manières.

Une première manière pour générer une image échographique du milieu à étudier est d'émettre une impulsion ultrasonore depuis un des transducteurs du réseau dont la position est repérée par le vecteur $\mathbf{u_{in}}$ **(figure 1A, schéma de gauche)**. Cela donne lieu à une onde incidente cylindrique (ou sphérique) divergente pour un réseau 1D (ou 2D) de transducteurs. Cette onde est réfléchie par les diffuseurs 21 du milieu 20 et le champ rétrodiffusé est enregistré par chacun des transducteurs 11 en fonction du temps **(figure 1A, schéma de droite).** En répétant cette opération avec chaque transducteur utilisé successivement comme source, on mesure l'ensemble des réponses impulsionnelles R($\mathbf{u_{out}}$, $\mathbf{u_{in}}$, t) entre chaque transducteur, où le vecteur $\mathbf{u_{out}}$ désigne la position du détecteur. Ces réponses forment la matrice de réflexion $\mathbf{R_{uu}}(t)$ exprimée dans la base des transducteurs. L'intérêt d'une telle mesure réside dans le fait que cette matrice contient toute l'information sur le milieu étudié, un ensemble d'opérations matricielles pouvant ensuite lui être appliquées à des fins d'imagerie du milieu, par exemple. En revanche, une telle acquisition suppose que le milieu reste fixe pendant toute la durée des mesures, ce qui peut être très difficile dans le cas d'utilisation in-vivo. De plus, l'énergie émise par un seul élément piézo-électrique est faible, ce qui peut induire un mauvais rapport signal à bruit.

D'autres méthodes sont connues pour générer une image du milieu à étudier dans lesquelles on effectue des émissions focalisées par une technique de formation de voies (ou « beamforming » en langue anglaise). Comme montré par la **figure 1B, schéma de gauche,** ces méthodes consistent à appliquer aux transducteurs 11 un jeu de retards appropriés, basés sur un modèle de vitesse homogène, afin de corriger les temps de parcours des ondes pour que toutes les impulsions arrivent ensemble au point focal visé, de position $\mathbf{r_{in}}.$ L'hypothèse de vitesse du son retenue sera notée $c_0.$ En raison des limites physiques de la diffraction, les ultrasons émis sont concentrés dans une zone délimitée par l'ouverture de la sonde échographique. Afin de construire une image échographique, une étape de focalisation est également effectuée en réception. L'ensemble des échos captés par les éléments 11 du réseau 10 est alors traité pour simuler l'effet d'une lentille en réception, comme décrit sur la **figure 1B, schéma de droite.** Les signaux reçus par les transducteurs sont remis en phase en les décalant temporellement. Ces délais sont identiques à ceux appliqués à l'émission. Dans la phase d'émission, tous les signaux interfèrent au point de position $\mathbf{r_{in}}.$ En réception, les signaux provenant de ce même point $\mathbf{r_{out}} = \mathbf{r_{in}}$ interfèrent électroniquement par sommation des signaux au temps balistique $t = (\|\mathbf{u_{out}} - \mathbf{r_{in}} + I\|\mathbf{u_{in}} - \mathbf{r_{in}}\|)/c_0.$ Cette sommation donne le résultat final de la focalisation en réception. La méthode illustrée sur la **figure 1B,** dite méthode confocale à double focalisation à l'émission et à la réception permet d'imager directement la réflectivité du milieu avec une résolution latérale limitée par la diffraction, une excellente résolution axiale seulement limitée par la durée de l'impulsion initiale et un excellent contraste. Toutefois, cette méthode est chronophage car elle nécessite de focaliser physiquement à l'émission en chacun des points du milieu ou au moins à une profondeur donnée, sur chacune des lignes de l'image.

L'article de W. LAMBERT ET AL: "Reflection matrix approach for quantitative imaging of scattering media", ARXIV.ORG, CORNELL UNIVERSITY LIBRARY, 201 OLIN LIBRARY CORNELL UNIVERSITY ITHACA. NY 14853,4 juin 2020 décrit que la formation de faisceaux focalisés permet la synthèse, en émission et en réception, d'un réseau de transducteurs virtuels qui cartographies l'ensemble du milieu à imager. Les réponses inter-éléments de ce réseau virtuel forment une matrice de réflexion focalisée a partir de laquelle il est possible d'obtenir des cartes spatiales des différentes caractéristiques de l'onde qui se propage.

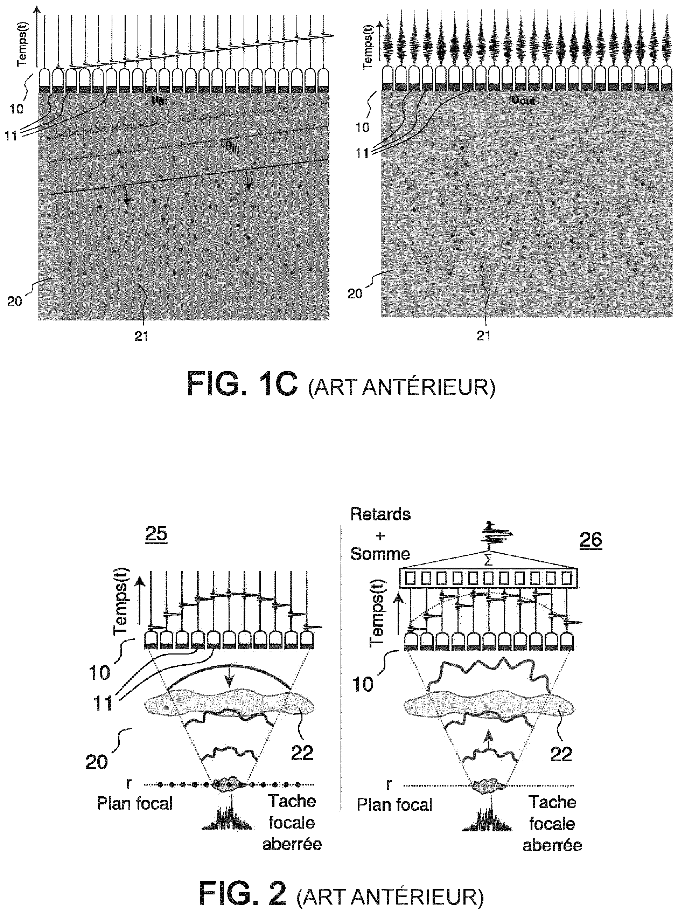

Une autre technique d'imagerie, développée plus récemment, consiste à générer une image du milieu en insonifiant le milieu avec une série d'ondes planes. La **figure 1C** illustre le principe de cette échographie dite en ondes planes, décrite par exemple dans l'article de G. Montaldo et al. « Cohérent plane-wave compounding for very high frame rate ultrasonography and transient elastography" (IEEE Trans. Ultrason., Ferroelect. Freq. Control 56 489-506, 2009). Des retards sont appliqués sur chaque signal à l'émission **(figure 1C, schéma de gauche)** pour la formation d'un front d'onde incliné d'un angle $\theta_{in}$ par rapport au réseau de transducteurs 10. A la réception **(figure 1C, schéma de droite),** le champ rétrodiffusé par le milieu, R($\mathbf{u_{out}}$, 0$_{in}$, t) est mesuré par tous les capteurs de positions $\mathbf{u_{out}}$ pour une série d'ondes planes incidentes dont on fait varier l'angle d'incidence $\theta_{Ln}$. L'ensemble de ces réponses forment une matrice de réflexion $\mathbf{R_{ue}}$(t) définie entre la base de Fourier spatiale (ou base d'ondes planes) en entrée et la base des transducteurs en sortie. Une fois cette matrice enregistrée, les signaux sont décalés temporellement avant d'être sommés de manière cohérente afin de focaliser numériquement les données à l'émission et à la réception pour chaque point de position $\mathbf{r_{in}}$. Le nombre d'acquisitions nécessaires pour former une image échographique est ainsi avantageusement réduit par rapport à l'échographie standard (émissions focalisées), et ceci pour un même niveau de contraste et de résolution de l'image échographique.

La **figure 2** illustre l'influence d'aberrations du milieu sur les méthodes d'imagerie échographique conventionnelle **(figures 1A à 1C).** Ces aberrations apparaissent lorsque la vitesse du son du milieu $c(r)$ ne correspond pas à l'hypothèse d'un milieu homogène avec une vitesse du son constante $c_0$. Les retards initialement déterminés à partir de cette hypothèse et à appliquer sur chacun des transducteurs du réseau à l'émission et à la réception ne sont alors pas optimaux pour l'évaluation d'une image du milieu. Sur la figure 2, une couche aberratrice 22 induit une distorsion du front d'onde incident. A l'émission ou excitation, étape 25, les lois de retard utilisées ne permettent pas de concentrer l'énergie acoustique dans une zone délimitée par les limites de la diffraction, zones usuellement appelée de tache focale. A la réception, en étape 26, les lois de retard utilisées ne permettent pas de sélectionner correctement les signaux ultrasonores provenant du point focal du milieu, et mélangent les signaux provenant d'une tache focale également aberrée. Il en résulte une double aberration dans le processus de construction d'image, ce qui dégrade fortement sa résolution. De nouvelles lois de retard peuvent alors être recalculées afin de compenser l'effet de la couche aberratrice en ajoutant par exemple une loi de retard supplémentaire aux délais généralement utilisés en formation de voies.

Cependant, ces corrections en aberration ne corrigent pas complètement ni ces aberrations ni la dégradation en résolution. I existe un besoin de mieux estimer la qualité de la focalisation dans le milieu.

Le document *"The van Cittert-Zernike theorem in pulse écho measurements "*, (Raoul Mallart and Mathias Fink, J. Acoust. Soc. Am. 90(5), November 1991) a étudié les propriétés statistiques du champ réfléchi par un milieu aléatoire en régime de diffusion simple. I est notamment montré que, pour une onde incidente focalisée, la covariance spatiale du champ réfléchi est proportionnelle, depuis le champ lointain, à la transformée de Fourier de la fonction d'ouverture en transmission. Autrement dit, ce théorème explique que l'étude des propriétés statistiques du champ réfléchi en champ lointain permet de déterminer la qualité de focalisation de l'onde incidente dans le milieu.

Cependant, cette approche ne fournit qu'une estimation globale et moyenne de la résolution d'une image échographique car elle nécessite de moyenner statistiquement les corrélations du champ réfléchi sur un grand nombre de réalisations du désordre, i.e sur un grand nombre de points de focalisation de l'onde incidente. Elle ne permet pas d'obtenir une évaluation précise et locale de la qualité de focalisation en chaque point de l'image. Par ailleurs, cette approche n'est valable qu'en régime de diffusion simple.

Il est donc nécessaire de proposer une méthode palliant chacun des inconvénients précités.

**RESUME**

**La présente description a pour objet, selon un premier aspect, un procédé** selon la revendication 1 de **caractérisation ultrasonore d'un milieu,** pour déterminer localement une direction privilégiée d'une anisotropie des diffuseurs dans le milieu, le procédé comprenant :

- une étape de génération d'une série d'ondes ultrasonores incidentes (US$_{in}$) dans une zone dudit milieu, au moyen d'un réseau (10) de transducteurs (11), ladite série d'ondes ultrasonores incidentes étant une base d'émission (i) ; et
- une étape de génération d'une matrice de réflexion expérimentale $\mathbf{R_{ui}}$(t) définie entre la base d'émission **(i)** en entrée et une base de réception **(u)** en sortie ;
- une étape de détermination d'une matrice de réflexion focalisée $\mathbf{RFoc(r_{in}, r_{out}}, \delta t)$ qui comprend des réponses du milieu entre un transducteur virtuel d'entrée ($TV_{in}$) de position spatiale $\mathbf{r_{in}}$ et un transducteur virtuel de sortie ($TV_{out}$) de position spatiale $\mathbf{r_{out}}$, les réponses du transducteur de virtuel de sortie ($TV_{out}$) étant prises à un instant temporel décalé d'un délai additionnel $\delta t$ par rapport à un instant temporel des réponses du transducteur virtuel d'entrée ($TV_{in}$),
- une étape de détermination d'une image du front d'onde pour le transducteur virtuel d'entrée ($TV_{in}$) et pour un intervalle de délai additionnel, ladite image du front d'onde étant déterminée en fonction de la vitesse du son $c_0$ dans le milieu, et ladite image du front d'onde étant déterminée à partir :

- de la matrice de réflexion focalisée $\mathbf{RFoc(r_{in}, r_{out}}, \delta t)$ et
- d'une relation de propagation balistique du type

$$\delta t(\Delta \mathbf{r_{out}}) = -sign(\Delta z_{out}) \cdot |\Delta \mathbf{r_{out}}|/c_0,$$

qui permet d'extraire des valeurs de la matrice de réflexion focalisée pour construire l'image du front d'onde, et dans laquelle :

$\delta t$ est le délai additionnel,
$|\Delta \mathbf{r_{out}}|$ est le module du vecteur entre le transducteur virtuel d'entrée ($TV_{in}$) et le transducteur virtuel de sortie ($TV_{out}$), avec $\Delta \mathbf{r_{out}} = \mathbf{r_{out}} - \mathbf{r_{in}}$ ,
$\Delta z_{out}$ est la composante selon un axe Z de profondeur du vecteur de position spatiales
$\Delta \mathbf{r_{out}}$. ladite image du front d'onde comprenant une tache focale,

- une étape de détermination d'une direction privilégiée de la tache focale dans l'image du front d'onde par traitement d'image de ladite image du front d'onde, ladite direction privilégiée correspondant à une direction de plus grande dimension pour ladite tache focale.

Grâce à ces dispositions, le procédé permet, de manière avantageuse, de sonder localement le milieu en tout point et en toute direction et avec tout décalage temporel par rapport à un temps balistique de propagation de l'onde ultrasonore dans le milieu.

Ce procédé permet alors de déterminer une image du front d'onde qui représente la propagation vers un point de focalisation, et qui permet d'estimer la qualité de focalisation. Il est alors possible de déduire de cette image du front d'onde une information de direction principale de la tache focale, qui est représentative d'une direction privilégiée ou principale d'anisotropie locale du milieu. Cette information est par exemple très représentative de l'inclinaison locale de fibres musculaires dans le cas de l'imagerie d'un muscle de mammifère. De plus, cette information d'anisotropie peut également être représentative d'une lésion locale.

Dans divers modes de réalisation du procédé selon la présente divulgation, on peut éventuellement avoir recours en outre à l'une et/ou à l'autre des dispositions suivantes.

Selon une variante, le traitement d'image comprend une extraction du contour de la tache focale, par un seuil à un niveau inférieur à la valeur maximum dans l'image du front d'onde, ledit contour servant à déterminer la direction privilégiée de la tache focale.

Selon une variante :

- on transforme l'image du front d'onde $U(\mathbf{r_{in}}, \Delta x_{out}, \Delta z_{out})$ d'un référentiel en coordonnées cartésiennes vers un référentiel en coordonnées polaires $U(\mathbf{r_{in}}, \Delta s_{out}, \Delta \phi_{out})$,
- on somme les valeurs de ladite image du front d'onde du référentiel en coordonnées polaires, sur une pluralité de valeurs d'écarts d'éloignement radial $\Delta s_{out}$ pour obtenir une fonction de sensibilité angulaire $f(\mathbf{r_{in}}, \Delta \phi_{out})$ pour une pluralité de valeurs angulaires $\Delta \phi_{out}$,
- on détermine la valeur angulaire optimale $\Delta \phi^{max}_{out}(\mathbf{r_{in}})$ correspondant au maximum de fonction de sensibilité angulaire, ladite valeur angulaire optimale $\Delta \phi^{max}_{out}(\mathbf{r_{in}})$ correspondant à la direction privilégiée de la tache focale associée au transducteur virtuel d'entrée ($TV_{in}$).

Selon une variante, la direction privilégiée est corrigée par un angle de vue du point de position spatiale $\mathbf{r_{in}}$ du transducteur virtuel d'entrée (TVin), vu depuis le réseau de transducteur (10).
Selon une variante, la direction privilégiée est corrigée par la formule :

$$\gamma_{\text{out}}(\mathbf{r_{in}}) = -2(\Delta\phi\,_{\text{out}}^{max}(\mathbf{r_{in}}) - \hat{\theta}_{\text{out}}(\mathbf{r_{in}}))$$

dans laquelle :

$\Delta\phi\,_{\text{out}}^{max}(\mathbf{r_{in}})$ est la valeur de la direction privilégiée,

$\hat{\theta}_{\text{out}}(\mathbf{r_{in}})$ est la correction de l'angle de vue,

$\gamma_{\text{out}}(\mathbf{r_{in}})$ est la valeur angulaire d'anisotropie .

Selon une variante, la correction de l'angle de vue est :

$$\hat{\theta}_{\text{out}}(\mathbf{r_{in}}) = \frac{1}{\sum_{u_{\text{out}}^-}^{u_{\text{out}}^+} \cos\big(\theta_{\text{out}}(\mathbf{r_{in}})\big)} \sum_{u_{\text{out}}^-}^{u_{\text{out}}^+} \theta_{\text{out}}(\mathbf{r_{in}}) \cos\big(\theta_{\text{out}}(\mathbf{r_{in}})\big)$$

avec

$$\theta_{\text{out}}(\mathbf{r_{in}}) = \text{atan}\left(\frac{u_{out} - x_{in}}{z_{in}}\right)$$

dans laquelle : $u_{\text{out}}^{\pm}(\mathbf{r})$ sont les valeurs maximale et minimale de positions de transducteurs dans le réseau.

Selon une variante :

- on intervertit le rôle du ou des transducteurs virtuels d'entrées et du ou des transducteurs virtuels de sortie pour déterminer une direction privilégiée par rapport à un transducteur virtuel de sortie, et
- on combine la direction privilégiée en référence au transducteur virtuel d'entrée et la direction privilégiée en référence au transducteur virtuel de sortie pour obtenir une direction privilégiée améliorée.

Selon une variante :

- on intervertit le rôle du ou des transducteurs virtuels d'entrées et du ou des transducteurs virtuels de sortie pour déterminer une valeur angulaire d'anisotropie par rapport à un transducteur virtuel de sortie $\gamma_{\text{out}}(\mathbf{r_{out}})$, et
- on combine la valeur angulaire d'anisotropie en référence au transducteur virtuel d'entrée $\gamma_{\text{out}}(\mathbf{r_{in}})$ et la valeur angulaire d'anisotropie en référence au transducteur virtuel de sortie $\gamma_{\text{out}}(\mathbf{r_{out}})$ pour obtenir une valeur angulaire d'anisotropie améliorée.

Selon une variante :

- entre l'étape de détermination d'une image du front d'onde et l'étape de détermination de la direction privilégiée de la tache focale, on effectue une étape d'amélioration de l'image du front d'onde dans laquelle on effectue une combinaison linéaire d'un ensemble d'images du front d'onde correspondant à une zone de cohérence, chaque image du front d'onde étant prise entre un transducteur virtuel d'entrée (TV$_{in}$) choisi de position spatiale $\mathbf{r_{in}}$ différente, et des transducteurs virtuels de sortie (TV$_{out}$) de position spatiale $\mathbf{r_{out}}$ tels que $\mathbf{r_{out}} = \Delta\mathbf{r_{out}} + \mathbf{r_{in}}$, avec $\Delta\mathbf{r_{out}}$ étant prédéfinis et identiques pour toutes les images du front d'onde de l'ensemble, et les transducteurs virtuels d'entrée choisis étant voisins les uns des autres, pour obtenir une image du front d'onde améliorée associé à un transducteur virtuel d'entrée de référence (TV$_{in,ref}$), ce transducteur virtuel d'entrée de référence TV$_{in,ref}$ étant caractéristique des transducteurs virtuels d'entrée de l'ensemble des images du front d'onde utilisées et associées à la zone de cohérence ZC, et
- dans l'étape de détermination d'une direction privilégiée de la tache focale, l'image du front d'onde améliorée est utilisée à la place de l'image du front d'onde, la direction privilégiée de la tache focale est relative à la position spatiale du transducteur virtuel d'entrée de référence TV$_{in,ref}$.

Selon une variante, la combinaison linéaire est déterminée par un calcul de décomposition en valeur singulière

(SVD) de l'ensemble des images du front d'onde pour obtenir un vecteur singulier ($W_1$) associé à la valeur singulière de décomposition en valeur singulière de plus grande valeur absolue, ce vecteur singulier ($W_1$) étant alors l'image du front d'onde améliorée correspondant audit transducteur virtuel d'entrée de référence ($TV_{in,ref}$) et pour les mêmes délais additionnels $\delta$t.

Selon une variante, à l'étape de détermination de la matrice de réflexion focalisée :

le calcul des réponses du transducteur virtuel d'entrée ($TV_{in}$) correspond à un processus de focalisation en entrée à partir de la matrice de réflexion expérimentale $R_{ui}$(t) qui utilise un temps de vol à l'aller des ondes entre la base d'émission et le transducteur virtuel en entrée ($TV_{in}$) pour créer une tache focale d'entrée à la position spatiale $r_{in}$,
le calcul des réponses du transducteur virtuel de sortie ($TV_{out}$) correspond à un processus de focalisation en sortie à partir de la matrice de réflexion expérimentale $R_{ui}$(t) qui utilise un temps de vol de retour des ondes entre le transducteur virtuel de sortie ($TV_{out}$) et les transducteurs de la base de réception u, pour créer une tache focale de sortie à la position spatiale $r_{out}$,
le délai additionnel $\delta$t étant un délai temporel ajouté aux temps de vols à l'aller et de retour lors des processus de focalisation.

Selon une variante, la matrice de réflexion focalisée est calculée par la formule suivante :

$$\mathbf{RFoc}(\mathbf{r_{in}}, \mathbf{r_{out}}, \delta t) = \frac{1}{N_{in}N_{out}} \sum_{i_{in}} \sum_{u_{out}} \mathbf{R_{ui}}\big(\mathbf{u_{out}}, \mathbf{i_{in}}, \tau(\mathbf{r_{in}}, \mathbf{r_{out}}, \mathbf{u_{out}}, \mathbf{i_{in}}, \delta t)\big)$$

dans laquelle

$N_{in}$ est le nombre d'éléments de la base d'émission **(i)**,
$N_{out}$ est le nombre d'éléments de la base de réception (u) en sortie,
$R_{ui}$(t) est la matrice de réflexion expérimentale, dont $R_{ui}(u_{out}, i_{in}, \tau(r_{in}, r_{out}, u_{out}, i_{in}, \delta t))$ est l'élément de la matrice de réflexion expérimentale $R_{ui}$(t) enregistré par le transducteur de position spatiale $u_{out}$ consécutif à l'émission d'indice $i_{in}$ dans la base d'émission et au temps $\tau$,
$\tau$ est un temps qui est la somme du temps de vol à l'aller $\tau_{in}$ de l'onde ultrasonore entre les transducteurs de la base d'émission **(i)** et le transducteur virtuel d'entrée ($TV_{in}$) de position spatiale $r_{in}$, et du temps de vol au retour $r_{out}$ de l'onde ultrasonore entre le transducteur de sortie ($TV_{out}$) de position spatiale $r_{out}$ et les transducteurs de la base de réception u, et du délai additionnel $\delta$t, comme explicité par la formule suivante :

$$\tau(\mathbf{r_{in}}, \mathbf{r_{out}}, \mathbf{u_{out}}, \mathbf{i_{in}}, \delta t) = \tau_{in}(\mathbf{r_{in}}, \mathbf{i_{in}}) + \tau_{out}(\mathbf{r_{out}}, \mathbf{u_{out}}) + \delta t$$

**La présente description concerne, selon un deuxième aspect, un système** selon la revendication 13 de caractérisation ultrasonore d'un milieu pour déterminer localement une direction privilégiée d'une anisotropie des diffuseurs dans le milieu, et configuré pour la mise en oeuvre de procédés de caractérisation ultrasonore tels que décrits précédemment. Le système de caractérisation ultrasonore selon le deuxième aspect comprend :

- un réseau de transducteurs adaptés pour générer une série d'ondes ultrasonores incidentes dans une zone du milieu, et pour enregistrer en fonction du temps les ondes ultrasonores rétrodiffusées par ladite zone ; et
- une unité de calcul associée au réseau de transducteurs et adaptée pour mettre en oeuvre le procédé selon le premier aspect.

**BREVE DESCRIPTION DES FIGURES**

D'autres avantages et caractéristiques de la technique présentée ci-dessus apparaîtront à la lecture de la description détaillée ci-dessous, présentée de manière non limitative à des fins d'illustration, faite par référence aux figures dans lesquelles :

Les **figures 1A à 1C** (déjà décrites) illustrent des mécanismes d'émission/réception connus pour l'imagerie et la quantification ultrasonore ;
La **figure 2** (déjà décrite) illustre l'impact des aberrations en imagerie ultrasonore, selon l'art antéri eur ;
La **figure 3** illustre un exemple de système de caractérisation ultrasonore pour la mise en oeuvre des procédés de caractérisation ultrasonore selon la présente description ;

La **figure 4** illustre les définitions utilisées dans le procédé de caractérisation ultrasonore selon la présente description ;

La **figure 5** montre une image échographique dans laquelle une position associée à un élément échogène est sélectionnée et des images de propagation autour de cette position sont déterminées pour plusieurs délais additionnels ;

La **figure 6** montre une image échographique dans laquelle une position associée à un ensemble de diffuseurs sous résolus et de réflectivité comparable est sélectionné et des images de propagation autour de cette position sont déterminées pour plusieurs délais additionnels ;

La **figure 7** montre une image échographique dans laquelle un ensemble de position associées à des diffuseurs sous résolus et de réflectivité comparable sont sélectionnées et des images de propagation d'onde cohérente résultant d'une combinaison des images de propagation associée à chaque position sélectionnée sont déterminées pour plusieurs délais additionnels ;

La **figure 8A** montre des courbes de variations temporelles de l'intensité du point central d'une image de propagation associé à une position correspondant à des diffuseurs sous résolus et de réflectivité comparable et de l'image de propagation d'onde cohérente ;

La **figure 8B** montre des spectres en fréquences des courbes de la figure 8A ;

La **figure 9A** montre l'amplitude d'image du front d'onde associé à la même position que celle de la figure 5 ;

La **figure 9B** montre la partie réelle de la même image du front d'onde que celle utilisée en figure 9A ;

La **figure 10** montre l'amplitude de plusieurs images du front d'onde associées à la même position que celle sélectionnée pour la figure 5 et obtenues pour 3 vitesses du son supposées, et des courbes d'intensité sur l'axe d'ordonnée $\Delta z$ de ces images du front d'onde ;

La **figure 11** montre des images échographiques et des images de vitesse du son intégrées correspondantes ;

La **figure 12** montre des images échographiques obtenues sans correction d'aberration (image A), avec une correction d'aberration latérale usuelle (image B) et avec une correction d'aberration axiale utilisant des mesures dans des images du front d'onde selon la présente divulgation ;

La **figure 13** montre une image échographique (A) comprenant plusieurs zones avec des fibres musculaires inclinées selon diverses directions, et des images du front d'onde (B, C, D, E) correspondant à ces différentes zones du milieu ;

La **figure 14** montre une courbe de calcul de l'angle d'une direction privilégiée d'inclinaison de fibre musculaire dans le milieu ;

La **figure 15** montre une matrice de directions privilégiées déterminées superposées à une image échographique d'un milieu avec des fibres musculaires ;

La **figure 16** montre une image échographique (A), un agrandissement d'une zone de cette image échographique (B) autour d'un point particulier, le signal temporel local en partie réelle (C) et amplitude (D) estimé pour ce point particulier et l'analyse spectrale de ce signal temporel local du milieu ;

La **figure 17** montre une image échographique (A) et une estimation du spectre moyen en fonction de la profondeur Z pour l'image échographique correspondante de cette figure ;

La **figure 18** montre une image échographique (A) et l'image de corrélation spectrale déterminée pour l'image échographique correspondante de cette figure.

Dans les différents modes de réalisation qui vont être décrits par référence aux figures, des éléments semblables ou identiques portent sauf stipulation contraire les mêmes références.

## DESCRIPTION DETAILLEE

Dans la description détaillée qui suit, seuls certains modes de réalisation sont décrits en détail pour assurer la clarté de l'exposé mais ces exemples ne visent pas à limiter la portée générale des principes ressortant de la présente description.

Les différents modes de réalisation et aspects décrits dans la présente description peuvent être combinés ou simplifiés de multiples manières. En particulier, les étapes des différents procédés peuvent être répétées, interverties, et/ou exécutées en parallèle, sauf précision contraire.

La présente description concerne des procédés et systèmes de caractérisation ultrasonore d'un milieu, et s'applique notamment à l'imagerie médicale de tissus vivants ou non. Le milieu est par exemple un milieu hétérogène que l'on cherche à caractériser pour par exemple identifier et/ou caractériser les hétérogénéités. Eventuellement, ces procédés et systèmes peuvent s'appliquer au contrôle non destructif de produits, tel que des pièces métalliques ou autre. Ces techniques de caractérisations sont ainsi non invasive dans le milieu, qui est alors préservé.

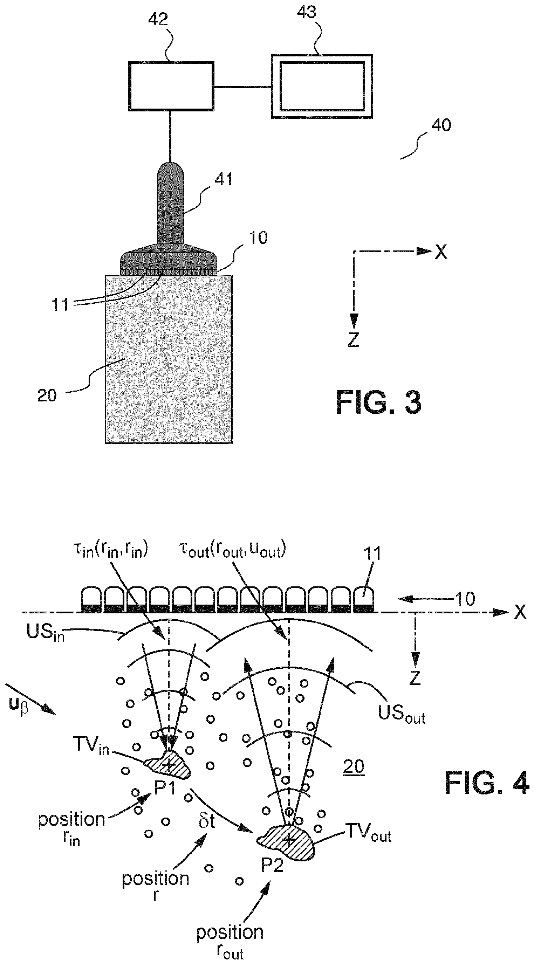

La **figure 3** illustre un exemple d'un système 40 de caractérisation ultrasonore pour la mise en oeuvre des procédés de caractérisation ultrasonore d'un milieu tel qu'un milieu hétérogène 20, selon la présente description. Le système 40 comprend au moins un réseau 10 de transducteurs 11, par exemple un réseau linéaire ou bidimensionnel ou matriciel ; les transducteurs sont par exemple des transducteurs piézoélectriques ultrasonores pouvant se présenter classiquement sous la forme d'une barrette rigide mise en contact directement ou indirectement avec le milieu 20. Le réseau de transducteurs fait par exemple partie d'un dispositif de sondage 41 (usuellement appelé sonde) ; le réseau de transducteurs est connecté à une unité de calcul 42, qui peut elle-même être reliée ou associée à un dispositif d'affichage 43 ; l'unité de calcul émet et enregistre des signaux électriques vers et/ou provenant de chacun des transducteurs 11. Les transducteurs ultrasonores transforment ensuite ces signaux électriques en ondes ultrasonores et inversement. Par "connexion" ou "liaison" entre le dispositif de sondage 41, l'unité de calcul 42 et le dispositif d'affichage 43, on entend tout type de liaison filaire de type électrique ou optique, ou tout type de liaison sans fil utilisant tout protocole tel que le WiFi™, Bluetooth™ ou autres. Ces connexions ou liaisons sont à simple sens ou double sens.

L'unité de calcul 42 est configurée pour la mise en oeuvre d'étapes de calcul ou traitement notamment pour la mise en oeuvre d'étapes de procédés selon la présente description. Par convention, on définit un repère spatial du milieu 20, en prenant un premier axe X et un deuxième axe Z perpendiculaire à celui-ci. Par simplification, le premier axe X correspond à la direction transversale dans laquelle les transducteurs 11 sont alignés pour un réseau linéaire, et le deuxième axe Z correspond à la profondeur du milieu 20 par rapport à ce réseau 10 de transducteurs 11. Cette définition peut être adaptée au contexte et ainsi par exemple étendue à un repère spatial à trois axes dans le cas d'un réseau 10 bidimensionnel.

Dans la **figure 3** comme dans la suite de la description, il est fait référence à un réseau de transducteurs pour l'émission et la réception, étant bien entendu que, dans un cas plus général, plusieurs réseaux de transducteurs pourront être utilisés simultanément. De même, un réseau peut être constitué de un (1) à N transducteurs, de type identique ou de natures différentes. Les transducteurs pourront être à la fois émetteur puis récepteur, ou bien seulement émetteur pour certains et seulement récepteur pour d'autres.

Le réseau de transducteurs sert par exemple à la fois comme émetteur et comme récepteur, ou est constitué de plusieurs sous-réseaux de transducteurs, certains étant dédiés à l'émission, d'autres à la réception des ondes ultrasonores. Par réseau de transducteurs, on entend au moins un transducteur, une suite alignée ou non de transducteurs, ou une matrice de transducteurs.

Lorsque dans la présente description, il est fait référence à des étapes de calcul ou traitement pour la mise en oeuvre notamment d'étapes de procédés, il est entendu que chaque étape de calcul ou traitement peut être mis en oeuvre par logiciel, hardware, firmware, microcode ou toute combinaison appropriée de ces technologies ou technologies avoisinantes. Lorsqu'un logiciel est utilisé, chaque étape de calcul ou traitement peut être mise en oeuvre par des instructions de programme d'ordinateur ou du code qui peut être par exemple interprété, ou exécuté. Ces instructions peuvent être stockées ou transmises vers un support de stockage lisible par un ordinateur (ou unité de calcul) et/ou être exécutées par un ordinateur (ou unité de calcul) afin de mettre en oeuvre ces étapes de calcul ou traitement.

## Analyse d'un point du milieu par matrice de réflexion focalisée

La présente description décrit des procédés et systèmes de caractérisation ultrasonore d'un milieu. Dans les cas pratiques, le milieu est supposé hétérogène. Ces procédés et systèmes sont basés sur des définitions représentées en **figure 4** :
On définit dans le milieu :

- un premier point P1 de position spatiale $r_{in}$ dans le repère spatial du milieu,
- un deuxième point P2 de position spatiale $r_{out}$ dans le repère spatial du milieu.

Ces positions spatiales $r_{in}$ et $r_{out}$ sont notés en gras, pour signifier que ces éléments sont des vecteurs de position, vecteurs pris dans le repère spatial du milieu (X, Z). D'autres représentations et définitions des positions des points sont possibles et accessibles à tout spécialiste du métier des ultrasons.

Ces deux points P1 et P2 sont choisis plutôt à faible distance l'un de l'autre, c'est à dire à quelques millimètres l'un de l'autre, et par exemple à vingt (20) millimètres ou moins, aux fréquences ultrasonores.

Comme représenté en **figure 4,** le procédé de caractérisation ultrasonore mis en oeuvre par l'unité de calcul 42 du système 40 comprend :

- une étape de génération d'une série d'ondes ultrasonores incidentes $US_{in}$ dans une zone dudit milieu, au moyen d'un réseau 10 de transducteurs 11, ladite série d'ondes ultrasonores incidentes étant une base d'émission i ; et
- une étape de génération d'une matrice de réflexion expérimentale $R_{ui}(t)$ définie entre la base d'émission **i** en entrée et une base de réception u en sortie ;
- une étape de détermination d'une matrice de réflexion focalisée $RFoc(r_{in}, r_{out}, \delta t)$ qui comprend des réponses du milieu entre un transducteur virtuel d'entrée $TV_{in}$ de position spatiale $r_{in}$ et un transducteur virtuel de sortie $TV_{out}$

de position spatiale $\mathbf{r_{out}}$, les réponses du transducteur de virtuel de sortie $TV_{out}$ étant prises à un instant temporel décalé d'un délai additionnel $\delta t$ par rapport à un instant temporel des réponses du transducteur virtuel d'entrée $TV_{in}$.

Les réponses de la matrice de réflexion focalisée $\mathbf{RFoc}(\mathbf{r_{in}}, \mathbf{r_{out}}, \delta t)$ correspondent à un champs de pression acoustique calculé en tout point du milieu.

La base d'émission **i** en entrée est par exemple une base d'ondes générées chacune par un seul des transducteurs 11 du réseau 10 ou une base d'ondes planes d'inclinaison angulaire $\theta$ par rapport à l'axe X, comme décrit précédemment dans la description des **figures 1A à 1C**.

La base de réception **u** est par exemple la base des transducteurs 11. Eventuellement, une autre base de réception peut être utilisée en réception.

Ainsi, l'étape de génération des ondes ultrasonores s'entend entre la base d'émission i et la base de réception **u.** Cette étape de génération ultrasonore est donc définie pour tout type d'ondes ultrasonores de type focalisées ou non focalisées, telles que des ondes planes.

Dans l'étape de génération de la matrice, la matrice de réflexion expérimentale $\mathbf{R_{ui}}(t)$ est définie entre la base d'émission i en entrée et une base de réception **u** en sortie. Cette matrice contient l'ensemble des réponses temporelles du milieu, mesurées au temps t par chaque transducteur 11 de coordonnée spatiale $\mathbf{u_{out}}$ et pour chaque émission $\mathbf{i_{in}}$. On comprend que les éléments dénommés avec l'indice "in" font référence à l'émission (i.e. l'entrée) et les éléments dénommés avec l'indice "out" font référence à la réception (i.e. la sortie). Cette matrice expérimentale peut également être enregistré et/ou stockée, par exemple en mémoire de l'unité de calcul, ou sur un tout autres support, amovible ou non, permettant un stockage permanent ou temporaire.

Plus précisément, dans l'étape de détermination de la matrice de réflexion focalisée $\mathbf{RFoc}(\mathbf{r_{in}}, \mathbf{r_{out}}, \delta t)$, on applique :

- un processus de focalisation en entrée à partir de la matrice de réflexion expérimentale $\mathbf{R_{ui}}(t)$ qui utilise un temps de vol à l'aller des ondes entre la base d'émission (i) et le transducteur virtuel en entrée $TV_{in}$ et qui crée une tache focale dite d'entrée autour du premier point P1 de position spatiale $\mathbf{r_{in}}$, ladite tache focale d'entrée correspondant au transducteur virtuel d'entrée $TV_{in}$,
- un processus de focalisation en sortie à partir de la matrice de réflexion expérimentale $\mathbf{R_{ui}}(t)$ qui utilise un temps de vol de retour des ondes entre le transducteur virtuel de sortie ($TV_{out}$) et les transducteurs de la base de réception **(u)** et qui crée une tache focale dite de sortie autour du deuxième point P2 de position spatiale $\mathbf{r_{out}}$, ladite tache focale de sortie correspondant au transducteur virtuel de sortie $TV_{out}$,
- un délai additionnel $\delta t$ qui est un délai temporel ajouté aux temps de vol à l'aller et de retour lors des processus de focalisation.

Ces processus de focalisation en entrée et en sortie forment en fait un processus de focalisation en entrée-sortie, dénommé dans la suite de cette description processus de focalisation.

Autrement dit, dans ce procédé de caractérisation ultrasonore, le transducteur virtuel d'entrée $TV_{in}$ correspond à une "source virtuelle" ultrasonore située à la position spatiale $\mathbf{r_{in}}$ dans le milieu et le transducteur virtuel de sortie $TV_{out}$ correspond à un "capteur virtuel" ultrasonore situé à la position spatiale $\mathbf{r_{out}}$. Cette source et ce capteur virtuels sont spatialement séparées de la différence de leurs positions spatiales $\Delta\mathbf{r} = \mathbf{r_{out}} - \mathbf{r_{in}}$. Ils sont également séparés temporellement du délai additionnel $\delta t$, qui est un délai arbitraire et réglable indépendamment de la distance spatiale $|\Delta\mathbf{r}|$. Ainsi, le procédé est apte à sonder le milieu autour du point P1 et/ou du point P2, de manière spatiale et/ou de manière temporelle, ce qui permet d'obtenir de nouvelles informations dans ces deux dimensions (spatiale et temporelle) sur la propagation des ondes.

Par exemple, un calcul de la matrice de réflexion focalisée $\mathbf{RFoc}(\mathbf{r_{in}}, \mathbf{r_{out}}, \delta t)$ du milieu entre le transducteur virtuel d'entrée $TV_{in}$ et le transducteur virtuel de sortie $TV_{out}$ par lesdits processus de focalisation en entrée et en sortie, est un procédé de formation de voie amélioré, qui peut être exprimé par la formule simplifiée suivante :

$$\mathbf{RFoc}(\mathbf{r_{in}}, \mathbf{r_{out}}, \delta t) = \frac{1}{N_{in}N_{out}} \sum_{i_{in}} \sum_{u_{out}} \mathbf{R_{ui}}\big(\mathbf{u_{out}}, \mathbf{i_{in}}, \tau(\mathbf{r_{in}}, \mathbf{r_{out}}, \mathbf{u_{out}}, \mathbf{i_{in}}, \delta t)\big)$$

$$(\text{Equ. 1})$$

dans laquelle

$N_{in}$ est le nombre d'éléments de la base d'émission **i,**

$N_{out}$ est le nombre d'éléments de la base de réception **u** en sortie,

$R_{ui}(t)$ est la matrice de réflexion expérimentale, dont $R_{ui}(u_{out}, i_{in}, \tau(r_{in}, r_{out}, u_{out}, i_{in}, \delta t))$ est l'élément de la matrice de réflexion expérimentale $R_{ui}(t)$ enregistré par le transducteur $u_{out}$ consécutif à l'émission $i_{in}$ au temps $\tau$.

Le temps $\tau$ est la somme du temps de vol à l'aller $\tau_{in}$ de l'onde ultrasonore entre les transducteurs de la base d'émission i et le transducteur virtuel d'entrée $TV_{in}$ de position spatiale $r_{in}$ (premier point P1), du temps de vol au retour $r_{out}$ de l'onde ultrasonore entre le transducteur virtuel de sortie $TV_{out}$ de position spatiale $r_{out}$ (deuxième point P2) et les transducteurs de la base de réception **u,** et du délai additionnel $\delta t$, comme explicité par la formule suivante :

$$\tau(r_{in}, r_{out}, u_{out}, i_{in}, \delta t) = \tau_{in}(r_{in}, i_{in}) + \tau_{out}(r_{out}, u_{out}) + \delta t$$

$$(\text{Equ. 2})$$

Les temps de vols $\tau_{in}$ et $\tau_{out}$ sont calculés à partir d'un modèle de vitesse du son. L'hypothèse la plus simple consiste à faire l'hypothèse d'un milieu homogène avec une vitesse du son constante $c_0$. Dans ce cas, les temps de vols sont directement obtenus à partir des distances entre les transducteurs de la sonde et les transducteurs virtuels.

Le nombre d'éléments de la base d'émission $N_{in}$ est par exemple supérieur ou égal à un (1), et avantageusement supérieur ou égal à deux (2). Le nombre d'éléments de la base de réception $N_{out}$ est par exemple supérieur ou égal à deux (2).

Cette formule de formation de voie améliorée est donc une double somme des réponses temporelles enregistrées dans la matrice de réflexion expérimentale $R_{ui}$, une première somme selon la base d'émission **i** traduisant une focalisation à l'émission et une seconde somme selon la base de réception **u** liée à une focalisation en réception, ce calcul étant effectué pour les coordonnées spatiales des deux points P1 et P2 (de positions spatiales $r_{in}$, $r_{out}$). Le résultat de cette formule de formation de voie améliorée est donc un signal temporel pour ces deux coordonnées spatiales ($r_{in}$, $r_{out}$), mais qui est également fonction du délai additionnel $\delta t$ entre l'entrée et la sortie, ce délai additionnel étant réglé de manière arbitraire.

Une telle formulation de formation de voie peut aussi être complétée par des termes de pondération en entrée et sortie, souvent appelés apodisation en réception et/ou en émission. Toute la suite des formules de formation de voie peuvent être ainsi être complétés avec ces pondérations par un technicien du domaine.

La matrice de réflexion expérimentale $R_{ui}(t)$ enregistrée peut être une matrice « réelle », c'est-à-dire composée de coefficients réels dans le domaine temporel, les signaux électriques enregistrés par chacun des transducteurs étant des nombres réels. En variante, cette matrice peut être une matrice « complexe », c'est-à-dire composée de valeurs complexes, par exemple dans le cas d'une démodulation pour une formation de voies en phase et en quadrature (connu en langue anglaise sous la dénomination « beamforming IQ »).

On obtient ainsi une matrice de réflexion focalisée $RFoc(r_{in}, r_{out}, \delta t)$ qui contient des signaux temporels. Cette matrice de réflexion focalisée est à cinq (5) dimensions dans le cas d'une sonde linéaire ; deux espaces pour les positions spatiales $r_{in}$ et $r_{out}$, ainsi que le délai additionnel $\delta t$, ce qui est très différent et bien plus riche en informations que dans des matrices de réflexion focalisées de l'art antérieur.

Dans cette analyse, grâce au délai additionnel $\delta t$, les transducteurs virtuels d'entrée $TV_{in}$ et de sortie $TV_{out}$ ne sont pas définis au même instant temporel ce qui permet de faire ressortir virtuellement la propagation de l'onde ultrasonore entre le premier point P1 du transducteur virtuel d'entrée $TV_{in}$ et le deuxième point P2 du transducteur virtuel de sortie $TV_{out}$. Ce délai additionnel $\delta t$ peut être positif ou négatif, ce qui permet de sonder la focalisation de l'onde ultrasonore au deuxième point P2 respectivement avant et après un instant temporel de référence des trajets de l'onde ultrasonore dans le milieu.

Cet instant temporel de référence est dénommé le <u>temps balistique</u> $t_b$. Ce temps balistique est le temps aller-retour de l'onde ultrasonore entre les transducteurs de la base d'émission **i** vers le transducteur virtuel d'entrée $TV_{in}$, puis entre le transducteur virtuel de sortie $TV_{out}$, et les transducteurs de la base de réception **u.**

Ce temps balistique $t_b$ est défini par la formule suivante :

$$t_b = (\|u_{out} - r_{out}\| + \|u_{in} - r_{in}\|)/c_0$$

$$(\text{Equ. 3})$$

dans lequel :

$c_0$ est la vitesse du son supposée du milieu (vitesse de propagation des ondes ultrasonores).

Grâce à ces dispositions, le procédé permet de sonder très localement le milieu au deuxième point P2 par rapport au premier point P1, avec un délai additionnel $\delta t$ entre les signaux issus de ces deux points. Cette information locale est entièrement contenue dans les valeurs de la réponse temporelle calculée à partir de la matrice de réflexion focalisée, $\mathbf{RFoc}(\mathbf{r_{in}}, \mathbf{r_{out}}, \delta t)$ du milieu et que l'on peut exploiter à postériori (et sans nouvelles émission et/ou acquisition) pour caractériser chaque point du milieu.

Ainsi, il est possible de déduire de cette réponse temporelle après formation de voie, une estimation de la réflectivité du milieu en considérant la valeur absolue des signaux confocaux caractérisés par des positions spatiales égales en entrée et sortie $\mathbf{r_{in}} = \mathbf{r_{out}}$ et au délai additionnel nul $\delta t = 0$ (i.e. au temps balistique sans ce délai additionnel). Cette estimation de la réflectivité du milieu est la valeur d'un pixel d'une image échographique du milieu. Ainsi, pour construire une image échographique, on peut balayer ou choisir un ensemble de positions spatiales $\mathbf{r} = \mathbf{r_{in}} = \mathbf{r_{out}}$ qui correspond à un ensemble de positions de pixels dans l'image échographique.

L'image échographique $I^{(0)}(\mathbf{r})$ peut ensuite être construite à partir de la matrice de réflexion focalisée $\mathbf{RFoc}(\mathbf{r_{in}}, \mathbf{r_{out}}, \delta t)$ en prenant $\mathbf{r} = \mathbf{r_{in}} = \mathbf{r_{out}}$, et $\delta t = 0$, c'est-à-dire :

$$I^{(0)}(\mathbf{r}) = \mathbf{RFoc}(\mathbf{r_{in}}, \mathbf{r_{out}} = \mathbf{r_{in}}, \delta t = 0)$$

$$(\text{Equ. 4})$$

### Images de propagation autour d'un point du milieu

Le procédé de caractérisation ultrasonore mis en oeuvre par l'unité de calcul 42 du système 40 peut alors être complété en construisant une ou plusieurs images de propagation à partir de la matrice de réflexion focalisée $\mathbf{RFoc}(\mathbf{r_{in}}, \mathbf{r_{out}}, \delta t)$, cette ou ces images de propagation étant déterminées pour une ou plusieurs de valeurs du délai additionnel $\delta t$, pour un transducteur virtuel d'entrée $TV_{in}$ (premier points P1) et pour une pluralité de transducteurs virtuels de sortie $TV_{out}$ (deuxièmes points P2), les transducteurs virtuels de sortie $TV_{out}$ étant situés à des positions spatiales $\mathbf{r_{out}}$ autour du transducteur virtuel d'entrée $TV_{in}$ de position spatiale $\mathbf{r_{in}}$.

Dans le cas d'une seule image de propagation, cette image de propagation est déterminée à partir de la matrice de réflexion focalisée $\mathbf{RFoc}(\mathbf{r_{in}}, \mathbf{r_{out}}, \delta t)$ pour un unique délai additionnel $\delta t$ prédéterminé.

Cette image de propagation représente la manière dont une onde ultrasonore se propage entre les transducteurs virtuels, et par exemple à proximité du transducteur virtuel d'entrée, et à un instant temporel égal au délai additionnel (instant temporel pris en relatif par rapport au temps balistique).

Le système 40 est alors optionnellement apte à afficher une ou plusieurs images de propagation sur le dispositif d'affichage 43.

L'unité de calcul 42 peut également calculer une série d'images de propagation pour plusieurs délais additionnels temporellement successifs, par exemple pour construire un film de propagation de l'onde ultrasonore autour du transducteur virtuel d'entrée $TV_{in}$ (premier point P1). Ce film de propagation peut éventuellement être affiché sur le dispositif d'affichage 43 ou sur tout autre média.

Les délais additionnels temporellement successifs pris pour construire ce film de propagation sont pris dans notre exemple dans un intervalle de délai additionnel.

Par exemple, l'intervalle de délai additionnel peut prendre la forme d'une plage temporelle adaptée pour aller du transducteur virtuel d'entré $TV_{in}$ de position spatiale $\mathbf{r_{in}}$ à l'ensemble des transducteurs virtuels de sortie $TV_{out}$ de position spatiale $\mathbf{r_{out}}$. Cet intervalle de délai additionnel est alors par exemple noté $[-\delta t_{min}, +\delta t_{max}]$, avec $\delta t_{min} = z_{out}{}^{max} - z_{in} / c_0$ et $\delta t_{max} = z_{out}{}^{min} - z_{in} / c_0$, où $z_{in}$ et $z_{out}$ sont respectivement les profondeurs dans la direction positive du deuxième axe Z du transducteur virtuel d'entrée $TV_{in}$ de position spatiale $\mathbf{r_{in}}$ et du transducteur virtuel de sortie $TV_{out}$ de position spatiale $\mathbf{r_{out}}$.

Par exemple, l'intervalle de délai additionnel peut être symétrique autour de la valeur nulle ($\delta t = 0$) et d'amplitude $\delta t_{max}$, cet intervalle de délai additionnel étant noté $[-\delta t_{max}, +\delta t_{max}]$. Par exemple, il peut être défini par $\delta t_{max} = \max(|\Delta \mathbf{r}|) / c_0$ pour les transducteurs de sortie $TV_{out}$ utilisés pour l'image de propagation.

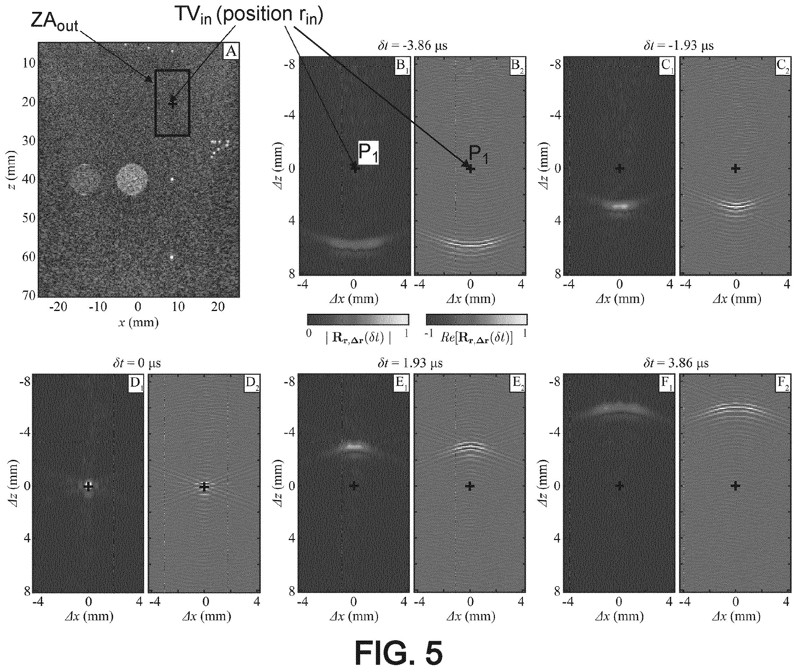

L'image de **référence A** de la **figure 5** montre une image échographique d'un phantom ou milieu exemple d'étude, qui comprend des hétérogénéités prédéterminées de plusieurs types. Dans ce milieu, on considère une zone d'analyse $ZA_{out}$ rectangulaire (composée de deuxièmes points P2 de transducteurs virtuels de sortie $TV_{out}$) qui est balayée par calcul pour construire une ou plusieurs images de propagation autour du premier point P1 de transducteur virtuel d'entrée $TV_{in}$ de position spatiale $\mathbf{r_{in}}$, ici situé au milieu de la zone d'analyse $ZA_{out}$. La zone d'analyse peut être positionnée à toute position indépendamment de la position du transducteur virtuel d'entrée. Cependant, il est particulièrement intéressant que la zone d'analyse entoure le transducteur virtuel d'entrée.

Le transducteur virtuel d'entrée $TV_{in}$ (premier point P1) est, dans cette image de référence A, localisé sur ou à proximité d'un élément réfléchissant (cible échogène) du milieu.

Les images de **références B à F** de la **figure 5** sont des images de propagation de la zone d'analyse $ZA_{out}$ de l'image A de la figure 5, pour cinq (5) valeurs de délais additionnels $\delta t$. Ces délais additionnels sont de -3,86 $\mu$s,

-1,93 $\mu$s, 0 $\mu$s, 1,93 $\mu$s, 3,86 $\mu$s dans notre exemple illustratif. Chaque image de propagation est composée :

- d'une première image d'indice 1 (par exemple $B_1$) correspondant à l'amplitude des valeurs de la matrice de réflexion focalisée pour un ensemble de points de la zone d'analyse $ZA_{out}$, et
- d'une deuxième image d'indice 2 (par exemple $B_2$) correspondant à la partie réelle des valeurs de la matrice de réflexion focalisée pour le même ensemble de points de la zone d'analyse $ZA_{out}$.

Dans ces images, le niveau de l'amplitude ou le niveau de la partie réelle est représenté par un niveau de gris dont l'échelle figure sur les images $B_1$ et $B_2$ de la figure 5. Les points ou pixels de ces images de propagation ont pour position spatiale $\Delta\mathbf{r} = \mathbf{r_{out}} - \mathbf{r_{in}}$, c'est à dire la position relative des transducteurs virtuels de sortie $TV_{out}$ de position spatiale $\mathbf{r_{out}}$ par rapport à la position $\mathbf{r_{in}}$ du transducteur virtuel d'entrée $TV_{in}$. Sur la figure illustrant cet exemple, les coordonnées sont notées $\Delta x$ en abscisse, et $\Delta z$ en ordonnée sur ces images.

Ces images de propagation illustrent les explications données précédemment à propos de la matrice de réflexion focalisée calculée avec un délai additionnel $\delta t$: Elles permettent de visualiser la propagation d'une onde cohérente. Notamment, pour les délais additionnels négatifs allant vers zéro, cette onde cohérente est convergente vers le premier point P1 du transducteur virtuel de d'entrée $TV_{in}$, et elle est idéalement concentrée et focalisée en une tache focale délimitée par les limites de diffraction pour le délai additionnel nul ($\delta t = 0$). Cette onde cohérente est ensuite divergente pour des délais additionnels positifs et en augmentation.

Cette onde cohérente résulte d'un processus de retournement temporel numérique des échos provenant de la source virtuelle localisée au transducteur virtuel d'entrée de position spatiale $\mathbf{r_{in}}$ et mesurés par les transducteurs de la sonde. En effectuant la formation de voie en réception pour un ensemble de position spatiales $\mathbf{r_{out}}$ autour du transducteur virtuel d'entrée de position spatiale $\mathbf{r_{in}}$, et aux divers temps additionnels $\delta t$, on illustre la focalisation en réception en dehors de la position confocale (i.e. $\mathbf{r_{in}} = \mathbf{r_{out}}$).

Comme ces images de propagation sont obtenues pour un premier point P1 du transducteur virtuel d'entrée $TV_{in}$ localisé sur ou à proximité d'un élément réfléchissant (cible échogène) du milieu, l'onde cohérente est aisément identifiable sur ces images de propagation et présente un bon rapport signal sur bruit en comparaison des signaux avoisinants.

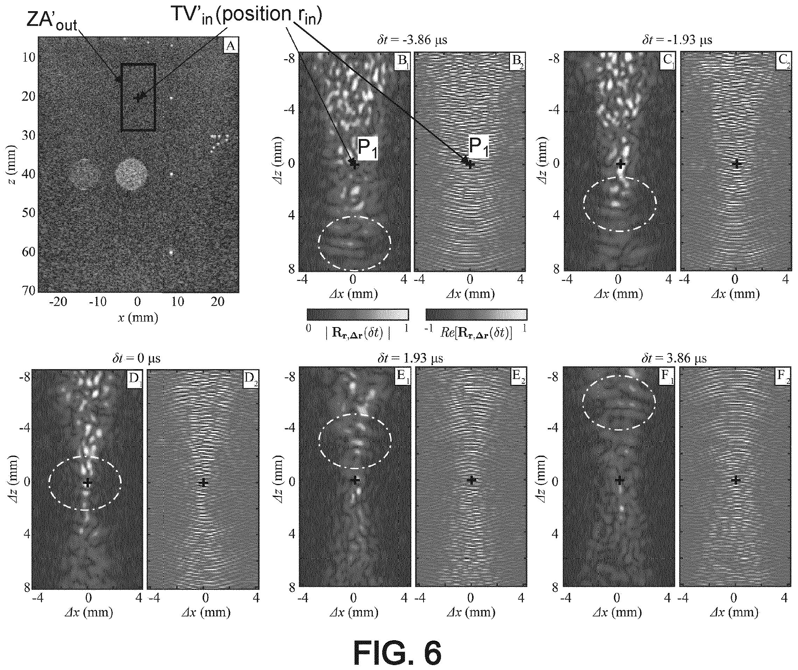

L'image de **référence A** de la **figure 6** montre la même image échographique que celle de la figure 5, pour laquelle on considère une autre zone d'analyse $ZA'_{out}$ rectangulaire (de même dimension dans cet exemple) qui est balayée par calcul pour construire des images de propagation autour d'un autre premier point P1' de transducteur virtuel d'entrée $TV'_{in}$ de position spatiale $\mathbf{r_{in}}$.

Cet autre premier point P1' de transducteur virtuel d'entrée $TV'_{in}$ est ici associé à une cellule de résolution contenant un ensemble de diffuseurs sous-résolus, arrangés aléatoirement et ayant une réflectivité comparable. A l'échelle de la longueur d'onde, un tel milieu est appelé "speckle ultrasonore" et est caractérisé par une réflectivité aléatoire résultant des interactions destructives et constructives entre chacun des diffuseurs sous résolus, responsable de l'effet granulaire de l'image échographique B-mode.

Les images de **référence B à F** de la **figures 6** sont des images de propagations de cette autre zone d'analyse $ZA'_{out}$ de l'image A de la figure 6, pour les mêmes 5 valeurs de délais additionnels $\delta t$ que pour les images B à F de la figures 5.

Les amplitudes et parties réelles des valeurs de la matrice de réflexion focalisée pour un ensemble de deuxièmes points de cette autre zone d'analyse $ZA'_{out}$ sont ici représentées de la même façon.

Ces images de propagation pour un diffuseur montrent aussi une onde ultrasonore cohérente (entourée avec des lignes en trait mixte) qui converge, se concentre au premier point P1' du transducteur virtuel d'entrée $TV'_{in}$ , puis qui diverge. Cependant, il est plus difficile de discerner cette onde cohérente en raison des échos générés par les diffuseurs situés en amont ou en aval du plan focal, lesquels présentent une réflectivité comparable avec celle de la source virtuelle étudiée.

En outre et réciproquement à la précédente définition des images de propagation, il est aussi possible de construire une ou plusieurs images de propagation entre une pluralité de transducteurs virtuels d'entrée $TV_{in}$ (premiers points P1) et un transducteur virtuel de sortie $TV_{out}$ (deuxième points P2). Ainsi, la ou les images de propagations sont construites à partir de la matrice de réflexion focalisée $\mathbf{RFoc(r_{in}, r_{out}, \delta t)}$, cette ou ces images de propagation étant déterminées pour une ou plusieurs de valeurs du délai additionnel $\delta t$, pour un transducteur virtuel de sortie $TV_{out}$ (deuxième point P2) et pour une pluralité de transducteurs virtuels d'entrée $TV_{in}$ (premiers points P1), les transducteurs virtuels d'entrée $TV_{in}$ étant situés à des positions spatiales $\mathbf{r_{in}}$ autour du transducteur virtuel de sortie $TV_{out}$ de position spatiale $\mathbf{r_{out}}$.

Les définitions des images de propagations par rapport aux transducteurs d'entrée et de sortie s'en trouvent de fait inversées. Du fait de la réciprocité de la propagation des ondes, les images produites sont très similaires et les divers calculs et déterminations effectuées à partir de ces images de propagation et explicités ci-après peuvent être effectués de manière similaire. Par esprit de simplicité, la présente description détaillée n'explicitera que le premier sens entre un transducteur d'entrée et une pluralité de transducteurs virtuels de sortie. Mais il sera compris que, dans chacune des définitions figurant dans ce document, il est possible d'intervertir les éléments d'indice "out" et d'indice "in", et les dénominations "entrée" et "sortie".

De plus, il est également possible d'utiliser les deux types d'images de propagation (selon le premier et deuxième sens), et de les combiner ou d'effectuer une moyenne de ces deux images de propagation pour obtenir une image de propagation moyenne plus représentative et plus contrastée de la propagation des ondes dans le milieu. Il est aussi possible de combiner des résultats issus ou déterminés à partir de ces deux types d'images pour obtenir un résultat souvent plus précis.

La matrice de réflexion focalisée $RFoc(\mathbf{r_{in}}, \mathbf{r_{out}}, \delta t)$ telle que définie précédemment utilise les positions spatiales $\mathbf{r_{in}}$, $\mathbf{r_{out}}$ des transducteurs virtuels d'entrée $TV_{in}$ et de sortie $TV_{out}$. Ces positions spatiales sont des positions absolues dans un référentiel de l'espace. Il est cependant possible d'utiliser également une seule position spatiale absolue et une position spatiale relative par rapport à cette position spatiale absolue. Par exemple, on peut prendre la position spatiale absolue $\mathbf{r_{in}}$ du transducteur virtuel d'entrée et la position spatiale relative $\Delta\mathbf{r_{out}}$ du transducteur virtuel de sortie, avec $\Delta\mathbf{r_{out}} = \mathbf{r_{out}} - \mathbf{r_{in}}$. Réciproquement, on peut prendre la position spatiale absolue $\mathbf{r_{out}}$ du transducteur virtuel de sortie et la position spatiale relative $\Delta\mathbf{r_{in}}$ du transducteur virtuel d'entrée, avec $\Delta\mathbf{r_{in}} = \mathbf{r_{in}} - \mathbf{r_{out}}$. Chacun des calculs et/ou déterminations de la présente description peut être effectué à l'aide de l'une ou l'autre des précédentes définitions, ou de toute autre définition similaire et/ou équivalente.

### Extraction de l'onde cohérente

Le procédé de caractérisation ultrasonore mis en oeuvre par l'unité de calcul 42 du système 40 peut être complété en appliquant une étape de combinaison dans laquelle on effectue une combinaison linéaire d'un ensemble de films de propagation, chaque film de propagation de l'ensemble étant pris entre un transducteur virtuel d'entrée $TV_{in}$ choisi de position spatiale $\mathbf{r_{in}}$ différente, et des transducteurs virtuels de sortie $TV_{out}$ de position spatiale $\mathbf{r_{out}}$ tels que $\mathbf{r_{out}} = \Delta\mathbf{r_{out}} + \mathbf{r_{in}}$, avec $\Delta\mathbf{r_{out}}$ étant prédéfinis et identiques pour tous les films de propagation de l'ensemble, et les transducteurs virtuels d'entrée choisis étant voisins les uns des autres.

Autrement dit, on sélectionne un ensemble de positions spatiales voisines de transducteurs virtuels d'entrée $TV_{in}$ choisis, cet ensemble de positions spatiales formant une zone d'intérêt pour corrélation, dite plus simplement zone spatiale de corrélation ZC, et permettant de corréler les films de propagation de ces transducteurs virtuels d'entrée. Cette zone spatiale de corrélation est par exemple une zone rectangulaire autour d'un point de référence. Elle peut également être l'image dans sa globalité ou toute zone de forme symétrique ou non. Les positions spatiales voisines sont par exemple des positions spatiales proches les unes des autres.

Par cette combinaison d'un ensemble de plusieurs films de propagation, on obtient alors un film de propagation d'onde cohérente amélioré par exemple en termes de cohérence et de contraste. Les images de ce nouveau film de propagation dit film de propagation d'onde cohérente est obtenu pour les mêmes délais additionnels $\delta t$, et pour les mêmes positions relatives $\Delta\mathbf{r_{out}}$.

Ce nouveau film de propagation d'onde cohérente peut alors être associé à un transducteur virtuel d'entrée de référence $TV_{in,ref}$ de position spatiale $\mathbf{r_{in,ref}}$ qui représente les transducteurs virtuels d'entrée choisis de l'ensemble des films de propagation (les transducteurs virtuels d'entrée de la zone spatiale de corrélation).

Selon un premier exemple, le transducteur virtuel d'entrée de référence $TV_{in,ref}$ est un transducteur virtuel d'entrée de position spatiale correspondant à la moyenne des positions spatiales des transducteurs virtuels d'entrée choisis Ainsi, dans cette précédente variante, on peut exprimer la position spatiale du transducteur virtuel d'entrée de référence par :

$$\mathbf{r_{in,ref}} = \frac{1}{N_{\overline{in}}} \sum_{\overline{\mathbf{r_{in}}}} \overline{\mathbf{r_{in}}}$$

(Equ. 5)

$\overline{\mathbf{r_{in}}}$ étant les transducteurs virtuels d'entrée choisis,

$N_{\overline{in}}$ étant le nombre de transducteurs virtuels d'entrée choisis, composant la zone spatiale de corrélation.

Selon un autre exemple, le transducteur virtuel d'entrée de référence $TV_{in,ref}$ est un transducteur virtuel d'entrée de position spatiale correspondant une moyenne pondérée des positions spatiales des transducteurs virtuels d'entrée choisis, ladite pondération étant par exemple basée sur la valeur de réflectivité de chaque point des transducteurs virtuels d'entrée choisis. Ainsi, dans cette variante, on peut exprimer la position spatiale du transducteur virtuel d'entrée

de référence par :

$$\mathbf{r_{in,ref}} = \frac{\sum_{\overline{\mathbf{r_{in}}}} \overline{\mathbf{r_{in}}} \cdot |RFoc(\overline{\mathbf{r_{in}}} = \overline{\mathbf{r_{out}}}, \delta t = 0)|}{\sum_{\overline{\mathbf{r_{in}}}} |RFoc(\overline{\mathbf{r_{in}}} = \overline{\mathbf{r_{out}}}, \delta t = 0)|}$$

(Equ. 6)

Par exemple, cette combinaison linéaire est déterminée ou réalisée par une décomposition en valeur singulières, notée SVD durant laquelle on effectue un calcul de décomposition en valeurs singulières de l'ensemble de films de propagation pour obtenir un vecteur singulier $\mathbf{V_1}$ associé à la valeur singulière de plus grande valeur absolue, ce vecteur singulier $\mathbf{V_1}$ étant alors le film de propagation d'onde cohérente associé audit transducteur virtuel d'entrée de référence TV$_{in,ref}$ et pour les mêmes délais additionnels $\delta t$.

La pluralité des films de propagation de l'ensemble est ici traitée par décomposition en valeurs singulières pour combiner plusieurs films, c'est-à-dire plusieurs mesures ou expériences du désordre acoustique d'une région voisine d'un transducteur virtuel d'entrée, ce qui permet d'améliorer le contraste du film de propagation, et ainsi avantageusement améliorer son exploitation.

Pour effectuer ce calcul de décomposition en valeurs singulières (en particulier parce que les outils actuels de décomposition en valeur singulières usuels fonctionnent sur des matrices à deux dimensions), il est possible de construire une matrice de réflexion focalisée concaténée RFoc' dans laquelle les lignes de cette matrice de réflexion focalisée concaténée RFoc' sont les indices du transducteur virtuel d'entrée TV$_{in}$ choisis de position spatiale $\mathbf{r_{in}}$, et les colonnes de cette matrice de réflexion focalisée concaténée RFoc' sont les films de propagation concaténés $\{\Delta\mathbf{r_{out}}, \delta t\}$ (ensemble d'images) pour chaque transducteur virtuel d'entrée TV$_{in}$ choisi, ces films de propagation étant pris pour une même succession temporelle de délais additionnels $\delta t$. Cette matrice de réflexion focalisée concaténée est ainsi la matrice de réflexion focalisée RFoc recentrée sur le point de focalisation en entrée $\mathbf{r_{in}}$.

Par exemple, on note cette matrice de réflexion focalisée concaténée RFoc' par :
RFoc' = [$RFoc(\mathbf{r_{in}}, \{\Delta\mathbf{r_{out}}, \delta t\})$] = [$RFoc(\mathbf{r_{in}}, \{\mathbf{r_{in}} + \Delta\mathbf{r_{out}}, \delta t\})$]

Cette étape de décomposition en valeurs singulières SVD fourni alors un vecteur singulier $\mathbf{V_1}$ qui maximise les corrélations entre chacune des sources des transducteurs virtuels d'entrée TV$_{in}$ choisis. Le vecteur singulier $\mathbf{V_1}$ est associé à la valeur singulière de décomposition en valeur singulière de plus grande valeur absolue. Le vecteur singulier $\mathbf{V_1}$ est alors le film de propagation d'onde cohérente associé à un transducteur virtuel d'entrée de référence TV$_{in,ref}$ et pour les mêmes délais additionnels $\delta t$.

L'utilisation de la décomposition en valeurs singulières SVD permet donc de combiner plusieurs films de propagation d'onde tout en s'affranchissant de la réflectivité aléatoire introduite par le régime de type speckle. L'onde cohérente étant un élément commun à chacun des films de propagation, celle-ci émerge lors du processus de combinaison, tandis que les contributions des diffuseurs situés en dehors de chaque transducteur virtuel d'entrée TV$_{in}$ sont gommés par interférence destructive. Cela revient à effectuer un filtrage des films de propagation pour en extraire l'onde cohérente.

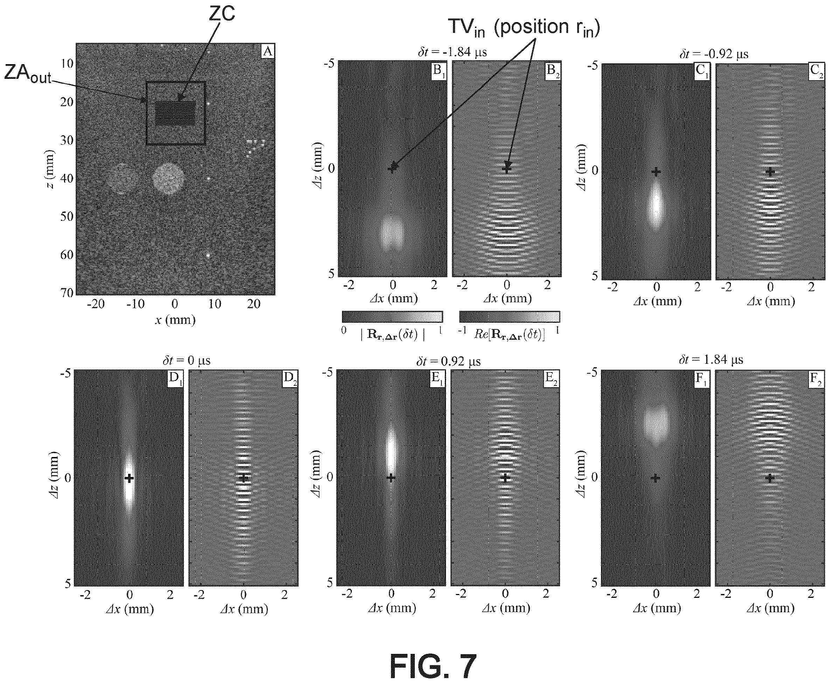

L'image de **référence** A de la **figure** 7 présente la même image échographique que celles de la figure 5 et 6. On considère dans cette figure un exemple de zone d'analyse ZA$_{out}$ associé à un transducteur virtuel d'entrée choisi parmi l'ensemble des transducteurs virtuels d'entrée TV$_{in}$ choisis, ces transducteurs virtuels choisis étant représentés sur cette image A par une grille de points de forme rectangulaire. Les points de cette grille représentent l'ensemble des transducteurs virtuels d'entrée TV$_{in}$ choisis, dite zone de cohérence ZC (i.e. les transducteurs virtuels d'entrée choisis voisins) pour effectuer la combinaison cohérente des films de propagation.

Les images de **références B à F** de la **figure 7** sont des images de propagation d'onde cohérente de la zone d'analyse ZA$_{out}$ de l'image A de la figure 7 pour plusieurs valeurs de délai additionnels $\delta t$ qui représentent le premier vecteur singulier $\mathbf{V_1}$. La même représentation de première image d'amplitude et de deuxième image de partie réelle que celle présentée pour les figures précédentes est utilisée dans cet exemple.

Les images de la figure 7 montrent que l'on peut extraire la partie cohérente de l'onde ultrasonore également pour un ensemble de premier point P1 (transducteur virtuel d'entrée TV$_{in}$) situés dans le speckle. En effet, sur ces images, on observe une unique onde cohérente qui se déplace du bas vers le haut en se concentrant au niveau de la position du transducteur virtuel d'entrée TV$_{in}$, alors que les images de propagations non traitées par le processus de décomposition en valeur singulière SVD de cette expérience ressembleraient à celles présentées en images de référence B à F de la figure 6.

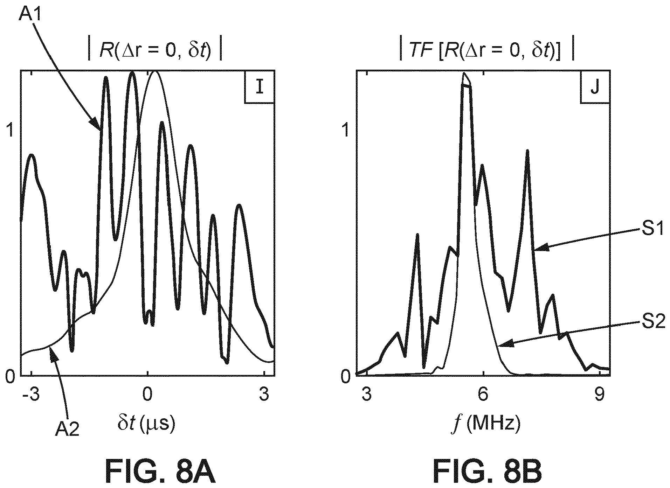

La décomposition en valeurs singulières permet d'extraire de manière très fiable l'onde cohérente des images/films de propagation. Par exemple, en **figure 8A,** la première courbe A1 correspond à l'amplitude du signal au point confocal en fonction du délai additionnel $\delta t$ (ici entre -3 $\mu$s et +3 $\mu$s) d'un des films de propagation obtenu pour un transducteur virtuel d'entré TV$_{in}$ appartenant à la zone de cohérence ZC. Le point confocal est le point des images de propagation défini par $\Delta x = \Delta z = |\Delta\mathbf{r}| = 0$ ($\mathbf{r_{in}} = \mathbf{r_{out}}$) représenté dans notre exemple par la croix située au centre de chaque image de propagation et qui correspond à la position du transducteur virtuel d'entrée. Cette courbe A1 est très chaotique dans le cas représenté, car la position confocale |**r**| coïncide avec une zone de type dit "speckle". L'onde cohérente est alors tout ou partie cachée par les échos provenant de diffuseurs localisés en amont ou en aval de cette zone confocale. Sur cette figure, la courbe A2 correspond à l'amplitude du signal du film de propagation d'onde cohérente (premier vecteur singulier $V_1$) issue de la décomposition en valeur singulière du film de propagation précédent, et pour le même point confocal. Cette courbe A2 montre un unique maximum centré au délai additionnel $\delta t$ nul, ce qui démontre une bonne focalisation de l'onde même pour ce cas d'espèce portant sur un élément peu réfléchissant.

La **figure 8B** montre les spectres de fréquence des signaux de la figure 8A, la courbe S1 correspondant au spectre de fréquence du signal de la courbe A1, et la courbe S2 correspondant au spectre de fréquence du signal de la courbe A2. On observe néanmoins une perte de la résolution temporelle de l'onde cohérente (visible sur la figure 7), qui se traduit par une réduction de la largeur spectrale des signaux étudiés. Si besoin, ce phénomène peut être corrigé à l'aide d'une étape d'égalisation de spectre.

Les images de propagation d'onde cohérente sont analogues à des images de propagation associées à un diffuseur échogène mais dont la largeur spectrale a été réduite.

Ces courbes A2, S2 illustrent l'efficacité de l'étape de combinaison/décomposition en valeur singulière pour extraire ou filtrer des films de propagation d'onde cohérente avec un seul maximum (une seule onde principale).

## Onde cohérente en référentiel balistique

Le calcul de la matrice de réflexion focalisée **RFoc(r$_{in}$, r$_{out}$, $\delta t$)** suppose un modèle de vitesse des ondes ultrasonores dans le milieu (par exemple, une vitesse du son $c_0$, constante). En effet, les temps de vol à l'aller $\tau_{in}$ et les temps de vol au retour **r$_{out}$** de l'onde sont calculés classiquement avec des formules géométriques de calcul de distance entre les transducteurs 11 et chaque point dans le milieu, et avec cette hypothèse de vitesse du son constante.

Par conséquent, les images de propagation, les films de propagation et les films de propagation d'onde cohérente calculés précédemment incluent cette hypothèse de vitesse du son $c_0$ constante. Dans ces images et films, l'onde cohérente résulte d'un processus de retournement temporel numérique basé sur le modèle de vitesse du son supposé. Cette onde se propage donc à la vitesse du son supposée $c_0$. Au temps $\delta t$ = 0, elle est localisée à la profondeur du transducteur virtuel d'entrée TV$_{in}$ (la croix centrale sur ces figures), c'est à dire pour $\Delta z$ = 0. Le temps de vol de l'onde cohérente suit donc la relation de propagation balistique suivante :

$$\delta t(\Delta \mathbf{r_{out}}) = -sign(\Delta z_{out}) \cdot |\Delta \mathbf{r_{out}}|/c_0$$

$$(\text{Equ. 7})$$

dans laquelle :

$c_0$ est la vitesse du son dans le milieu,
|$\Delta$**r$_{out}$**| est le module du vecteur entre le transducteur virtuel d'entrée TV$_{in}$ et le transducteur virtuel de sortie TV$_{out}$,
$\Delta$**r$_{out}$** = **r$_{out}$** - **r$_{in}$** ,
$\delta t$ est le délai additionnel,
$\Delta z_{out}$ est la composante selon le deuxième axe Z du vecteur de position spatiale $\Delta$**r$_{out}$**.

Autrement dit, dans ces images de propagation, l'onde théorique qui se propage à la vitesse du son $c_0$ forme un arc de cercle centré sur l'origine de l'image (i.e. le transducteur virtuel d'entrée TV$_{in}$ de position spatiale **r$_{in}$**). La relation de propagation balistique relie donc la position relative $\Delta$**r$_{out}$** au délai additionnel $\delta t$ par la vitesse du son $c_0$. Le signe négatif souligne le fait qu'il s'agit d'un processus de retournement temporel numérique.

Il est alors possible d'extraire du film de propagation ou du film de propagation d'onde cohérente, une image de focalisation de l'onde dans le référentiel balistique, image que l'on nomme image du front d'onde et qui suit cette onde théorique à la vitesse du son $c_0$ : Pour chaque image de propagation ou image de propagation d'onde cohérente, à un délai additionnel $\delta t$, on extrait les valeurs (valeur de pression acoustique) qui se situent sur cet arc de cercle (i.e. qui respectent la précédente relation de propagation balistique). On construit ainsi une nouvelle image, dite image du front d'onde qui représente l'évolution du film de propagation ou film de propagation d'onde cohérente dans le référentiel balistique. Cette image du front d'onde est donc une image du front d'onde dans le référentiel balistique.

Selon une première variante, on détermine l'image du front d'onde indirectement en calculant un film de propagation ou film de propagation d'onde cohérente, et en extrayant les données adéquates de ce film comme explicité ci-dessus pour déterminer l'image du front d'onde durant l'intervalle de délai additionnel.

Le procédé de caractérisation ultrasonore mis en oeuvre par l'unité de calcul 42 du système 40 peut donc être complété en appliquant une étape de détermination d'une image du front d'onde pour un transducteur virtuel d'entrée

$TV_{in}$ ou pour un transducteur virtuel d'entrée de référence $TV_{in,ref}$ et pour un intervalle de délai additionnel, ladite image du front d'onde étant déterminée à partir :

- des images d'un film de propagation ou d'un film de propagation d'onde cohérente, et
- d'une relation de propagation balistique du type :

$$\delta t(\Delta \mathbf{r_{out}}) = -sign(\Delta z_{out}) \cdot |\Delta \mathbf{r_{out}}|/c_0$$

qui permet d'extraire des valeurs de chacune des images des films pour construire l'image du front d'onde.

Selon une deuxième variante, on détermine une image du front d'onde directement à partir de la matrice de réflexion expérimentale $R_{ui}(t)$, en imposant la relation de propagation balistique ci-dessus.

Le procédé de caractérisation ultrasonore mis en oeuvre par l'unité de calcul 42 du système 40 peut donc être complété en appliquant une étape de détermination d'une image du front d'onde pour un transducteur virtuel d'entrée $TV_{in}$ et pour un intervalle de délai additionnel, ladite image du front d'onde étant déterminée à partir :

- de la matrice de réflexion focalisée $\mathbf{RFoc(r_{in}, r_{out}, \delta t)}$ et
- d'une relation de propagation balistique du type

$$\delta t(\Delta \mathbf{r_{out}}) = -sign(\Delta z_{out}) \cdot |\Delta \mathbf{r_{out}}|/c_0,$$

qui permet d'extraire des valeurs de la matrice de réflexion focalisée pour construire l'image du front d'onde.

Dans toutes ces variantes, l'image du front d'onde permet d'estimer le champ de pression (réponse en émission-réception) généré par le transducteur virtuel d'entrée $TV_{in}$ ou de référence $TV_{in,ref}$ à partir des échos mesurés par les transducteurs de la sonde.

Notons que les signaux contenus dans l'image du front d'onde est une sous-matrice de la matrice de réflexion focalisée. Donc, pour les calculs, on peut se limiter à des signaux qui vérifient la relation de propagation balistique ci-dessus. Dans ce cas, l'image du front d'onde est la matrice de réflexion focalisée $\mathbf{RFoc(r_{in}, r_{out}, \delta t)}$.

Les points ou pixels de ces images du front d'onde ont pour position spatiale $\Delta \mathbf{r_{out}} = \mathbf{r_{out}} - \mathbf{r_{in}}$, c'est à dire une position relative par rapport à la position $\mathbf{r_{in}}$ du transducteur virtuel d'entrée $TV_{in}$. Ainsi, les coordonnées sont notées $\Delta x$ en abscisse, et $\Delta z$ en ordonnée sur ces images. Ces images du front d'onde peuvent être aussi déterminées pour un procédé d'imagerie à trois dimensions. On utilise alors d'autres coordonnées pour représenter des images du front d'onde dans divers plans.

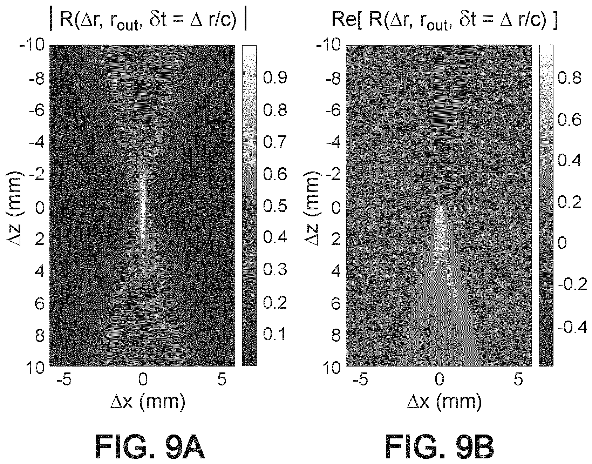

La **figure 9A** montre l'amplitude d'une telle image du front d'onde et la **figure 9B** montre la partie réelle de cette image du front d'onde. On constate dans cette figure 9B un saut de phase de $\pi$ radians au passage du point de focalisation du transducteur virtuel d'entrée $TV_{in}$ (position spatiale $\mathbf{r_{in}}$ de coordonnées $\Delta x = \Delta z = 0$ sur cette figure). Ce saut de phase est connu sous la désignation de saut de phase de Gouy. L'image du front d'onde permet d'illustrer clairement ce phénomène.

De même que pour les images de propagation, il est possible d'inverser le rôle joué par les transducteurs virtuels d'entrée $TV_{in}$ et les transducteurs virtuels de sortie $TV_{out}$. Dans ce cas on obtient une estimation du champ de pression généré par la focalisation en sortie.

**Détermination de la vitesse du son intégrée**

Le procédé et système de caractérisation ultrasonore d'un milieu selon la présente divulgation et mis en oeuvre par l'unité de calcul 42 du système 40 est également apte à déterminer la vitesse du son intégrée en un point du milieu. La vitesse du son intégrée est une estimation de valeur moyenne de la vitesse du son entre les transducteurs du dispositif de sondage 41 et un point du milieu. Plus exactement, cette vitesse du son intégrée intègre l'ensemble des vitesses du son locales des zones traversées par le trajet aller puis retour de l'onde ultrasonore.

Dans ce cas, le procédé comprend :

- une étape de détermination d'une image du front d'onde pour un transducteur virtuel d'entrée $TV_{in}$ et pour un intervalle de délai additionnel, ladite image du front d'onde étant déterminée comme décrit précédemment en fonction d'une vitesse du son $c_0$ dans le milieu,
- une étape de détermination d'une position en profondeur $\Delta z^{(0)}$ du centre de la tache focale dans l'image du front d'onde pour le transducteur virtuel d'entrée $TV_{in}$, et

- une étape de calcul d'une vitesse du son intégrée $c^{(1)}$ à partir de la formule suivante:

$$c^{(1)}(\mathbf{r_{in}}) = c_0 \sqrt{1 + \frac{\Delta z^{(0)}(\mathbf{r_{in}})}{z_{in}}}$$

(Equ. 8)

dans laquelle $z_{in}$ est la composante selon un deuxième axe Z du vecteur de position spatiale $\mathbf{r_{in}}$ du transducteur virtuel d'entrée $TV_{in}$.

Par "centre de la tache focale dans l'image du front d'onde", on entend par exemple la position du maximum de la tache focale dans l'image du front d'onde ; c'est à dire la position du pixel ayant la plus grande valeur de toute l'image du front d'onde. Il faut noter que dans l'image du front d'onde, on n'observe qu'une seule tache focale, et sa position est donc unique. Ainsi, la position du centre de la tache focale est également unique, et représente la position en profondeur $\Delta z^{(0)}(\mathbf{r_{in}})$ à utiliser pour corriger la vitesse du son $c_0$, pour le point du milieu correspondant à la position spatiale $\mathbf{r_{in}}$ du transducteur virtuel d'entrée $TV_{in}$.

Par exemple, le centre de la tache focale est déterminé en recherchant dans l'image du front d'onde la position spatiale du point de plus grande valeur, et la position en profondeur $\Delta z^{(0)}$ du centre de la tache focale est alors la composante dans la direction de l'axe Z de profondeur, correspondant à l'axe $\Delta z$, de ce point de plus grande valeur.

Notons que la position en profondeur $\Delta z^{(0)}$ est déterminée pour chaque transducteur virtuel d'entrée $TV_{in}$ pris dans le milieu ou inversement pour chaque transducteur virtuel de sortie $TV_{out}$ pris dans le milieu. Plus généralement, cette position en profondeur dépend de chaque point de position spatiale $\mathbf{r}$ considéré est peut être notée $\Delta z^{(0)}(\mathbf{r})$ avec $\mathbf{r} = \mathbf{r_{in}}$ ou $\mathbf{r} = \mathbf{r_{out}}$.

En effet, dans les images du film de propagation ou du film de propagation d'onde cohérente, l'onde ultrasonore se focalise à l'instant du délai additionnel $\delta t$ nul ($\delta t=0$) seulement si la vitesse du son $c_0$, utilisée pour le calcul de la matrice de réflexion focalisée $\mathbf{RFoc}(\mathbf{r_{in}}, \mathbf{r_{out}}, \delta t)$ à travers les calculs de temps de vol à l'aller et de temps de vol au retour, et pour le calcul de l'image du front d'onde à travers la relation de propagation balistique, est une valeur de vitesse du son qui correspond à une vitesse du son intégrée correcte pour le milieu réel entre les transducteurs 11 du dispositif de sondage 41 et le point du milieu correspondant au transducteur virtuel d'entrée $TV_{in}$ de position spatiale $\mathbf{r_{in}}$.

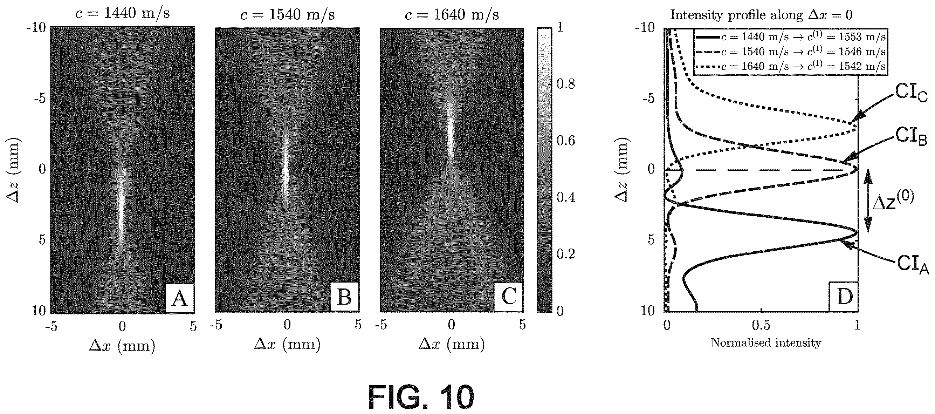

Par exemple, la **figure 10** illustre ce processus. Dans cette figure 10, les images référencées A, B et C montrent des images du front d'onde obtenues avec des vitesses du son $c_0$ prédéfinies respectivement de 1440 m/s, 1540 mis et 1640 m/s. Dans ces images du front d'onde, on observe une tache focale qui se déplace selon l'axe des ordonnées $\Delta z$, c'est à dire en profondeur (direction Z). Le graphique référencé D montre alors les trois courbes d'intensité $CI_A$, $CI_B$ et $CI_C$ de ces images du front d'onde sur cet axe des ordonnées $\Delta z$, c'est à dire l'axe pour lequel $\Delta x = 0$.

Par exemple, la position en profondeur $\Delta z^{(0)}(\mathbf{r_{in}})$ de la tache focale est obtenue comme illustré en figure 10, c'est à dire en déterminant la position en profondeur du maximum des valeurs de l'image du front d'onde sur l'axe des ordonnées $\Delta z$, tel que $\Delta x = 0$. La position en profondeur $\Delta z^{(0)}(\mathbf{r_{in}})$ de la tache focale dans l'image du front d'onde est effectuée en recherchant dans l'image du front d'onde la position sur l'axe des ordonnées $\Delta z$ ayant une valeur maximale dans cette image du front d'onde, cet axe des ordonnées $\Delta z$ correspondant à une abscisse $\Delta x$ nulle dans l'image du front d'onde.

Par exemple, pour la courbe d'intensité $CI_A$ du graphique D, la position en profondeur $\Delta z^{(0)}(\mathbf{r_{in}})$ vaut sensiblement 4,5 mm, ce qui va conduire à une estimation de la vitesse du son intégrée $c^{(1)}(\mathbf{r_{in}})$ supérieure à la vitesse du son initialement supposée $c^{(0)}$, à la position $\mathbf{r_{in}}$ du transducteurs virtuel d'entrée choisi $TV_{in}$, de sorte que la position verticale selon l'axe $\Delta z$ de la tache focale de l'image A sera déplacé vers le haut et donc vers le point d'origine ($\Delta x = \Delta z = 0$) du transducteur virtuel d'entrée, ce qui correspond à un recalage par calcul de la vitesse du son intégrée pour ce point du milieu du transducteur virtuel d'entrée $TV_{in}$.

Par conséquent, en pratique, on peut éventuellement se satisfaire de calculer les valeurs de l'image du front d'onde sur l'axe $\Delta z$, pour lequel $\Delta x=0$, pour déterminer une vitesse du son ou vitesse du son intégrée.

Ainsi, le procédé de caractérisation ultrasonore d'un milieu, pour déterminer une vitesse du son intégrée, comprend les étapes suivantes :

- une étape de génération d'une série d'ondes ultrasonores incidentes $US_{in}$ dans une zone dudit milieu, au moyen d'un réseau 10 de transducteurs 11, ladite série d'ondes ultrasonores incidentes étant une base d'émission $\mathbf{i}$ ; et
- une étape de génération d'une matrice de réflexion expérimentale $\mathbf{R_{ui}}(t)$ définie entre la base d'émission $\mathbf{i}$ en entrée et une base de réception $\mathbf{u}$ en sortie ;
- une étape de détermination d'une matrice de réflexion focalisée $\mathbf{RFoc}(\mathbf{r_{in}}, \mathbf{r_{out}}, \delta t)$ qui comprend des réponses du milieu entre un transducteur virtuel d'entrée $TV_{in}$ de position spatiale $\mathbf{r_{in}}$ et un transducteur virtuel de sortie $TV_{out}$

de position spatiale $r_{out}$, les réponses du transducteur de virtuel de sortie $TV_{out}$ étant prises à un instant temporel décalé d'un délai additionnel $\delta t$ par rapport à un instant temporel des réponses du transducteur virtuel d'entrée $TV_{in}$,

- une étape de détermination d'une image du front d'onde pour le transducteur virtuel d'entrée $TV_{in}$ et pour un intervalle de délai additionnel, ladite image du front d'onde étant déterminée en fonction de la vitesse du son $c_0$ dans le milieu, et ladite image du front d'onde étant déterminée à partir :

  - de la matrice de réflexion focalisée $\mathbf{RFoc}(\mathbf{r_{in}}, \mathbf{r_{out}}, \delta t)$ et
  - d'une relation de propagation balistique du type

$$\delta t(\Delta\mathbf{r_{out}}) = -sign(\Delta z_{out}) \cdot |\Delta\mathbf{r_{out}}|/c_0,$$

qui permet d'extraire des valeurs de la matrice de réflexion focalisée pour construire l'image du front d'onde, et dans laquelle :

$\delta t$ est le délai additionnel,
$|\Delta\mathbf{r_{out}}|$ est le module du vecteur entre le transducteur virtuel d'entrée $TV_{in}$ et le transducteur virtuel de sortie $TV_{out}$, avec $\Delta\mathbf{r_{out}} = \mathbf{r_{out}} - \mathbf{r_{in}}$ ,
$\Delta z_{out}$ est la composante selon un axe Z de profondeur du vecteur de position spatiales $\Delta\mathbf{r_{out}}$,

- une étape de détermination d'une position en profondeur $\Delta z^{(0)}$ du centre de la tache focale dans l'image du front d'onde, et
- une étape de calcul d'une vitesse du son intégrée $c^{(1)}$, à partir de la formule suivante :

$$c^{(1)}(\mathbf{r_{in}}) = c_0 \sqrt{1 + \frac{\Delta z^{(0)}(\mathbf{r_{in}})}{z_{in}}}$$

(Equ. 9)

dans laquelle $z_{in}$ est la composante selon l'axe Z en profondeur du vecteur de position spatiale $\mathbf{r_{in}}$ du transducteur virtuel d'entrée $TV_{in}$.

Eventuellement, ce procédé peut être itéré une ou plusieurs fois comme défini précédemment, en calculant une nouvelle vitesse du son intégrée $c^{(n+1)}$ à partir de la détermination d'une image du front d'onde obtenue avec la précédente vitesse du son intégrée $c^{(n)}$, de la détermination d'une position en profondeur $\Delta z^{(n)}$ du centre de la tache focale, et du calcul de la nouvelle vitesse du son intégrée $c^{(n)}$ par la même formule d'itération :

$$c^{(n+1)}(\mathbf{r_{in}}) = c^{(n)}(\mathbf{r_{in}}) \sqrt{1 + \frac{\Delta z^{(n)}(\mathbf{r_{in}})}{z_{in}}}$$

(Equ. 10)

En pratique, ce processus itératif converge extrêmement rapidement vers une vitesse du son intégrée optimale qui correspond à la meilleure vitesse du son intégrée pour les transducteurs 11 du dispositif de sondage et le point choisi du milieu (transducteur virtuel d'entrée).

En outre, dans une variante, ce procédé pour déterminer la vitesse du son intégré peut être amélioré en effectuant entre l'étape de détermination d'une image du front d'onde et l'étape de détermination de la position en profondeur $\Delta z^{(0)}(\mathbf{r_{in}})$ d'une tache focale, une étape d'amélioration de l'image du front d'onde dans laquelle on effectue une combinaison linéaire d'un ensemble d'images du front d'onde correspondant à une zone de cohérence ZC donnée, chaque image du front d'onde de l'ensemble étant prise entre un transducteur virtuel d'entrée $TV_{in}$ choisi de position spatiale $\mathbf{r_{in}}$ différente, et des transducteurs virtuels de sortie $TV_{out}$ de position spatiale $\mathbf{r_{out}}$ tels que $\mathbf{r_{out}} = \Delta\mathbf{r_{out}} + \mathbf{r_{in}}$, avec $\Delta\mathbf{r_{out}}$ étant prédéfinis et identiques pour toutes les images du front d'onde de l'ensemble, et les transducteurs virtuels d'entrée choisis étant voisins les uns des autres. On obtient ainsi une image du front d'onde améliorée ou image du front d'onde cohérente associée à un transducteur virtuel d'entrée de référence $TV_{in,ref}$, ce transducteur virtuel d'entrée de référence $TV_{in,ref}$ représentant les transducteurs virtuels d'entrée de l'ensemble des images du front d'onde utilisées associés à la zone de cohérence ZC choisie, et pour les mêmes positions relatives $\Delta r_{out}$.

Par exemple, le transducteur virtuel d'entrée de référence $TV_{in,ref}$ est un transducteur virtuel d'entrée de position spatiale correspondant à la moyenne des positions spatiales des transducteurs virtuels d'entrée choisis ou une moyenne pondérée des positions spatiales des transducteurs virtuels d'entrée choisis, comme déjà explicité précédemment dans le cas de films de propagation.

En résumé, dans le procédé de la présente divulgation, on ajoute les étapes suivantes :

- entre l'étape de détermination d'une image du front d'onde et l'étape de détermination de la position en profondeur $\Delta z^{(0)}(r_{in})$ d'une tache focale, on effectue une étape d'amélioration de l'image du front d'onde dans laquelle on effectue une combinaison linéaire d'un ensemble d'images du front d'onde correspondant à une zone de cohérence, chaque image du front d'onde étant prise entre un transducteur virtuel d'entrée ($TV_{in}$) choisi de position spatiale $r_{in}$ différente, et des transducteurs virtuels de sortie ($TV_{out}$) de position spatiale $r_{out}$ tels que $r_{out} = \Delta r_{out} + r_{in}$, avec $\Delta r_{out}$ étant prédéfinis et identiques pour toutes les images du front d'onde de l'ensemble, et les transducteurs virtuels d'entrée choisis étant voisins les uns des autres, pour obtenir une image du front d'onde améliorée associé à un transducteur virtuel d'entrée de référence ($TV_{in,ref}$), ce transducteur virtuel d'entrée de référence $TV_{in,ref}$ étant caractéristique des transducteurs virtuels d'entrée de l'ensemble des images du front d'onde utilisées et associées à la zone de cohérence ZC, et

- dans l'étape de détermination d'une position en profondeur $\Delta z^{(0)}(r_{in})$, l'image du front d'onde améliorée est utilisée à la place de l'image du front d'onde, la position en profondeur du centre de la tache focale est relative à la position spatiale du transducteur virtuel d'entrée de référence $TV_{in,ref}$, et cette position en profondeur du centre de la tache focale permet d'estimer une vitesse du son intégrée $c^{(1)}(r_{in,ref})$ à la position spatiale du transducteur virtuel d'entrée de référence $TV_{in,ref}$.

L'image du front d'onde améliorée (image du front d'onde cohérente) est alors utilisée (à la place de l'image du front d'onde.) afin de déterminer la position axiale du centre de la tache focale. Cette distance ou position en profondeur $\Delta z^{(0)}(r_{in,ref})$ est alors caractéristique d'un modèle de vitesse du son incorrecte et peut être utilisé afin d'estimer la vitesse du son intégrée $c^{(1)}(r_{in,ref})$ associée à la position spatiale $r_{in,ref}$ du transducteurs virtuel d'entrée de référence $TV_{in,ref}$.

Selon un mode de réalisation, la combinaison linéaire est déterminée par un calcul de décomposition en valeur singulière SVD de l'ensemble des images du front d'onde pour obtenir un vecteur singulier $W_1$ associé à la valeur singulière de décomposition en valeur singulière de plus grande valeur absolue, ce vecteur singulier $W_1$ étant alors l'image du front d'onde améliorée correspondant audit transducteur virtuel d'entrée de référence $TV_{in,ref}$ et pour les mêmes délais additionnels $\delta t$.

La pluralité des images du front d'onde de l'ensemble peut ici être traitée par décomposition en valeurs singulières pour combiner plusieurs mesures ou expériences du désordre acoustique d'une région voisine d'un transducteur virtuel d'entrée, ce qui permet de s'affranchir des fluctuations liées au désordre et d'améliorer le contraste de l'image du front d'onde, et son exploitation.

En outre, il est possible de déterminer une vitesse du son optimale du milieu (réaliste pour le milieu dans sa globalité) par calcul d'une vitesse du son intégrée comme décrit précédemment, et en prenant pour la combinaison linéaire de l'ensemble d'images du front d'onde, un ensemble d'images du front d'onde correspondant à des transducteurs virtuels d'entrée ($TV_{in}$) choisis qui couvrent sensiblement l'ensemble d'une zone d'intérêt du milieu. Notamment, Ces transducteurs virtuels d'entrée choisis peuvent être répartis de manière régulière sur toute la zone d'intérêt du milieu, avec un espacement prédéterminé. Par exemple, ces transducteurs virtuels d'entrée choisis peuvent représenter 20% ou plus du nombre des transducteurs virtuels d'entrée utilisés par exemple pour construire une image échographique du milieu couvrant la zone à étudier.

Notons que les distances ou positions en profondeur $\Delta z^{(0)}(r_{in})$ ou $\Delta z^{(0)}(r_{in,ref})$ peuvent être interprétées comme une erreur de focalisation en sortie due aux aberrations subies lors de l'étape de rétropropagation des échos provenant des positions spatiales $r_{in}$ ou $r_{in,ref}$. La mesure de la vitesse du son intégrée peut aussi être déterminée en sondant les aberrations subies par les fronts d'onde lors du chemin aller. Cette mesure est décrite en inversant les dénomination "in" et "out" dans les équations ci-dessus en intervertissant le rôle des transducteurs virtuels d'entrée et de sortie, pour obtenir une autre estimation de vitesse du son intégrée $c^{(1)}{}_{out}$.

En outre il est possible d'améliorer l'estimation de vitesse du son intégrée en combinant les mesures ou estimations de vitesse du son intégrées obtenues à partir des aberrations engendrées à l'aller et/ou au retour, c'est à dire les vitesses du son intégrées $c^{(1)}{}_{in}$ et $c^{(1)}{}_{out}$.

Le procédé est alors complété par les étapes suivantes :

- on intervertit le rôle du ou des transducteurs virtuels d'entrées et du ou des transducteurs virtuels de sortie pour déterminer une vitesse du son intégrée $c^{(1)}(r_{out})$ par rapport à un transducteur virtuel de sortie, et
- on combine la vitesse du son intégrée $c^{(1)}(r_{in})$ en référence au transducteur virtuel d'entrée et la vitesse du son

intégrée $c^{(1)}(\mathbf{r_{out}})$ en référence au transducteur virtuel de sortie pour obtenir une vitesse du son intégrée améliorée.

## Images de vitesse du son intégrée

Le procédé de caractérisation ultrasonore mis en oeuvre par l'unité de calcul 42 du système 40 peut être complété en construisant une ou plusieurs <u>images de vitesse du son intégrée,</u> cette ou ces images de vitesse du son intégrée étant déterminées par au moins un calcul d'une vitesse du son intégrée tel que décrit ci-dessus et pour une pluralité de points du milieu correspondant à des transducteurs virtuels d'entrée $TV_{in}$ (premier points P1) de position spatiale $\mathbf{r_{in}}$.

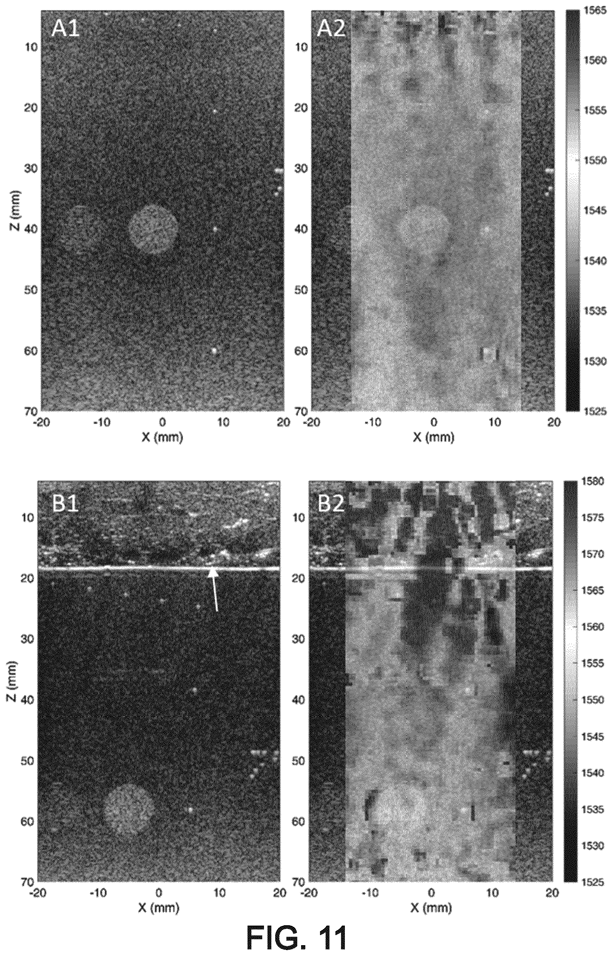

La **figure 11** illustre deux exemples de telles images.

Dans le premier exemple correspondant aux images A1 et A2 de la figure 11, le milieu est de type phantom avec une vitesse du son de référence donnée pour sensiblement $c_{ref}$ = 1542 m/s. L'image A1 est l'image échographique standard, alors que l'image A2 est l'image de vitesse du son intégrée obtenue par le procédé précédent. Cette image du son intégrée A2 permet d'estimer une valeur moyenne de la vitesse du son dans le milieu de 1544 mis avec un écart type de +/- 3 m/s, ce qui est tout à fait en accord avec la valeur de vitesse du son de référence de ce milieu.

Dans le deuxième exemple, correspondant aux images B1 et B2 de la figure 11, le milieu est un milieu stratifié avec une première couche de presque 20 mm d'épaisseur, avec une structure fibreuse et une vitesse du son de sensiblement 1570 mis positionnée sur le même milieu de type phantom ayant une vitesse du son prédéterminée par construction de sensiblement 1542 m/s. L'image B1 est l'image échographique standard de ce milieu, alors que l'image B2 est l'image de vitesse du son intégrée obtenue par le procédé divulgué précédemment. Cette image du son intégrée B2 traduit une vitesse du son plus élevée dans la première couche de l'ordre de 1580 mis et une vitesse du son plus faible en dessous, mais pas identique à celle de la vitesse du son attendue et correspondant à celle de ce milieu étudié dans le premier exemple. Cet effet est dû au fait que car la vitesse du son calculée par la méthode est une vitesse du son intégrée qui correspond à une vitesse du son moyenne ou intégrée sur la totalité du trajet aller et retour des ondes entre les transducteurs 11 et le point du milieu.

## Correction des aberrations axiales

Le procédé et système de caractérisation ultrasonore d'un milieu selon la présente divulgation et mis en oeuvre par l'unité de calcul 42 du système 40 est également apte à déterminer <u>une correction axiale.</u>

Le procédé de caractérisation ultrasonore d'un milieu, pour déterminer une caractérisation temporelle et locale d'une focalisation ultrasonore avec une correction axiale comprend les étapes suivantes déjà explicités pour obtenir une matrice de réflexion focalisée, c'est à dire :

- une étape de génération d'une série d'ondes ultrasonores incidentes $US_{in}$ dans une zone dudit milieu, au moyen d'un réseau 10 de transducteurs 11, ladite série d'ondes ultrasonores incidentes étant une base d'émission i ; et
- une étape de génération d'une matrice de réflexion expérimentale $\mathbf{R_{ui}}(t)$ définie entre la base d'émission i en entrée et une base de réception u en sortie ;
- une étape de détermination d'une matrice de réflexion focalisée $\mathbf{RFoc}(\mathbf{r_{in}}, \mathbf{r_{out}}, \delta t)$ qui comprend des réponses du milieu entre un transducteur virtuel d'entrée $TV_{in}$ de position spatiale $\mathbf{r_{in}}$ et un transducteur virtuel de sortie $TV_{out}$ de position spatiale $\mathbf{r_{out}}$, les réponses du transducteur de virtuel de sortie $TV_{out}$ étant prises à un instant temporel décalé d'un délai additionnel $\delta t$ par rapport à un instant temporel des réponses du transducteur virtuel d'entrée $TV_{in}$, et
- une étape de détermination d'une image du front d'onde pour un transducteur virtuel d'entrée $TV_{in}$ et pour un intervalle de délai additionnel, ladite image du front d'onde étant déterminée comme décrit précédemment en fonction d'une vitesse du son $c_0$ dans le milieu,
- une étape de détermination d'une position en profondeur $\Delta z^{(0)}(\mathbf{r_{in}})$ du centre de la tache focale dans l'image du front d'onde.

Par "centre de la tache focale dans l'image du front d'onde", on entend par exemple la position du maximum de la tache focale dans l'image du front d'onde ; c'est à dire la position du pixel ayant la plus grande valeur de toute l'image du front d'onde. Le centre de la tache focale et la position en profondeur peuvent être trouvés / déterminés selon une des techniques déjà exposée ci-dessus.

Ce procédé comprend en outre une étape de détermination d'une matrice de réflexion focalisée corrigée $\mathbf{RFoc^{(1)}}(\mathbf{r_{in}}, \mathbf{r_{out}}, \delta t)$ par une translation des réponses de la matrice de réflexion focalisée $\mathbf{RFoc}(\mathbf{r_{in}}, \mathbf{r_{out}}, \delta t)$ d'une translation spatiale dans la direction Z de profondeur, ladite translation spatiale étant fonction de la position de profondeur $\Delta z^{(0)}(\mathbf{r_{in}})$ déterminée précédemment.

Selon une première variante, la translation spatiale est effectuée par translation spatiale de la composante axiale du transducteur virtuel de sortie $TV_{out}$ de position spatiale $\mathbf{r_{out}}$ (selon l'axe Z de profondeur) d'une valeur de

correction $\Delta z_{corr}(\mathbf{r_{in}})$ égale à $2.\Delta z^{(0)}(\mathbf{r_{in}})$, pour obtenir la matrice de réflexion focalisée corrigée $\mathbf{RFoc}^{(1)}(\mathbf{r_{in}}, \mathbf{r_{out}}, \delta t)$, telle que :

$$\mathbf{RFoc}^{(1)}(\mathbf{r_{in}}, \mathbf{r_{out}}, \delta t) = \mathbf{RFoc}(\mathbf{r_{in}}, \{x_{out}, z_{out} + \Delta z_{corr}(\mathbf{r_{out}})\}, \delta t)$$

(Equ. 11)

Une image échographique corrigée $I^{(1)}(\mathbf{r_{in}})$ peut ensuite être construite à partir de la matrice de réflexion focalisée corrigée $\mathbf{RFoc}^{(1)}(\mathbf{r_{in}}, \mathbf{r_{out}}, \delta t)$ caractérisés par $r = \mathbf{r_{in}} = \mathbf{r_{out}}$, et $\delta t = 0$ pour obtenir :

$$I^{(1)}(\mathbf{r}) = \mathbf{RFoc}^{(1)}(\mathbf{r_{in}}, \mathbf{r_{out}} = \mathbf{r_{in}}, \delta t = 0)$$

(Equ. 12)

Réciproquement, la translation spatiale peut aussi correspondre à la translation spatiale des composantes selon l'axe Z de profondeur du transducteur virtuel d'entrée $TV_{in}$ de position spatiale $\mathbf{r_{in}}$ d'une valeur de correction $\Delta z_{corr}(\mathbf{r_{in}})$ égale à $2.\Delta z^{(0)}(\mathbf{r_{in}})$ pour obtenir la matrice de réflexion focalisée corrigée $\mathbf{RFoc}^{(1)}(\mathbf{r_{in}}, \mathbf{r_{out}}, \delta t)$ suivante :

$$\mathbf{RFoc}^{(1)}(\mathbf{r_{in}}, \mathbf{r_{out}}, \delta t) = \mathbf{RFoc}(\{x_{in}, z_{in} + \Delta z_{corr}(\mathbf{r_{in}})\}, \mathbf{r_{out}}, \delta t)$$

(Equ. 13)

Notons que la position en profondeur $\Delta z^{(0)}$ est déterminée pour chaque transducteur virtuel d'entrée $TV_{in}$ pris dans le milieu et est caractéristique de l'aberration subie lors du trajet retour. En inversant les notation "in" et "out", il est possible de déterminer la position $\Delta z^{(0)}(\mathbf{r_{out}})$ caractéristique de l'aberration subie lors du trajet aller, pour chaque transducteur virtuel de sortie $TV_{out}$ pris dans le milieu. Autrement dit, plus généralement, cette position en profondeur dépend de chaque point de position spatiale $\mathbf{r}$ considéré est peut aussi être noté $\Delta z^{(0)} = \Delta z^{(0)}(\mathbf{r})$ avec $\mathbf{r} = \mathbf{r_{in}}$ ou $\mathbf{r} = \mathbf{r_{out}}$.

Selon une deuxième variante, la translation spatiale est effectuée par :

- calcul d'une valeur de correction $\Delta z_{corr}(\mathbf{r})$ égale à $\Delta z^{(1)}(\mathbf{r})$ qui est déterminée par la formule suivante :

$$\Delta z^{(1)}(\mathbf{r}) = z^{(1)}(\mathbf{r}) - z_{in}$$

avec

$$z^{(1)}(\mathbf{r}) = z\sqrt{1 + \frac{\Delta z^{(0)}(\mathbf{r_{in}})}{z_{in}}}$$

(Equ. 14)

dans laquelle on applique cette équation pour
$\mathbf{r} = \mathbf{r_{in}}$ et $\mathbf{r} = \mathbf{r_{out}}$
$z = z_{in}$ et $z = z_{out}$ est la composante selon un axe Z de profondeur de la position spatiale $\mathbf{r_{in}}$ du transducteur virtuel d'entrée $TV_{in}$ ou de la position spatiale $\mathbf{r_{out}}$ du transducteur virtuel de sortie $TV_{out}$,

- calcul matrice de réflexion focalisée corrigée $\mathbf{RFoc}^{(1)}(\mathbf{r_{in}}, \mathbf{r_{out}}, \delta t)$ par translation spatiale des composantes selon l'axe Z de profondeur du transducteur virtuel d'entrée $TV_{in}$ de position spatiale $\mathbf{r_{in}}$ de ladite valeur de correction $\Delta z_{corr}(\mathbf{r_{in}})$ et du transducteur virtuel de sortie $TV_{out}$ de position spatiale $\mathbf{r_{out}}$ de ladite valeur de correction $\Delta z_{corr}(\mathbf{r_{out}})$, tel que :

$$\mathbf{RFoc}^{(1)}(\mathbf{r_{in}}, \mathbf{r_{out}}, \delta t) = \mathbf{RFoc}(\{x_{in}, z_{in} + \Delta z_{corr}(\mathbf{r_{in}})\}, \{x_{out}, z_{out} + \Delta z_{corr}(\mathbf{r_{out}})\}, \delta t)$$

Ce précédent calcul, peut aussi être exprimé en fonction de la vitesse du son intégrée $c^{(1)}(\mathbf{r})$ à partir de la formule suivante :

$$c^{(1)}(\mathbf{r_{in}}) = c_0 \sqrt{1 + \frac{\Delta z^{(0)}(\mathbf{r_{in}})}{z_{in}}}$$

(Equ. 15)

dans laquelle $z_{in}$ est la composante selon un deuxième axe Z du vecteur de position spatiale $\mathbf{r_{in}}$ du transducteur virtuel d'entrée TV$_{in}$, et

- le calcul de translation $\Delta z^{(1)}(\mathbf{r})$ par :

$$\Delta z^{(1)}(\mathbf{r}) = z^{(1)}(\mathbf{r}) - z_{in}$$

avec

$$z^{(1)}(\mathbf{r}) = z_{in}\frac{c^{(1)}(\mathbf{r})}{c_0} = z_{in}\sqrt{1 + \frac{\Delta z^{(0)}(\mathbf{r})}{z_{in}}}$$

(Equ. 16)

Selon des modifications des deux variantes précédentes, on peut implémenter les translations par un calcul de transformée de Fourier spatiale, déphasage par une rampe de phase dont la pente dépend de la valeur de correction, puis transformée de Fourier spatiale inverse. Cette implémentation a pour intérêt de permettre de combiner translation et interpolation pour de nouvelles coordonnées spatiales.

A titre d'exemple, le procédé ainsi implémenté effectue :

- une étape de détermination d'une matrice fréquentielle spatiale **RFreq**$_z$($\mathbf{r_{in}}$, x$_{out}$, k$_{zout}$, $\delta t$) qui est une transformée de Fourier spatiale selon une direction de profondeur de la matrice de réflexion focalisée **RFoc**($\mathbf{r_{in}}$, $\mathbf{r_{out}}$, $\delta t$), selon l'équation :

$$RFreq_z(\mathbf{r_{in}}, x_{out}, k_{z,out}, \delta t) = TF_{zout}[RFoc(\mathbf{r_{in}}, \mathbf{r_{out}}, \delta t)]$$

(Equ. 17)

dans laquelle

TF$_{zout}$ est la transformée de Fourier spatiale selon la direction de profondeur $\Delta z_{out}$,
k$_{zout}$ est le nombre d'onde correspondant compris dans l'intervalle [$\omega^-/c_0$, $\omega^+/c_0$], avec les pulsations $\omega^-$ et $\omega^+$ qui sont les pulsations limites de la bande passante des ondes ultrasonores, et
x$_{out}$ est la composante transverse dans la direction de l'axe X, de chaque transducteur virtuel de sortie TV$_{out}$ de position spatiale $\mathbf{r_{out}}$.

- une étape de détermination d'une matrice de réflexion focalisée corrigée **RFoc**$^{(1)}$($\mathbf{r_{in}}$, $\mathbf{r_{out}}$, $\delta t$) correspondant à la transformée de Fourier spatiale inverse selon la même direction de profondeur, du produit de la matrice fréquentielle spatiale **RFreq**$_z$($\mathbf{r_{in}}$, x$_{out}$, k$_{zout}$, $\delta t$) par une rampe de phase de la valeur de correction $\Delta z_{corr}$ en profondeur, c'est à dire égale à 2.$\Delta z^{(0)}$($\mathbf{r_{in}}$) selon les variantes précédentes, et déterminée pour chaque position spatiale du transducteur virtuel d'entrée TV$_{in}$, et dans laquelle on applique la formule suivante:

$$RFoc^{(1)}(\mathbf{r_{in}}, \mathbf{r_{out}}, \delta t) = TF^{-1}_{kz_{out}}\left[RFreq_z(\mathbf{r_{in}}, \mathrm{x_{out}}, k_{z,out}, \delta t)e^{-i\,k_{z,out}\Delta z_{corr}}\right]$$

$$(Equ.\ 18)$$

dans laquelle

$e^{-ix}$ est la fonction exponentielle complexe,
$\Delta z_{corr}$ est la valeur de correction déterminée par la position en profondeur du centre de la tache focale dans l'image du front d'onde.

La transformée de Fourier spatiale dans la direction $\Delta z_{out}$ peut par exemple être explicitée par la formule de transformée de Fourier discrète spatiale suivante :

$$RFreq_z(\mathbf{r_{in}}, \mathrm{x_{out}}, k_{z,out}, \delta t) = TF_{zout}[RFoc(r_{in}, r_{out}, \delta t)]$$

$$= \sum_{\Delta z_{out}} RFoc(\mathbf{r_{in}}, \mathbf{r_{out}}, \delta t)e^{-ik_{z,out}\cdot\Delta z_{out}}$$

$$(Equ.\ 19)$$

D'autres formulations de transformé de Fourier et transformée de Fourier spatiale existent.
La transformée de Fourier spatiale inverse dans la direction $\Delta z_{out}$ peut être alors explicitée par la formule réciproque suivante :

$$RFoc^{(1)}(\mathbf{r_{in}}, \mathbf{r_{out}}, \delta t) = TF^{-1}_{kz_{out}}\left[RFreq_z(\mathbf{r_{in}}, \mathrm{x_{out}}, k_{z,out}, \delta t)\,e^{-i\,k_{z,out}\Delta z_{corr}}\right]$$

$$= \sum_{k_{z,out}} RFreq_z(r_{in}, \mathrm{x_{out}}, k_{z,out}, \delta t)\,e^{-i\,k_{z,out}\Delta z_{corr}}\,e^{ik_{z,out}\,\Delta z_{out}}$$

$$(Equ.\ 20)$$

Selon une troisième variante, on translate axialement les réponses par un calcul ou détermination d'une matrice de réflexion focalisée corrigée $\mathbf{RFoc}^{(1)}(\mathbf{r_{in}}, \mathbf{r_{out}}, \delta t)$ avec une nouvelle vitesse du son $c_1(r)$ qui remplace la vitesse du son supposée $c_0$.
Le procédé de cette troisième variante comprend ainsi en outre les étapes suivantes pour obtenir une matrice de réflexion focalisée corrigée axialement :

- une étape de calcul d'une vitesse du son intégrée $c^{(1)}(\mathbf{r})$ à partir de la formule suivante :

$$c^{(1)}(\mathbf{r_{in}}) = c_0\sqrt{1 + \frac{\Delta z^{(0)}(\mathbf{r_{in}})}{z_{in}}}$$

$$(Equ.\ 21)$$

dans laquelle $z_{in}$ est la composante selon un deuxième axe Z du vecteur de position spatiale $\mathbf{r_{in}}$ du transducteur virtuel d'entrée $TV_{in}$, et
- une étape de détermination d'une matrice de réflexion focalisée corrigée $\mathbf{RFoc}^{(1)}(\mathbf{r_{in}}, \mathbf{r_{out}}, \delta t)$ qui comprend des réponses du milieu entre un transducteur virtuel d'entrée $TV_{in}$ de position spatiale $\mathbf{r_{in}}$ et un transducteur virtuel de sortie $TV_{out}$ de position spatiale $\mathbf{r_{out}}$, les réponses étant chacune obtenues avec une vitesse du son corrigé dépendant du transducteur virtuel d'entrée.

Pour chacune de ces variantes, la matrice de réflexion focalisée corrigée $\mathbf{RFoc}^{(1)}(\mathbf{r_{in}}, \mathbf{r_{out}}, \delta t)$ est une correction

axiale de la matrice de réflexion focalisée, c'est à dire une matrice de réflexion focalisée dont on a corrigé les aberrations axiales. Grâce à cette matrice de réflexion focalisée corrigée, il devient avantageusement possible de construire une image échographique avec des aberrations axiales réduites. Ainsi, les distances dans la direction axiale dans cette image échographique corrigée sont plus précises et permettent par exemple l'obtention d'images de meilleure qualité.

La matrice de réflexion focalisée corrigée $\mathbf{RFoc}^{(1)}(\mathbf{r_{in}}, \mathbf{r_{out}}, \delta t)$ est obtenue par une translation spatiale, qui est soit une translation de position spatiale en direction Z de la composante axial de l'un ou des deux transducteurs virtuels ($TV_{in}$ et/ou $TV_{out}$), soit une translation par changement de vitesse du son c. Ces alternatives permettent d'améliorer l'étape de formation de voie qui s'apparente à un processus de traduction d'une information temporelle donnée par les signaux expérimentaux de la matrice de réflexion expérimentale $\mathbf{R_{ui}}(t)$ (aussi souvent dénommés par signaux RF) en une information spatiale via la relation $t = z / c$. Ainsi, les positions spatiales des points du milieu sont corrigées axialement dans la direction Z de profondeur, ce qui permet d'obtenir des images avec un positionnement vertical plus précis.

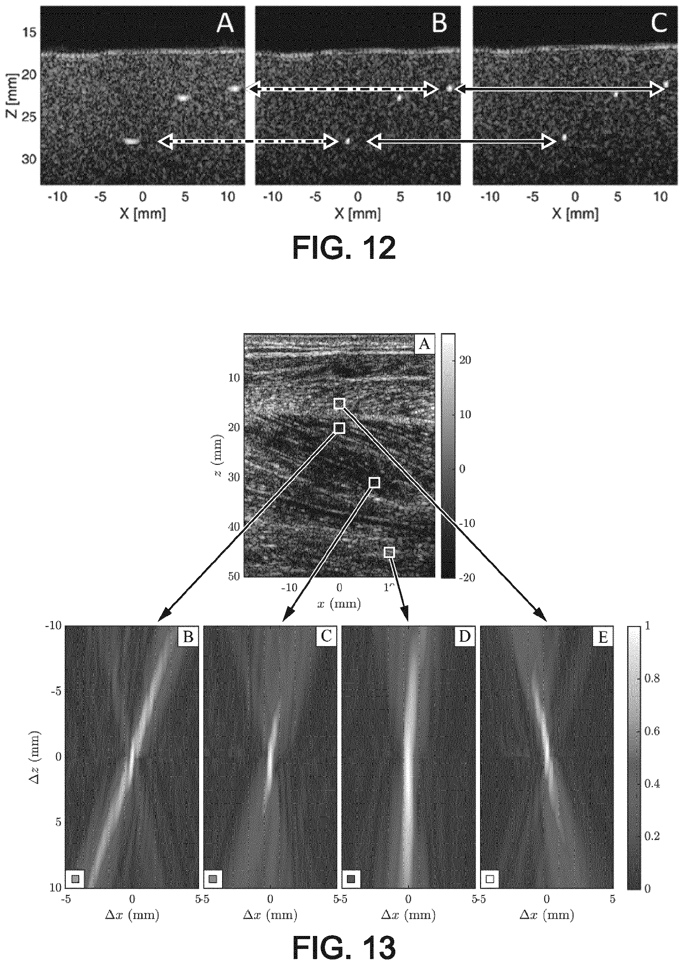

Par exemple, la **figure 12** illustre ce processus. Dans cette figure 12, l'image référencée A correspond à une image échographique obtenue avec une vitesse du son $c_0$ de $c_0$ = 1540 m/s ce qui est la vitesse du son dans le phantom, mais pas celle dans la couche d'eau au-dessus du phantom qui a une vitesse du son de $c_{eau}$ = 1480 m/s. On obtient donc usuellement une image échographique A avec une résolution et un contraste dégradé dus à l'hétérogénéité des milieux étudiés. Des techniques connues de correction d'aberration permettent d'obtenir l'image référencée B améliorée en latéral et qui donne une image de meilleure qualité que les techniques classiques. Cependant, dans cette image les positions en profondeur des éléments réfléchissants ne sont pas corrigées (voir les flèches horizontales entre les images A et B).

L'image référencée C correspond à une image échographique obtenue par la correction axiale proposée dans le procédé présenté ci-dessus. Dans cette image C, les éléments réfléchissants sont légèrement déplacés vers le haut (vers la surface externe), ce qui montre l'influence de la vitesse du son réduite dans l'eau par rapport à celle du phantom. Ainsi, grâce à cette correction axiale, les positions axiales (en profondeur) des points de l'image sont plus proches de la véritable nature du milieu observé et et des distances mesurées dans une telle image sont plus proches des valeurs exactes.

Il est de plus possible d'améliorer la technique de l'une ou l'autre des trois variantes ci-dessus, en déterminant des images du front d'onde améliorées utilisant des combinaisons d'un ensemble d'images du front d'onde et déterminant la vitesse du son à la fois en entrée et en sortie comme explicité précédemment dans la partie détermination de la vitesse du son intégrée, et par exemple par une technique de décomposition en valeur singulières.

La pluralité des images du front d'onde de l'ensemble sont ici traitées par décomposition en valeurs singulières pour combiner plusieurs mesures ou expériences du désordre acoustique d'une région voisine d'un transducteur virtuel d'entrée, ce qui permet très avantageusement d'améliorer le contraste de l'image du front d'onde, et son exploitation.

**Image échographique corrigée en correction des aberrations axiales**

Le procédé de caractérisation ultrasonore pour déterminer une correction axiale et mis en oeuvre par l'unité de calcul 42 du système 40 peut alors être complété en construisant une ou plusieurs images échographiques corrigées, une image échographique corrigée étant déterminée en calculant une valeur d'intensité échographique pour une pluralité de points du milieu correspondant chacun à un transducteur virtuel d'entrée $TV_{in}$ de position spatiale $\mathbf{r_{in}}$ à partir de matrice de réflexion focalisée corrigée $\mathbf{RFoc}^{(1)}(\mathbf{r_{in}}, \mathbf{r_{out}}, \delta t)$ et en imposant un transducteur virtuel de sortie $TV_{out}$ confondu avec le transducteur virtuel d'entrée $TV_{in}$, c'est à dire $\mathbf{r_{in}} = \mathbf{r_{out}}$.

**Détermination d'une direction privilégiée d'anisotropie des diffuseurs du milieu**

Le procédé et système de caractérisation ultrasonore d'un milieu selon la présente divulgation et mis en oeuvre par l'unité de calcul 42 du système 40 est également apte à déterminer localement une direction privilégiée d'une anisotropie des diffuseurs dans le milieu.

L'anisotropie de diffuseur caractérise tout diffuseur étant susceptible de générer des échos dans une direction privilégiée lorsque celui-ci est insonifié selon une direction incidente particulière. Cette anisotropie concerne donc tout diffuseur dont les dimensions sont plus grandes que la longueur d'onde. En particulier on s'intéressera dans le cas de l'imagerie médicale à des fibres, des parois d'organes, des instruments chirurgicaux tels que des aiguilles de biopsies, ...

Dans ce cas, le procédé comprend des étapes similaires ou identiques à celles déjà explicité ci-dessus, jusqu'à :

- une étape de détermination d'une image du front d'onde pour un transducteur virtuel d'entrée $TV_{in}$ et pour un intervalle de délai additionnel, ladite image du front d'onde étant déterminée comme décrit précédemment en fonction d'une vitesse du son $c_0$ dans le milieu.

Le procédé comprend en outre :

- une étape de détermination d'une direction privilégiée de la tache focale dans l'image du front d'onde par traitement d'image de ladite image du front d'onde.

Par exemple, la **figure 13** illustre ce processus. Dans cette figure 13, l'image référencée A correspond à une image échographique avec une variation spatiale de la direction d'anisotropie des tissus. Le milieu imagé dans cette échographie correspond à un muscle d'un patient (un mollet dans cet exemple) dans lequel on observe plusieurs régions avec des fibres inclinées selon des directions très différentes. Cette application à une image d'un muscle n'est qu'un exemple de milieu anisotrope pour lequel le procédé peut être appliqué. Cependant une telle image échographie usuelle donne des informations générales déformées. L'échographie de l'état de l'art ne permet pas une observation fiable de cette anisotropie qui est une caractéristique locale du milieu, car la propagation des ondes ultrasonores dans un tel milieu n'est ni à vitesse constante ni se propageant en direction rectiligne depuis les transducteurs de la sonde.

Les images référencées B, C, D, et E de cette figure 13 correspondent aux images du front d'onde construites pour les petites régions de l'image échographique reliées par les flèches. Ces images du front d'onde sont ici traitées par décomposition en valeurs singulières d'une pluralité de transducteurs virtuels dans chacune de ces régions pour capter ou sonder une pluralité d'expériences du désordre acoustique de cette région et pour ainsi améliorer le contraste de l'image du front d'onde produite, et son analyse.

Toutes ces images du front d'onde B, C, D et E montrent une tache focale très allongée en direction verticale (direction de l'axe $\Delta z$ de profondeur), mais avec une inclinaison différente. Cette inclinaison de la tache focale dans l'image du front d'onde est une information d'inclinaison locale qui est très corrélée avec la valeur réelle d'inclinaison des fibres du muscle dans la région considérée. L'axe d'inclinaison de la tache focale est en effet sensiblement perpendiculaire à la direction des fibres, notamment au centre de l'image, lieux où l'onde incidente a une direction sensiblement dans la direction Z de profondeur.

Ainsi, le procédé détermine la direction privilégiée de la tache focale dans l'image du front d'onde par un traitement d'image sur cette image du front d'onde.

Selon une première variante, le procédé peut par exemple extraire un contour de la tache focale par un seuil à un niveau inférieur la valeur maximum dans cette image du front d'onde, par exemple à 50% ou 70% de ce maximum. De ce contour, on peut déduire la direction privilégiée ou direction principale (la direction de plus grande dimension pour la tache focale), et la direction secondaire (la direction de plus faible dimension). Cependant, d'autres techniques de traitement d'image sont possibles pour extraire la direction privilégiée de la tache focale.

Selon une deuxième variante, le procédé peut par exemple :

- transformer l'image du front d'onde $U(\mathbf{r_{in}}, \Delta x_{out}, \Delta z_{out})$ d'un référentiel en coordonnées cartésiennes vers un référentiel en coordonnées polaires, du type $U(\mathbf{r_{in}}, \Delta s_{out}, \Delta\phi_{out})$
- sommer les valeurs de ladite image du front d'onde du référentiel en coordonnées polaires, sur une pluralité de valeurs d'écarts d'éloignement radial $\Delta s_{out}$, pour obtenir une fonction de sensibilité angulaire $f(\mathbf{r_{in}}, \Delta\phi_{out})$ pour une pluralité de valeurs angulaires $\Delta\phi_{out}$,
- déterminer la valeur angulaire optimale $\Delta\phi^{max}_{out}(\mathbf{r_{in}})$ correspondant au maximum de la fonction de sensibilité angulaire, ladite valeur angulaire optimale $\Delta\phi^{max}_{out}(\mathbf{r_{in}})$ correspondant à la direction privilégiée de la tache focale associée au transducteur virtuel d'entrée $TV_{in}$.

On a ainsi :

$$\Delta\phi_{\text{out}}^{\,max}(\mathbf{r_{in}}) = \max_{\Delta\phi_{\text{out}}}\left[\Sigma_{\Delta s_{\text{in}}}\, U(\mathbf{r_{in}}, \Delta s_{\text{out}}, \Delta\phi_{\text{out}})\right]$$

$$(\text{Equ. 22})$$

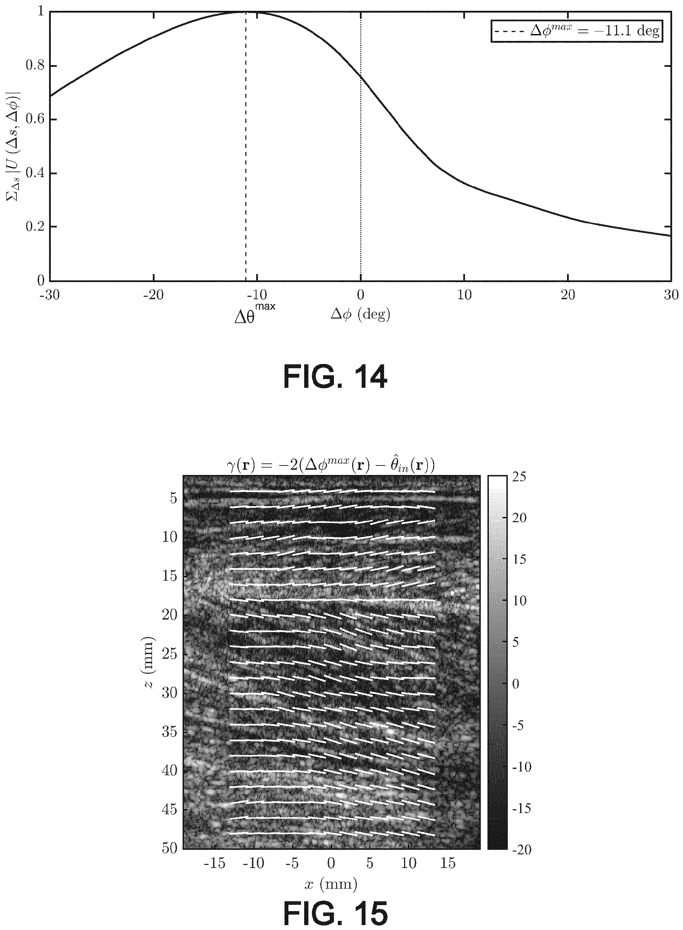

La **figure 14** montre un exemple de courbe de fonction de sensibilité angulaire $f(\mathbf{r_{in}}, \Delta\phi_{out})$ de ladite image du front d'onde du référentiel en coordonnées polaires correspondant à la figure 13B, ladite fonction de sensibilité angulaire étant dans cet exemple illustratif normalisée pour avoir une valeur maximale égale à un (1). Cette courbe présente un maximum vers $\Delta\phi_{out}$ = - 11° qui est l'angle estimé de la direction privilégiée locale au point considéré du milieu de cet exemple.

Eventuellement, on applique à la valeur angulaire $\Delta\phi_{out}$, une correction correspondant à l'angle de vue du point considéré du milieu vu depuis les transducteurs pour obtenir une valeur angulaire d'anisotropie $\gamma_{out}(\mathbf{r_{in}})$, qui est caractéristique de la direction d'anisotropie des diffuseurs localisée à la position spatiale du transducteur virtuel d'entrée.

Cette estimation est ici effectuée à partir de corrélation des signaux en sortie. Réciproquement, on peut estimer une autre valeur angulaire d'anisotropie $\gamma_{in}(\mathbf{r_{out}})$, qui est caractéristique de la direction d'anisotropie des diffuseurs localisée à la position spatiale du transducteur virtuel de sortie. Avantageusement, il est possible de combiner les deux valeurs angulaires d'anisotropie $\gamma_{out}(\mathbf{r_{in}})$ et $\gamma_{in}(\mathbf{r_{out}})$, afin d'obtenir une meilleure caractérisation locale de la direction d'anisotropie du milieu.

Selon un exemple, le procédé eut être complété par les étapes suivantes :

- on intervertit le rôle du ou des transducteurs virtuels d'entrées et du ou des transducteurs virtuels de sortie pour déterminer une direction privilégiée par rapport à un transducteur virtuel de sortie, et
- on combine la direction privilégiée en référence au transducteur virtuel d'entrée et la direction privilégiée en référence au transducteur virtuel de sortie pour obtenir une direction privilégiée améliorée.

Selon un autre exemple, le procédé eut être complété par les étapes suivantes :

- on intervertit le rôle du ou des transducteurs virtuels d'entrées et du ou des transducteurs virtuels de sortie pour déterminer une valeur angulaire d'anisotropie par rapport à un transducteur virtuel de sortie $\gamma_{out}(\mathbf{r_{out}})$, et
- on combine la valeur angulaire d'anisotropie en référence au transducteur virtuel d'entrée $\gamma_{out}(\mathbf{r_{in}})$ et la valeur angulaire d'anisotropie en référence au transducteur virtuel de sortie $\gamma_{out}(\mathbf{r_{out}})$ pour obtenir une valeur angulaire d'anisotropie améliorée.

Un exemple de calcul de valeur angulaire d'anisotropie peut être donné par la formule suivante (dans le premier cas de valeur angulaire d'anisotropie en référence au transducteur virtuel d'entrée $Y_{out}(\mathbf{r_{in}})$) :

$$\gamma_{\text{out}}(\mathbf{r_{in}}) = -2(\Delta\phi\,_{\text{out}}^{\,max}(\mathbf{r_{in}}) - \hat{\theta}_{\text{out}}(\mathbf{r_{in}}))$$

$$(Equ.\ 23)$$

Un tel calcul de valeur angulaire d'anisotropie provient par exemple de calculs explicités dans le document *"Specular Beamforming"*, Alfonso Rodriguez-Molares et Al., publié dans IEEE Transactions on Ultrasonics, Ferroelectrics, and Frequency Control (Volume: 64, Issue: 9, Sept. 2017*)*.

On ajoute une définition d'un angle de vision du point de position spatiale $\mathbf{r_{in}}$ du transducteur virtuel d'entrée, par exemple du type :

$$\hat{\theta}_{\text{out}}(\mathbf{r_{in}}) = \frac{1}{\sum_{u_{\text{out}}^{-}}^{u_{\text{out}}^{+}} \cos\big(\theta_{\text{out}}(\mathbf{r_{in}})\big)} \sum_{u_{\text{out}}^{-}}^{u_{\text{out}}^{+}} \theta_{\text{out}}(\mathbf{r_{in}}) \cos\big(\theta_{\text{out}}(\mathbf{r_{in}})\big)$$

$$(Equ.\ 24)$$

avec

$$\theta_{\text{out}}(\mathbf{r_{in}}) = \text{atan}\left(\frac{u_{out} - x_{in}}{z_{in}}\right)$$

dans lequel: $u_{\text{out}}^{\pm}(\mathbf{r})$ sont les valeurs maximale et minimale de positions spatiales de transducteurs du réseau.

D'autres formules de calcul de valeur angulaire d'anisotropie sont envisageables pour le technicien du domaine afin d'obtenir des valeurs plus réalistes d'angle de direction privilégiée.

La **figure 15** montre une image échographique sur laquelle sont superposés des traits correspondant à des estimations de directions privilégiées pour un ensemble de points répartis sur l'image échographique. On constate une grande cohérence entre les directions privilégiées estimées et les structures sous-jacentes visibles sur l'image échographique. Le procédé proposé permet avantageusement d'estimer de façon adéquate les directions privilégiées sur la totalité de l'image échographique.

La mesure de cette direction privilégiée, angle d'inclinaison de la tache focale pour sa plus grande dimension, est un paramètre important pour améliorer la qualité de l'image échographique dans cette région : Sa connaissance peut permettre d'adapter les caractéristiques des ondes ultrasonores incidentes $US_{in}$ ; par exemple en choisissant des ondes planes avec une inclinaison spécifique ou des ondes focalisées en un lieux spécifique. Cela permet aussi d'adapter les apodisations choisies en réceptions lors de l'étape de formation de voie.

La mesure de cette direction privilégiée locale peut permettre d'analyser le degré d'anisotropie d'une plus grande région, et ainsi de déterminer l'existence potentielle de lésions dans le tissu et de les localiser.

Ainsi, le procédé de caractérisation ultrasonore d'un milieu, pour déterminer localement une direction privilégiée d'une anisotropie, comprend les étapes suivantes :

- une étape de génération d'une série d'ondes ultrasonores incidentes $US_{in}$ dans une zone dudit milieu, au moyen d'un réseau 10 de transducteurs 11, ladite série d'ondes ultrasonores incidentes étant une base d'émission i ; et
- une étape de génération d'une matrice de réflexion expérimentale $\mathbf{R_{ui}}(t)$ définie entre la base d'émission i en entrée et une base de réception u en sortie ;
- une étape de détermination d'une matrice de réflexion focalisée $\mathbf{RFoc(r_{in}, r_{out}}, \delta t)$ qui comprend des réponses du milieu entre un transducteur virtuel d'entrée $TV_{in}$ de position spatiale $\mathbf{r_{in}}$ et un transducteur virtuel de sortie $TV_{out}$ de position spatiale $\mathbf{r_{out}}$, les réponses du transducteur de virtuel de sortie $TV_{out}$ étant prises à un instant temporel décalé d'un délai additionnel $\delta t$ par rapport à un instant temporel des réponses du transducteur virtuel d'entrée $TV_{in}$,
- une étape de détermination d'une image du front d'onde pour le transducteur virtuel d'entrée $TV_{in}$ et pour un intervalle de délai additionnel, ladite image du front d'onde étant déterminée en fonction de la vitesse du son $c_0$ dans le milieu, et ladite image du front d'onde étant déterminée à partir :

  - de la matrice de réflexion focalisée $\mathbf{RFoc(r_{in}, r_{out}}, \delta t)$ et
  - d'une relation de propagation balistique du type

$$\delta t(\Delta \mathbf{r_{out}}) = -sign(\Delta z_{out}) \cdot |\Delta \mathbf{r_{out}}|/c_0,$$

  qui permet d'extraire des valeurs de la matrice de réflexion focalisée pour construire l'image du front d'onde, et dans laquelle :

    $\delta t$ est le délai additionnel,
    $|\Delta \mathbf{r_{out}}|$ est le module du vecteur entre le transducteur virtuel d'entrée $TV_{in}$ et le transducteur virtuel de sortie $TV_{out}$, avec $\Delta \mathbf{r_{out}} = \mathbf{r_{out}} - \mathbf{r_{in}}$,
    $\Delta z_{out}$ est la composante selon un axe Z de profondeur du vecteur de position spatiale $\Delta \mathbf{r_{out}}$,

- une étape de détermination d'une direction privilégiée de la tache focale dans l'image du front d'onde par traitement d'image de ladite image du front d'onde.

Il est possible d'améliorer la technique proposée en déterminant des images du front d'onde améliorées utilisant des combinaisons d'un ensemble d'images du front d'onde comme explicité précédemment dans la partie détermination de la vitesse du son intégrée, et par exemple par une technique de décomposition en valeur singulières. Dans ce cas, la direction privilégiée obtenue à partir d'une image du front d'onde améliorée permet de caractériser l'anisotropie du milieu correspondant à une zone de cohérence choisie et est attribuée à la position spatiale $\mathbf{r_{in,ref}}$ du transducteur virtuel de référence.

La pluralité des images du front d'onde de l'ensemble sont ici traitées par décomposition en valeurs singulières pour combiner plusieurs mesures ou expériences du désordre acoustique d'une région voisine d'un transducteur virtuel d'entrée, ce qui permet d'améliorer le contraste de l'image du front d'onde, et ainsi son exploitation.

Dans le procédé, on peut ainsi ajouter les étapes suivantes :

- entre l'étape de détermination d'une image du front d'onde et l'étape de détermination de la direction privilégiée de la tache focale, on effectue une étape d'amélioration de l'image du front d'onde dans laquelle on effectue une combinaison linéaire d'un ensemble d'images du front d'onde correspondant à une zone de cohérence, chaque image du front d'onde étant prise entre un transducteur virtuel d'entrée ($TV_{in}$) choisi de position spatiale $\mathbf{r_{in}}$ différente, et des transducteurs virtuels de sortie ($TV_{out}$) de position spatiale $\mathbf{r_{out}}$ tels que $\mathbf{r_{out}} = \Delta \mathbf{r_{out}} + \mathbf{r_{in}}$, avec $\Delta \mathbf{r_{out}}$ étant prédéfinis et identiques pour toutes les images du front d'onde de l'ensemble, et les transducteurs virtuels d'entrée choisis étant voisins les uns des autres, pour obtenir une image du front d'onde améliorée associé à un transducteur virtuel d'entrée de référence ($TV_{in,ref}$), ce transducteur virtuel d'entrée de référence $TV_{in,ref}$ étant caractéristique des transducteurs virtuels d'entrée de l'ensemble des images du front d'onde utilisées et associées à la zone de cohérence ZC, et
- dans l'étape de détermination d'une direction privilégiée de la tache focale, l'image du front d'onde améliorée est utilisée à la place de l'image du front d'onde, la direction privilégiée de la tache focale est relative à la position spatiale du transducteur virtuel d'entrée de référence TV$_{in,ref}$.

En outre, en inversant le rôle des transducteurs virtuels d'entrée TV$_{in}$ et de sortie TV$_{out}$, c'est-à-dire, en inversant les notations "in" et "out", il est possible de déterminer la direction privilégiée $\Delta\phi^{max}{}_{in}(\mathbf{r_{out}})$ de la tache focale associée au transducteur virtuel de sortie TV$_{out}$ de position spatiale $\mathbf{r_{out}}$. La combinaison des deux directions privilégiés associées à la position r, c'est-à-dire $\Delta\phi^{max}{}_{in}(\mathbf{r})$ et $\Delta\phi^{max}{}_{out}(\mathbf{r})$ permet d'améliorer la mesure d'anisotropie de diffuseur.

Grâce à ce calcul de direction privilégiée et de ces images, on peut caractériser l'anisotropie des diffuseurs du milieu, ou caractériser par exemple une structure anisotrope dans le milieu, telle qu'une aiguille introduite dans un tissus, ou une paroi séparant des tissus différents. On comprend par anisotropie de diffuseurs, tout élément plus grand que la longueur d'onde des ondes ultrasonores.

**Analyse des signaux temporels pour des points confocaux**

Le procédé et système de caractérisation ultrasonore d'un milieu selon la présente divulgation et mis en oeuvre par l'unité de calcul 42 du système 40 est également apte à effectuer une analyse spectrale locale d'une focalisation ultrasonore.

Dans une telle analyse, on s'intéresse en particulier aux réponses confocales, c'est à dire à un transducteur virtuel d'entrée TV$_{in}$ de position spatiale $\mathbf{r_{in}}$ superposé au transducteur virtuel de sortie TVout de position spatiale $\mathbf{r_{out}}$; c'est à dire avec $\mathbf{r_{in}} = \mathbf{r_{out}} = \mathbf{r}$.

Le délai additionnel $\delta t$ est alors utilisé pour sonder la réponse temporelle des diffuseurs sélectionnés par ces transducteurs virtuels.

Dans ce cas, le procédé comprend les étapes suivantes déjà explicités pour obtenir une matrice de réflexion focalisée, mais appliquées à une même position spatiale, c'est à dire à une position confocale :

- une étape de génération d'une série d'ondes ultrasonores incidentes US$_{in}$ dans une zone dudit milieu, au moyen d'un réseau 10 de transducteurs 11, ladite série d'ondes ultrasonores incidentes étant une base d'émission **i** ; et
- une étape de génération d'une matrice de réflexion expérimentale $\mathbf{R_{ui}}(t)$ définie entre la base d'émission **i** en entrée et une base de réception **u** en sortie ;
- une étape de détermination d'une matrice de réflexion focalisée $\mathbf{RFoc(r}, \delta t)$ qui comprend des réponses du milieu entre un transducteur virtuel d'entrée TV$_{in}$ de position spatiale $\mathbf{r_{in}}$ et un transducteur virtuel de sortie TV$_{out}$ de position spatiale $\mathbf{r_{out}}$, les transducteurs virtuels d'entrée et de sortie étant superposés à la même position spatiale $\mathbf{r}$, avec $\mathbf{r_{in}} = \mathbf{r_{out}} = \mathbf{r}$, et les réponses du transducteur de virtuel de sortie TV$_{out}$ étant prises à un instant temporel décalé d'un délai additionnel $\delta t$ par rapport à un instant temporel des réponses du transducteur virtuel d'entrée TV$_{in}$.

Le procédé comprend ensuite les étapes suivantes permettant d'effectuer l'analyse spectrale locale :

- une étape de détermination d'une matrice fréquentielle $\mathbf{RFreq_t(r}, \omega)$ qui est une transformée de Fourier temporelle de la matrice de réflexion focalisée $\mathbf{RFoc(r}, \delta t)$

$$RFreq_t(\mathbf{r},\omega) = TF_t[RFoc(\mathbf{r},\delta t)]$$

(Equ. 25)

dans laquelle

TF$_t$ est la transformée de Fourier temporelle, et
$\omega$ est une pulsation avec $\omega = 2\pi f$, f étant la fréquence correspondant à ladite pulsation.

La transformée de Fourier temporelle peut être explicitée par exemple par la formule de transformée de Fourier discrète temporelle suivante :

$$RFreq_t(r,\omega) = TF_t[RFoc(r,\delta t)] = \sum_{\Delta\omega} RFoc(\mathbf{r},\delta t)e^{-i\omega\,\delta t}$$

(Equ. 26)

D'autres formulations de transformé de Fourier et transformée de Fourier temporelle existent, par exemple sous forme discrète ou intégrale, avec ou sans normalisation, et peuvent aussi être utilisées.

$RFreq_t(\mathbf{r}, \omega)$ contient alors une estimation locale du spectre des échos rétrodiffusés par le milieu. Plus précisément, ces échos proviennent de diffuseurs qui sont compris dans la tache focale monochromatique centrée sur la position r. En absence d'aberration, ces dimensions sont donc prévues par les limites de la diffraction définies à la fréquence centrale des échos rétrodiffusés par le milieu.

Ce procédé peut donc être complété par toute technique d'imagerie médicale basée sur une analyse fréquentielle des échos rétrodiffusés afin d'améliorer la résolution spatiale. Plus précisément, ce processus permet d'effectuer une formation de voie spatiale en réception pour chaque fréquence, avant d'effectuer une quelconque analyse spectrale. Notons que la configuration confocale permet avantageusement de limiter les phénomènes de diffractions impulsionnels.

Par exemple, ce procédé peut être complété par une étape de filtrage durant laquelle on effectue un filtrage en fréquence des éléments de la matrice fréquentielle $RFreq_t(\mathbf{r}, \omega)$. Notamment, il est possible d'effectuer un filtrage en fréquence passe-bas, passe-bande ou passe haut, pour extraire des composantes souhaitées dans les réponses de la matrice de réflexion focalisée, en fonction de l'application visée. Par exemple, le filtrage en fréquence peut être éventuellement adapté pour extraire des composantes harmoniques d'une fréquence fondamentale des ondes ultrasonores incidentes $US_{in}$.

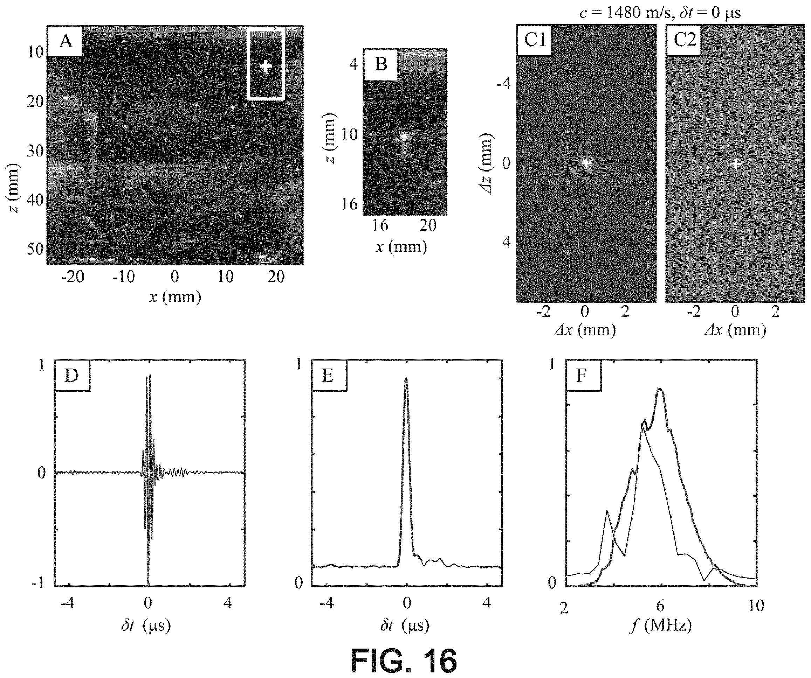

Par exemple, la **figure 16** illustre ce processus. Dans cette figure 16, l'image référencée A illustre une image échographique d'un milieu comprenant des bulles. Ces bulles sont des structures résonantes dans le milieu qui perturbent l'image ultrasonore, car elles continuent à osciller après le passage d'une onde incidente. Elles génèrent ainsi des échos qui parviennent sur les transducteurs de réception avec un temps de vol supérieur au temps de vol balistique, ce qui produit des artéfacts dans l'image échographique en aval de la bulle. L'image référencée B de la figure 14 montre un agrandissement de l'image A dans lequel on observe un écho brillant d'une bulle à la position spatiale $\mathbf{r} = [x, z] = [11, 17]$ mm, et de son artéfact en aval situé en dessous de cette position (i.e. verticalement en profondeur) Les images référencées C1 et C2 correspondent à l'image de propagation respectivement en amplitude et en partie réelle, pour un délai additionnel δt nul.

La matrice de réflexion focalisée $RFoc(\mathbf{r}, \delta t)$ du procédé permet d'étudier les signaux temporels de l'oscillation de cette bulle. Les images référencées D-E-F de la figure 14 correspondent respectivement au tracés de la partie réelle, l'amplitude et du spectre fréquentiel de laa réponse $RFoc(\mathbf{r}, \delta t)$ au point de position spatiale r correspondant à la position de cette bulle. Sur les images D et E, on observe un second écho vers 1,5 μs après l'écho principal centré à 8t = 0. L'image F montre un premier tracé de spectre excluant ce second écho et un deuxième tracé de spectre avec ce second écho. Ce deuxième tracé de spectre comprend une fréquence principale autour de 6 MHz qui correspond à la fréquence de l'onde incidente, et une autre fréquence vers 3 MHz qui correspond à la fréquence de résonance (oscillation) de la bulle.

Le procédé effectue ainsi une analyse spectrale qui permet par exemple d'identifier des fréquences de résonances de bulles ou de toute autre structure résonante dans le milieu observé.

Il est ainsi possible de filtrer, par exemple par un filtre passe-bande prédéterminé, les réponses de la matrice de réflexion focalisée, et alors de calculer une image échographique à l'aide de ces réponses filtrées qui sera améliorée. L'effet des résonances peut alors être atténué ou supprimé dans l'image échographique.

Réciproquement, il est possible de construire des images de fréquence de résonance en conservant uniquement ces résonances dans les réponses de la matrice de réflexion focalisée. Notons, que la fréquence de résonance d'une bulle est liée à sa taille, et peut permettre d'estimer une pression locale dans le milieu.

Dans un second exemple, $RFreq_t(\mathbf{r}, \omega)$ peut être utilisé afin d'étudier l'atténuation du milieu. En effet, ce phénomène dépend de la fréquence. Les hautes fréquences étant plus atténuée que les basses fréquences, il est possible d'en déduire un coefficient d'atténuation par exemple en comparant le spectre des échos provenant de deux profondeurs différentes dans le milieu observé. La technique décrite ci-dessus permettant d'estimer le spectre local des échos provenant d'une zone donnée est donc tout indiqué pour la détermination de l'atténuation. Pour cela, le procédé peut être par exemple complété avec une étape de détermination d'un spectre moyen à une profondeur $S(z, \omega)$ déterminé par une moyenne des spectres de la matrice fréquentielle à une profondeur z prédéterminée dans le milieu.

Par exemple, ce spectre moyen à une profondeur est calculé par la formule suivante, qui est une moyenne normalisée, moyenne effectuée sur un ensemble de positions spatiales de même profondeur z et de coordonnée latérale x comprise sur un intervalle prédéterminé.

$$S(z, \omega) = \langle \frac{RFreq_t(\mathbf{r}, \omega)}{\max_{\omega}[RFreq_t(\mathbf{r}, \omega)]} \rangle_x$$

(Equ. 27)

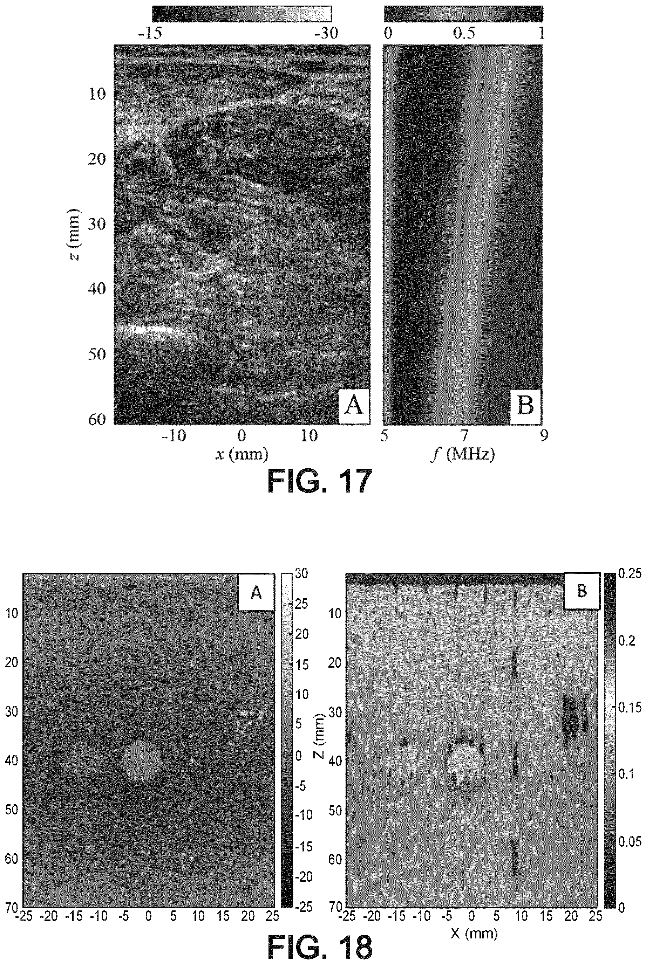

Par exemple, la **figure 17** illustre ce calcul de spectre moyen en profondeur, en construisant une image de l'ensemble des spectres pour toutes les profondeurs d'une image échographique. Dans cette figure 17, l'image référencée A illustre une image échographique *in-vivo* du mollet d'un individu sain et l'image référencée B présente les spectres moyens en profondeurs avec une échelle de niveau de gris. Cette image de spectres en profondeurs montre la plus forte atténuation des hautes fréquences pour les grandes profondeurs.

A l'aide d'une telle image, on peut estimer l'évolution de l'atténuation en fonction de la profondeur grâce à l'ensemble du contenu fréquentiel par des technique d'ajustement entre un modèle théorique et/ou expérimental, et une telle image.

Dans un troisième exemple, le procédé peut aussi être complété avec une étape de détermination de largeur spectrale de corrélation $\delta\omega(\mathbf{r})$ pour le point de position spatiale r, par un calcul de largeur à mi-hauteur de l'autocorrélation de chaque spectre de la matrice fréquentielle $\mathbf{RFreq_t}(\mathbf{r}, \omega)$, c'est à dire par la formule suivante :

$$\delta\omega(\mathrm{r}) = FWHM(\frac{1}{\Delta\omega}\int_{\omega^-}^{\omega^+} RFreq_t(r,\omega)RFreq_t^\star(r,\omega+d\omega)\ d\omega$$

$$(\mathrm{Equ.}\ 28)$$

dans laquelle

FWHM est la fonction de calcul de largeur à mi-hauteur
()* est la fonction de conjugaison complexe,
$\omega^-$ et $\omega^+$ qui sont des pulsations limites, $\Delta\omega = \omega^+ - \omega^-$ est l'intervalle des pulsations limites, i.e. la bande passante considérée des ondes ultrasonores.

Grâce à la résolution spatiale de la matrice $\mathbf{RFreq_t}(\mathbf{r}, \omega)$, la largeur spectrale de corrélation $\delta\omega(\mathbf{r})$ est une valeur locale qui peut permet de caractériser la nature des diffuseurs contenu dans la tache focale monochromatique centrée sur la position spatiale r. Si la tache focale contient un unique diffuseur non résonant, la largeur spectrale de corrélation $\delta\omega(\mathbf{r})$ est de l'ordre de grandeur de la bande passante du signal ultrasonore. Si la tache focale contient un ensemble de diffuseurs aléatoirement distribués et de même intensité (régime du speckle ultrasonore), la valeur de largeur spectrale de corrélation $\delta\omega(\mathbf{r})$ devient bien plus petite que la bande passante $\Delta\omega$.

Le procédé peut comprendre aussi une étape de détermination d'au moins une image de corrélation spectrale, ladite image de corrélation spectrale étant obtenue en déterminant les largeurs spectrales $\delta\omega(\mathbf{r})$ pour une pluralité de points du milieu correspondant chacun à un point du milieu de position spatiale r.

Par exemple la **figure 18** illustre ce processus. Dans cette figure 18, l'image référencée A est une image échographique d'un milieu phantom contenant plusieurs éléments différents : des cibles ponctuelles et un cylindre échogène. L'image référencée B correspondante est l'image de corrélation spectrale de l'image échographique précédente et obtenue par calcul de la largeur spectrale de corrélation $\delta\omega(\mathbf{r})$ pour un ensemble de point de ce milieu. Dans cette image B, les bords du cylindre et les cibles ponctuelles ont une largeur spectrale de corrélation $\delta\omega(\mathbf{r})$ plus grande que le reste du milieu qui est constitué d'un grand nombre de diffuseurs sous-résolus répartis aléatoirement.

Grâce à ce calcul de largeur spectrale de corrélation et de ces images, on peut caractériser la nature des cibles dans le milieu. Par exemple, il est possible de différencier entre un grain de speckle brillant et un diffuseur unique. Par exemple cela peut aider à l'identification de bulles pour l'imagerie de contraste, ou de micro-calcifications caractéristiques de la présence de tumeurs notamment dans le cas du cancer du sein.

**Revendications**

1. **Procédé de caractérisation ultrasonore d'un milieu,** pour déterminer localement une direction privilégiée d'une anisotropie des diffuseurs dans le milieu, le procédé comprenant :

- une étape de génération d'une série d'ondes ultrasonores incidentes ($US_{in}$) dans une zone dudit milieu, au moyen d'un réseau (10) de transducteurs (11), ladite série d'ondes ultrasonores incidentes étant une base d'émission (i) ; et
- une étape de génération d'une matrice de réflexion expérimentale $\mathbf{R_{ui}}(t)$ définie entre la base d'émission **(i)** en entrée et une base de réception **(u)** en sortie ;
- une étape de détermination d'une matrice de réflexion focalisée $\mathbf{RFoc}(\mathbf{r_{in}}, \mathbf{r_{out}}, 8t)$ qui comprend des réponses du milieu entre un transducteur virtuel d'entrée ($TV_{in}$) de position spatiale $\mathbf{r_{in}}$ et un transducteur virtuel de sortie

(TV$_{out}$) de position spatiale **r$_{out}$**, les réponses du transducteur de virtuel de sortie (TV$_{out}$) étant prises à un instant temporel décalé d'un délai additionnel δt par rapport à un instant temporel des réponses du transducteur virtuel d'entrée (TV$_{in}$),

- une étape de détermination d'une image du front d'onde pour le transducteur virtuel d'entrée (TV$_{in}$) et pour un intervalle de délai additionnel, ladite image du front d'onde étant déterminée en fonction de la vitesse du son $c_0$ dans le milieu, et ladite image du front d'onde étant déterminée à partir :

    - de la matrice de réflexion focalisée **RFoc(r$_{in}$, r$_{out}$**, δt) et
    - d'une relation de propagation balistique du type

$$\delta t(\Delta \mathbf{r_{out}}) = -sign(\Delta z_{out}) \cdot |\Delta \mathbf{r_{out}}|/c_0,$$

qui permet d'extraire des valeurs de la matrice de réflexion focalisée pour construire l'image du front d'onde, et dans laquelle :

    δt est le délai additionnel,
    $|\Delta \mathbf{r_{out}}|$ est le module du vecteur entre le transducteur virtuel d'entrée (TV$_{in}$) et le transducteur virtuel de sortie (TV$_{out}$), avec $\Delta \mathbf{r_{out}} = \mathbf{r_{out}} - \mathbf{r_{in}}$ ,
    $\Delta z_{out}$ est la composante selon un axe Z de profondeur du vecteur de position spatiales $\Delta \mathbf{r_{out}}$.
    ladite image du front d'onde comprenant une tache focale,

- une étape de détermination d'une direction privilégiée de la tache focale dans l'image du front d'onde par traitement d'image de ladite image du front d'onde, ladite direction privilégiée correspondant à une direction de plus grande dimension pour ladite tache focale.

2. Procédé selon la revendication 1, dans lequel le traitement d'image comprend une extraction du contour de la tache focale, par un seuil à un niveau inférieur à la valeur maximum dans l'image du front d'onde, ledit contour servant à déterminer la direction privilégiée de la tache focale.

3. Procédé selon la revendication 1, dans lequel :

- on transforme l'image du front d'onde U(**r$_{in}$**, $\Delta x_{out}$, $\Delta z_{out}$) d'un référentiel en coordonnées cartésiennes vers un référentiel en coordonnées polaires U(**r$_{in}$**, $\Delta s_{out}$, $\Delta \phi_{out}$),
- on somme les valeurs de ladite image du front d'onde du référentiel en coordonnées polaires, sur une pluralité de valeurs d'écarts d'éloignement radial $\Delta s_{out}$ pour obtenir une fonction de sensibilité angulaire f(**r$_{in}$**, $\Delta \phi_{out}$) pour une pluralité de valeurs angulaires $\Delta \phi_{out}$,
- on détermine la valeur angulaire optimale $\Delta \phi^{max}_{out}$(**r$_{in}$**) correspondant au maximum de fonction de sensibilité angulaire, ladite valeur angulaire optimale $\Delta \phi^{max}_{out}$(**r$_{in}$**) correspondant à la direction privilégiée de la tache focale associée au transducteur virtuel d'entrée (TV$_{in}$).

4. Procédé selon l'une des revendications 1 à 3, dans lequel la direction privilégiée est corrigée par un angle de vue du point de position spatiale **r$_{in}$** du transducteur virtuel d'entrée (TVin), vu depuis le réseau de transducteur (10).

5. Procédé selon la revendication 4, dans lequel la direction privilégiée est corrigée par la formule :

$$\gamma_{out}(\mathbf{r_{in}}) = -2(\Delta \phi \, ^{max}_{out}(\mathbf{r_{in}}) - \hat{\theta}_{out}(\mathbf{r_{in}}))$$

dans laquelle :

$\Delta \phi \, ^{max}_{out}(\mathbf{r_{in}})$ est la valeur de la direction privilégiée,
$\theta_{out}$(**r$_{in}$**) est la correction de l'angle de vue,
$\gamma_{out}$(**r$_{in}$**) est la valeur angulaire d'anisotropie .

6. Procédé selon la revendication 5, dans lequel la correction de l'angle de vue est :

$$\hat{\theta}_{\text{out}}(\mathbf{r_{in}}) = \frac{1}{\sum_{u_{\text{out}}^-}^{u_{\text{out}}^+} \cos(\theta_{\text{out}}(\mathbf{r_{in}}))} \sum_{u_{\text{out}}^-}^{u_{\text{out}}^+} \theta_{\text{out}}(\mathbf{r_{in}}) \cos(\theta_{\text{out}}(\mathbf{r_{in}}))$$

avec

$$\theta_{\text{out}}(\mathbf{r_{in}}) = \text{atan}\left(\frac{u_{out} - x_{in}}{z_{in}}\right)$$

dans laquelle : $u_{\text{out}}^{\pm}(\mathbf{r})$ sont les valeurs maximale et minimale de positions de transducteurs dans le réseau.

**7.** Procédé selon l'une des revendications 1 à 6, dans lequel :

- on intervertit le rôle du ou des transducteurs virtuels d'entrées et du ou des transducteurs virtuels de sortie pour déterminer une direction privilégiée par rapport à un transducteur virtuel de sortie, et
- on combine la direction privilégiée en référence au transducteur virtuel d'entrée et la direction privilégiée en référence au transducteur virtuel de sortie pour obtenir une direction privilégiée améliorée.

**8.** Procédé selon l'une des revendications 5 à 6, dans lequel :

- on intervertit le rôle du ou des transducteurs virtuels d'entrées et du ou des transducteurs virtuels de sortie pour déterminer une valeur angulaire d'anisotropie par rapport à un transducteur virtuel de sortie $\gamma_{\text{out}}(\mathbf{r_{out}})$, et
- on combine la valeur angulaire d'anisotropie en référence au transducteur virtuel d'entrée $\gamma_{\text{out}}(\mathbf{r_{in}})$ et la valeur angulaire d'anisotropie en référence au transducteur virtuel de sortie $\gamma_{\text{out}}(\mathbf{r_{out}})$ pour obtenir une valeur angulaire d'anisotropie améliorée.

**9.** Procédé selon l'une des revendications 1 à 8, dans lequel :

- entre l'étape de détermination d'une image du front d'onde et l'étape de détermination de la direction privilégiée de la tache focale, on effectue une étape d'amélioration de l'image du front d'onde dans laquelle on effectue une combinaison linéaire d'un ensemble d'images du front d'onde correspondant à une zone de cohérence, chaque image du front d'onde étant prise entre un transducteur virtuel d'entrée ($TV_{\text{in}}$) choisi de position spatiale $\mathbf{r_{in}}$ différente, et des transducteurs virtuels de sortie ($TV_{\text{out}}$) de position spatiale $\mathbf{r_{out}}$ tels que $\mathbf{r_{out}} = \Delta\mathbf{r_{out}} + \mathbf{r_{in}}$, avec $\Delta\mathbf{r_{out}}$ étant prédéfinis et identiques pour toutes les images du front d'onde de l'ensemble, et les transducteurs virtuels d'entrée choisis étant voisins les uns des autres, pour obtenir une image du front d'onde améliorée associé à un transducteur virtuel d'entrée de référence ($TV_{\text{in,ref}}$), ce transducteur virtuel d'entrée de référence $TV_{\text{in,ref}}$ étant caractéristique des transducteurs virtuels d'entrée de l'ensemble des images du front d'onde utilisées et associées à la zone de cohérence ZC, et
- dans l'étape de détermination d'une direction privilégiée de la tache focale, l'image du front d'onde améliorée est utilisée à la place de l'image du front d'onde, la direction privilégiée de la tache focale est relative à la position spatiale du transducteur virtuel d'entrée de référence $TV_{\text{in,ref}}$.

**10.** Procédé selon la revendication 9, dans lequel la combinaison linéaire est déterminée par un calcul de décomposition en valeur singulière (SVD) de l'ensemble des images du front d'onde pour obtenir un vecteur singulier **(W₁)** associé à la valeur singulière de décomposition en valeur singulière de plus grande valeur absolue, ce vecteur singulier **(W₁)** étant alors l'image du front d'onde améliorée correspondant audit transducteur virtuel d'entrée de référence ($TV_{\text{in,ref}}$) et pour les mêmes délais additionnels $\delta t$.

**11.** Procédé selon l'une des revendications 1 à 10, dans lequel à l'étape de détermination de la matrice de réflexion focalisée :

le calcul des réponses du transducteur virtuel d'entrée ($TV_{\text{in}}$) correspond à un processus de focalisation en entrée à partir de la matrice de réflexion expérimentale $\mathbf{R_{ui}}(t)$ qui utilise un temps de vol à l'aller des ondes entre la base d'émission et le transducteur virtuel en entrée (TV$_{in}$) pour créer une tache focale d'entrée à la position spatiale **r$_{in}$,**

le calcul des réponses du transducteur virtuel de sortie (TV$_{out}$) correspond à un processus de focalisation en sortie à partir de la matrice de réflexion expérimentale **R$_{ui}$**(t) qui utilise un temps de vol de retour des ondes entre le transducteur virtuel de sortie (TV$_{out}$) et les transducteurs de la base de réception **u,** pour créer une tache focale de sortie à la position spatiale **r$_{out}$,**

le délai additionnel 8t étant un délai temporel ajouté aux temps de vols à l'aller et de retour lors des processus de focalisation.

12. Procédé selon l'une des revendications 1 à 11, dans lequel la matrice de réflexion focalisée est calculée par la formule suivante :

$$\mathbf{RFoc}(\mathbf{r_{in}}, \mathbf{r_{out}}, \delta t) = \frac{1}{N_{in}N_{out}} \sum_{i_{in}} \sum_{u_{out}} \mathbf{R_{ui}}\big(\mathbf{u_{out}}, \mathbf{i_{in}}, \tau(\mathbf{r_{in}}, \mathbf{r_{out}}, \mathbf{u_{out}}, \mathbf{i_{in}}, \delta t)\big)$$

dans laquelle

N$_{in}$ est le nombre d'éléments de la base d'émission **(i),**

N$_{out}$ est le nombre d'éléments de la base de réception **(u)** en sortie,

**R$_{ui}$**(t) est la matrice de réflexion expérimentale, dont **R$_{ui}$**(**u$_{out}$** , **i$_{in}$,** $\tau$(**r$_{in}$, r$_{out}$, u$_{out}$, i$_{in}$,** $\delta t$)) est l'élément de la matrice de réflexion expérimentale **R$_{ui}$**(t) enregistré par le transducteur de position spatiale **u$_{out}$** consécutif à l'émission d'indice **i$_{in}$** dans la base d'émission et au temps $\tau$,

$\tau$ est un temps qui est la somme du temps de vol à l'aller $\tau_{in}$ de l'onde ultrasonore entre les transducteurs de la base d'émission (i) et le transducteur virtuel d'entrée (TV$_{in}$) de position spatiale **r$_{in}$,** et du temps de vol au retour $\tau_{out}$ de l'onde ultrasonore entre le transducteur de sortie (TV$_{out}$) de position spatiale **r$_{out}$** et les transducteurs de la base de réception **u,** et du délai additionnel $\delta t$, comme explicité par la formule suivante :

$$\tau(\mathbf{r_{in}}, \mathbf{r_{out}}, \mathbf{u_{out}}, \mathbf{i_{in}}, \delta t) = \tau_{in}(\mathbf{r_{in}}, \mathbf{i_{in}}) + \tau_{out}(\mathbf{r_{out}}, \mathbf{u_{out}}) + \delta t$$

13. **Système (40) de caractérisation ultrasonore d'un milieu (20),** pour déterminer localement une direction privilégiée d'une anisotropie des diffuseurs dans le milieu, le système comprenant :

- un réseau (10) de transducteurs adaptés pour générer une série d'ondes ultrasonores incidentes dans une zone du milieu, et pour enregistrer en fonction du temps les ondes ultrasonores rétrodiffusées par ladite zone ; et
- une unité de calcul (42) reliée au réseau de transducteurs et adaptée pour mettre en oeuvre le procédé selon l'une des revendications précédentes.

**Patentansprüche**

1. **Verfahren zur Ultraschallcharakterisierung eines Mediums,** um lokal eine Vorzugsrichtung einer Anisotropie von Diffusoren in dem Medium zu bestimmen,

wobei das Verfahren Folgendes umfasst:

- einen Schritt des Erzeugens einer Reihe von einfallenden Ultraschallwellen (US$_{in}$) in einem Bereich des Mediums mittels einer Anordnung (10) von Wandlern (11), wobei die Reihe von einfallenden Ultraschallwellen eine Emissionsbasis (i) ist; und
- einen Schritt des Erzeugens einer experimentellen Reflexionsmatrix **R$_{ui}$**(t), die zwischen der Emissionsbasis (i) am Eingang und einer Rezeptionsbasis (u) am Ausgang definiert ist,
- einen Schritt der Bestimmung einer fokussierten Reflexionsmatrix **RFoc(r$_{in}$, r$_{out}$,** $\delta t$), die Antworten des Mediums zwischen einem virtuellen Eingangswandler (TV$_{in}$) mit der räumlichen Position **r$_{in}$** und einem virtuellen Ausgangswandler (TV$_{out}$) mit der räumlichen Position **r$_{out}$** umfasst, wobei die Antworten des virtuellen Ausgangswandlers (TV$_{out}$) zu einem Zeitpunkt erfasst werden, der um eine zusätzliche Verzögerung $\delta t$ in Bezug auf einen Zeitpunkt der Antworten des virtuellen Eingangswandlers (TV$_{in}$) verschoben ist,

- einen Schritt der Bestimmung eines Wellenfrontbildes für den virtuellen Eingangswandler (TVin) und für ein zusätzliches Verzögerungsintervall, wobei das Wellenfrontbild als Funktion der Schallgeschwindigkeit $c_0$ in dem Medium bestimmt wird und das Wellenfrontbild bestimmt wird aus:

- der fokussierten Reflexionsmatrix **RFoc($r_{in}$, $r_{out}$**, $\delta$t), und
- einer ballistischen Ausbreitungsbeziehung vom Typ $\delta t(\Delta \boldsymbol{r_{out}}) = -\text{sign}(\Delta z_{out}) \cdot |\Delta \boldsymbol{r_{out}}|/c_0$ die es ermöglicht, Werte aus der Matrix der fokussierten Reflexion zu extrahieren, um das Wellenfrontbild zu konstruieren, und wobei:

$\delta$t die zusätzliche Verzögerung ist,
$|\Delta \boldsymbol{r_{out}}|$ das Modul des Vektors zwischen dem virtuellen Eingangswandler (TV$_{in}$) und dem virtuellen Ausgangswandler (TV$_{out}$) ist,
wobei $\Delta \boldsymbol{r_{out}} = \boldsymbol{r_{out}} - \boldsymbol{r_{in}}$ ist,
$\Delta z_{out}$ t die Komponente entlang einer Z-Tiefenachse des räumlichen Positionsvektors $\Delta \boldsymbol{r_{out}}$ ist,

wobei das Bild der Wellenfront einen Brennfleck umfasst,

- einem Schritt des Bestimmens einer Vorzugsrichtung des Brennflecks im Wellenfrontbild durch Bildverarbeitung des Wellenfrontbildes, wobei die Vorzugsrichtung einer Richtung mit der größten Dimension für den Brennfleck entspricht.

2. Verfahren nach Anspruch 1, wobei die Bildverarbeitung eine Extraktion der Kontur des Brennflecks durch einen Schwellenwert auf ein Niveau unterhalb des Maximalwerts im Wellenfrontbild umfasst, wobei die Kontur dazu dient, die Vorzugsrichtung des Brennflecks zu bestimmen.

3. Verfahren nach Anspruch 1, wobei

- das Wellenfrontbild U($\boldsymbol{r_{in}}$, $\Delta x_{out}$, $\Delta z_{out}$) von einem Bezugssystem mit kartesischen Koordinaten in ein Bezugssystem mit Polarkoordinaten U($\boldsymbol{r_{in}}$, $\Delta s_{out}$, $\Delta \phi_{out}$) transformiert wird,
- die Werte des genannten Wellenfrontbildes des Polarkoordinaten-Bezugssystems über eine Vielzahl von Werten der radialen Entfernungsabweichungen $\Delta s_{out}$ summiert werden, um eine Winkelempfindlichkeitsfunktion f($\boldsymbol{r_{in}}$, $\Delta \Phi_{out}$) für eine Vielzahl von Winkelwerten $\Delta \Phi_{out}$ zu erhalten,
- der optimale Winkelwert $\Delta \Phi^{max}_{out}(\boldsymbol{r_{in}})$ bestimmt wird, der dem Maximum der Winkelempfindlichkeitsfunktion entspricht, wobei der optimale Winkelwert $\Delta \Phi^{max}_{out}(\boldsymbol{r_{in}})$ der Vorzugsrichtung des Brennflecks entspricht, der mit dem virtuellen Eingangswandler (TV$_{in}$) assoziiert ist.

4. Verfahren nach einem der Ansprüche 1 bis 3, wobei die Vorzugsrichtung durch einen Blickwinkel des räumlichen Positionspunktes $\boldsymbol{r_{in}}$ des virtuellen Eingangswandlers (TV$_{in}$), von der Wandleranordnung (10) betrachtet, korrigiert wird.

5. Verfahren nach Anspruch 4, wobei die Vorzugsrichtung durch die folgende Formel korrigiert wird:

$$\gamma_{\text{out}}(\mathbf{r_{in}}) = -2(\Delta \phi^{\,max}_{\text{out}}(\mathbf{r_{in}}) - \hat{\theta}_{\text{out}}(\mathbf{r_{in}}))$$

wobei

$\Delta \Phi^{max}_{out}(\boldsymbol{r_{in}})$ der Wert der Vorzugsrichtung ist,
$\theta_{out}(\boldsymbol{r_{in}})$ die Korrektur des Blickwinkels ist,
$\gamma_{out}(\boldsymbol{r_{in}})$ der Winkelwert der Anisotropie ist.

6. Verfahren nach Anspruch 5, wobei die Korrektur des Blickwinkels ist :

$$\hat{\theta}_{\text{out}}(\mathbf{r_{in}}) = \frac{1}{\sum_{u^-_{\text{out}}}^{u^+_{\text{out}}} \cos(\theta_{\text{out}}(\mathbf{r_{in}}))} \sum_{u^-_{\text{out}}}^{u^+_{\text{out}}} \theta_{\text{out}}(\mathbf{r_{in}}) \cos(\theta_{\text{out}}(\mathbf{r_{in}}))$$

mit

$$\theta_{\text{out}}(\mathbf{r_{in}}) = \operatorname{atan}\left(\frac{u_{out} - x_{in}}{z_{in}}\right)$$

wobei $u_{out}^{\pm}(\boldsymbol{r})$ die Maximal- und Minimalwerte der Wandlerpositionen in der Anordnung sind.

7. Verfahren nach einem der Ansprüche 1 bis 6, wobei:

- die Rolle des/der virtuellen Eingangswandler(s) und des/der virtuellen Ausgangswandler(s) vertauscht wird, um eine Vorzugsrichtung in Bezug auf einen virtuellen Ausgangswandler zu bestimmen, und
- die Vorzugsrichtung in Bezug auf den virtuellen Eingangswandler und die Vorzugsrichtung in Bezug auf den virtuellen Ausgangswandler kombiniert werden, um eine verbesserte Vorzugsrichtung zu erhalten.

8. Verfahren nach einem der Ansprüche 5 bis 6, wobei:

- die Rolle des/der virtuellen Eingangswandler(s) und des/der virtuellen Ausgangswandler(s) vertauscht wird, um einen Winkelwert der Anisotropie in Bezug auf einen virtuellen Ausgangswandler $\gamma_{out}(\boldsymbol{r_{out}})$ zu bestimmen, und
- der Winkelwert der Anisotropie in Bezug auf den virtuellen Eingangswandler $\gamma_{out}(\boldsymbol{r_{in}})$ und der Winkelwert der Anisotropie in Bezug auf den virtuellen Ausgangswandler $\gamma_{out}(\boldsymbol{r_{out}})$ kombiniert werden, um einen verbesserten Winkelwert der Anisotropie zu erhalten.

9. Verfahren nach einem der Ansprüche 1 bis 8, wobei:

- zwischen dem Schritt der Bestimmung eines Wellenfrontbildes und dem Schritt der Bestimmung der Vorzugsrichtung des Brennflecks ein Schritt der Verbesserung des Wellenfrontbildes durchgeführt wird, wobei eine Linearkombination einer Gruppe von Wellenfrontbildern durchgeführt wird, die einem Kohärenzbereich entsprechen, wobei jedes Wellenfrontbild zwischen einem ausgewählten virtuellen Eingangswandler (TV$_{in}$) mit einer unterschiedlichen räumlichen Position $\mathbf{r_{in}}$ und virtuellen Ausgangswandlern (TV$_{out}$) mit einer räumlichen Position $\mathbf{r_{out}}$ aufgenommen wird, so dass $\mathbf{r_{out}} = \Delta\boldsymbol{r_{out}} + \mathbf{r_{in}}$, wobei $\Delta\boldsymbol{r_{out}}$ vordefiniert und für alle Wellenfrontbilder des Satzes gleich ist, und die ausgewählten virtuellen Eingangswandler einander benachbart sind, um ein verbessertes Wellenfrontbild zu erhalten, das einem virtuellen Referenz-Eingangswandler (TV$_{in,ref}$) zugeordnet ist, wobei dieser virtuelle Referenz-Eingangswandler TV$_{in,ref}$ für die virtuellen Eingangswandler des Satzes der verwendeten und dem Kohärenzbereich ZC zugeordneten Wellenfrontbilder charakteristisch ist, und
- in dem Schritt des Bestimmens einer Vorzugsrichtung des Brennflecks das verbesserte Wellenfrontbild anstelle des Wellenfrontbildes verwendet wird, wobei die Vorzugsrichtung des Brennflecks relativ zu der räumlichen Position des virtuellen Referenz-Eingangswandlers TV$_{in,ref}$ ist.

10. Verfahren nach Anspruch 9, wobei die Linearkombination durch eine Singulärwertzerlegungsberechnung (SVD) der Gesamtheit der Wellenfrontbilder bestimmt wird, um einen Singulärvektor (W$_1$) zu erhalten, der dem Singulärwert der Singulärwertzerlegung mit dem größten Absolutwert zugeordnet ist, wobei dieser Singulärvektor **(W$_1$)** dann das verbesserte Wellenfrontbild entsprechend dem virtuellen Referenz-Eingangswandler (TV$_{in,ref}$) und für die gleichen zusätzlichen Verzögerungen $\delta t$ ist.

11. Verfahren nach einem der Ansprüche 1 bis 10, wobei bei dem Schritt der Bestimmung der Matrix der fokussierten Reflexion :

die Berechnung der Antworten des virtuellen Eingangswandlers (TV$_{in}$) einem Eingangs-Fokussierungsprozess aus der experimentellen Reflexionsmatrix **R$_{ui}$**(t) entspricht, der eine Hinlaufzeit der Wellen zwischen der Emissionsbasis und dem virtuellen Eingangswandler (TV$_{in}$) verwendet, um einen Eingangs-Brennfleck an der räumlichen Position $\mathbf{r_{in}}$ zu erzeugen,
die Berechnung der Antworten des virtuellen Ausgangswandlers (TV$_{out}$) einem Ausgangsfokussierungsprozess aus der experimentellen Reflexionsmatrix **R$_{ui}$**(t) entspricht, der eine Rücklaufzeit der Wellen zwischen dem virtuellen Ausgangswandler (TV$_{out}$) und den Wandlern der Rezeptionsbasis **u** verwendet, um einen Ausgangsbrennfleck an der räumlichen Position $\mathbf{r_{out}}$ zu erzeugen,

wobei die zusätzliche Verzögerung $\delta t$ eine zeitliche Verzögerung ist, die bei den Fokussierungsprozessen zu den Hinlauf- und Rücklaufzeiten addiert wird.

12. Verfahren nach einem der Ansprüche 1 bis 11, wobei die fokussierte Reflexionsmatrix nach folgender Formel berechnet wird.

$$\mathbf{RFoc}(\mathbf{r_{in}}, \mathbf{r_{out}}, \delta t) = \frac{1}{N_{in}N_{out}} \sum_{i_{in}} \sum_{u_{out}} \mathbf{R_{ui}}\left(u_{out}, i_{in}, \tau(\mathbf{r_{in}}, \mathbf{r_{out}}, u_{out}, i_{in}, \delta t)\right)$$

wobei

$N_{in}$ die Anzahl der Elemente in der Emissionsbasis (i) ist,

$N_{out}$ die Anzahl der Elemente der ausgebenden Rezeptionsbasis (u) ist,

$\mathbf{R_{ui}}(t)$ die experimentelle Reflexionsmatrix ist, wobei $\mathbf{R_{ui}}(u_{out}, i_{in}, \tau(\mathbf{r_{in}}, \mathbf{r_{out}}, u_{out}, i_{in}, \delta t))$ das Element der experimentellen Reflexionsmatrix $\mathbf{R_{ui}}(t)$ ist, das vom räumlichen Positionswandler infolge der Indexemission $\mathbf{i_{in}}$ in die Emissionsbasis und zur Zeit $\tau$ aufgezeichnet wird,

$\tau$ eine Zeit ist, die die Summe aus der Hinlaufzeit $\tau_{in}$ der Ultraschallwelle zwischen den Wandlern der Emissionsbasis (i) und dem virtuellen Eingangswandler (TV$_{in}$) mit der räumlichen Position $\mathbf{r_{in}}$ und der Rücklaufzeit $\tau_{out}$ der Ultraschallwelle zwischen dem Ausgangswandler (TV$_{out}$) mit der räumlichen Position $\mathbf{r_{out}}$ und den Wandlern der Rezeptionsbasis u sowie der zusätzlichen Verzögerung $\delta t$ ist, wie durch die folgende Formel erläutert:

$$\tau(\mathbf{r_{in}}, \mathbf{r_{out}}, \mathbf{u_{out}}, \mathbf{i_{in}}, \delta t) = \tau_{in}(\mathbf{r_{in}}, \mathbf{i_{in}}) + \tau_{out}(\mathbf{r_{out}}, \mathbf{u_{out}}) + \delta t$$

13. **System (40) zur Ultraschallcharakterisierung eines Mediums (20),** um lokal eine Vorzugsrichtung einer Anisotropie von Diffusoren in dem Medium zu bestimmen, wobei das System Folgendes umfasst:

- eine Wandleranordnung (10), die dazu ausgebildet ist, eine Reihe von Ultraschallwellen zu erzeugen, die in einen Bereich des Mediums einfallen, und die von dem Bereich zurückgestreuten Ultraschallwellen als Funktion der Zeit aufzeichnen; und
- eine Recheneinheit (42), die mit der Wandleranordnung verbunden und dazu ausgebildet ist, das Verfahren nach einem der vorhergehenden Ansprüche auszuführen.

**Claims**

1. **Method for ultrasonic characterization of a medium** in order to locally determine a preferred direction of anisotropy of scatterers in the medium, the method comprising:

- a step of generating a series of incident ultrasonic waves (US$_{in}$) in an area of said medium, by means of an array (10) of transducers (11), said series of incident ultrasonic waves being an emission basis (i); and
- a step of generating an experimental reflection matrix $\mathbf{R_{ui}}(t)$ defined between the emission basis (i) as input and a reception basis (u) as output;
- a step of determining a focused reflection matrix $\mathbf{RFoc}(\mathbf{r_{in}}, \mathbf{r_{out}}, \delta t)$ which comprises responses of the medium between an input virtual transducer (TV$_{in}$) of spatial position $\mathbf{r_{in}}$ and an output virtual transducer (TV$_{out}$) of spatial position $\mathbf{r_{out}}$, the responses of the output virtual transducer (TV$_{out}$) being obtained at a time instant that is shifted by an additional delay δt relative to a time instant of the responses of the input virtual transducer (TV$_{in}$),
- a step of determining a wavefront image for the input virtual transducer (TV$_{in}$) and for an additional delay interval, said wavefront image being determined as a function of the speed of sound $c_0$ in the medium, and said wavefront image being determined based on:

- the focused reflection matrix **RFoc(rin, r$_{out}$, δt)** and
- a ballistic propagation relation of the type

$$\delta t(\Delta \mathbf{r_{out}}) = -sign(\Delta z_{out}) \cdot |\Delta \mathbf{r_{out}}|/c_0,$$

which makes it possible to extract values from the focused reflection matrix in order to construct the wavefront image, and where:

δt is the additional delay,
$|\Delta\mathbf{r_{out}}|$ is the modulus of the vector between the input virtual transducer (TV$_{in}$) and the output virtual transducer (TV$_{out}$), with $\Delta\mathbf{r_{out}} = \mathbf{rout} - \mathbf{r_{in}}$,
$\Delta z_{out}$ is the component along a depth axis Z of the spatial position vector $\Delta\mathbf{r_{out}}$.
said wavefront image comprising a focal spot,

- a step of determining a preferred direction of the focal spot in the wavefront image by image processing said wavefront image, said preferred direction corresponding to a direction of larger dimension for said focal spot.

2. Method according to claim 1, wherein the image processing comprises extracting the outline of the focal spot by a threshold at a level lower than the maximum value in the wavefront image, this outline serving to determine the preferred direction of the focal spot.

3. Method according to claim 1, wherein:

- the wavefront image $U(\mathbf{r_{in}}, \Delta x_{out}, \Delta z_{out})$ is converted from a reference system in Cartesian coordinates to a reference system in polar coordinates $U(\mathbf{r_{in}}, \Delta s_{out}, \Delta\phi_{out})$,
- the values of said wavefront image in the reference system in polar coordinates are summed, over a plurality of radial distance deviation values $\Delta s_{out}$, to obtain an angular sensitivity function $f(r\varepsilon n, \Delta\phi_{out})$ for a plurality of angular values $\Delta\phi_{out}$,
- the optimum angular value $\Delta\phi^{max}_{out}(\mathbf{r_{in}})$ corresponding to the maximum of the angular sensitivity function is determined, said optimum angular value $\Delta\phi^{max}_{out}(\mathbf{r_{in}})$ corresponding to the preferred direction of the focal spot associated with the input virtual transducer (TV$_{in}$).

4. Method according to one of claims 1 to 3, wherein the preferred direction is corrected by an angle of view of the point of spatial position $\mathbf{r_{in}}$ of the input virtual transducer (TVin) as viewed from the array of transducers (10).

5. Method according to claim 4, wherein the preferred direction is corrected by the formula:

$$\gamma_{out}(\mathbf{r_{in}}) = -2(\Delta\phi\,^{max}_{out}(\mathbf{r_{in}}) - \hat{\theta}_{out}(\mathbf{r_{in}}))$$

where:

$\Delta\phi\,^{max}_{out}(\mathbf{r_{in}})$ is the value of the preferred direction,
$\theta_{out}(\mathbf{r_{in}})$ is the correction of the angle of view,
$\gamma_{out}(\mathbf{r_{in}})$ is the angular anisotropy value.

6. Method according to claim 5, wherein the correction of the angle of view is:

$$\hat{\theta}_{out}(\mathbf{r_{in}}) = \frac{1}{\sum_{u^-_{out}}^{u^+_{out}} \cos(\theta_{out}(\mathbf{r_{in}}))} \sum_{u^-_{out}}^{u^+_{out}} \theta_{out}(\mathbf{r_{in}}) \cos(\theta_{out}(\mathbf{r_{in}}))$$

with

$$\theta_{out}(\mathbf{r_{in}}) = atan\left(\frac{u_{out} - x_{in}}{z_{in}}\right)$$

where: $u^{\pm}_{out}(\mathbf{r})$ are the maximum and minimum values of positions of transducers in the array.

**7.** Method according to one of claims 1 to 6, wherein:

- the roles of the input virtual transducer(s) and of the output virtual transducer(s) are reversed in order to determine a preferred direction with respect to an output virtual transducer, and
- the preferred direction with reference to the input virtual transducer and the preferred direction with reference to the output virtual transducer are combined to obtain an improved preferred direction.

**8.** Method according to one of claims 5 to 6, wherein:

- the roles of the input virtual transducer(s) and of the output virtual transducer(s) are reversed in order to determine an angular anisotropy value with respect to an output virtual transducer $\gamma_{out}(\mathbf{r_{out}})$, and
- the angular anisotropy value with reference to the input virtual transducer $\gamma_{out}(\mathbf{r_{in}})$ and the angular anisotropy value with reference to the output virtual transducer $\gamma_{out}(\mathbf{r_{out}})$ are combined to obtain an improved angular anisotropy value.

**9.** Method according to one of claims 1 to 8, wherein:

- between the step of determining a wavefront image and the step of determining the preferred direction of the focal spot, a step of improving the wavefront image is performed in which a linear combination of a set of wavefront images corresponding to a coherence area is carried out, each wavefront image being obtained between a selected input virtual transducer ($TV_{in}$) of a different spatial position $\mathbf{r_{in}}$, and output virtual transducers ($TV_{out}$) of spatial position $\mathbf{r_{out}}$ such that $\mathbf{r_{out}} = \Delta\mathbf{r_{out}} + \mathbf{r_{in}}$, with $\Delta\mathbf{r_{out}}$ being predefined and identical for all wavefront images of the set, and the selected input virtual transducers being close to each other, in order to obtain an improved wavefront image associated with a reference input virtual transducer ($TV_{in,ref}$), this reference input virtual transducer $TV_{in,ref}$ being characteristic of the input virtual transducers of the set of wavefront images used and associated with the chosen coherence area ZC, and
- in the step of determining a preferred direction of the focal spot, the improved wavefront image is used instead of the wavefront image, the preferred direction of the focal spot is relative to the spatial position of the reference input virtual transducer $TV_{in,ref}$.

**10.** Method according to claim 9, wherein the linear combination is determined by calculating the singular value decomposition (SVD) of the set of wavefront images in order to obtain a singular vector (Wi) associated with the singular value of greatest absolute value of the singular value decomposition, this singular vector (Wi) then being the improved wavefront image corresponding to said reference input virtual transducer ($TV_{in,ref}$) and for the same additional delays $\delta t$.

**11.** Method according t one of claims 1 to 10, wherein, in the step of determining the focused reflection matrix:

the calculation of the responses of the input virtual transducer ($TV_{in}$) corresponds to a focusing process at input based on the experimental reflection matrix $\mathbf{R_{ui}}(t)$ which uses an outward time-of-flight of the waves between the emission basis and the input virtual transducer ($TV_{in}$) to create an input focal spot at spatial position $\mathbf{r_{in}}$, the calculation of the responses of the output virtual transducer ($TV_{out}$) corresponds to a focusing process at output based on the experimental reflection matrix $\mathbf{R_{ui}}(t)$ which uses a return time-of-flight of the waves between the output virtual transducer ($TV_{out}$) and the transducers of the reception basis $\mathbf{u}$, to create an output focal spot at spatial position $\mathbf{r_{out}}$, the additional delay $\delta t$ being a time lag added to the outward and return times-of-flight during the focusing processes.

**12.** Method according to one of claims 1 to 11, wherein the focused reflection matrix is calculated by the following formula:

$$\mathbf{RFoc}(\mathbf{r_{in}}, \mathbf{r_{out}}, \delta t) = \frac{1}{N_{in}N_{out}} \sum_{i_{in}} \sum_{u_{out}} \mathbf{R_{ui}}\big(\mathbf{u_{out}}, \mathbf{i_{in}}, \tau(\mathbf{r_{in}}, \mathbf{r_{out}}, \mathbf{u_{out}}, \mathbf{i_{in}}, \delta t)\big)$$

where

$N_{in}$ is the number of elements of the emission basis (i),

$N_{out}$ is the number of elements of the reception basis (u) at output,

$R_{ui}(t)$ is the experimental reflection matrix, in which $R_{ui}(u_{out}, i_{in}, \tau(r_{in}, r_{out}, u_{out}, i_{in}, \delta t))$ is the element of the experimental reflection matrix $R_{ui}(t)$ recorded by the transducer of spatial position $u_{out}$ following the emission of index $i_{in}$ in the emission basis and at time $\tau$,

$\tau$ is a time which is the sum of the outward time-of-flight $\tau_{in}$ of the ultrasonic wave between the transducers of the emission basis **(i)** and the input virtual transducer $(TV_{in})$ of spatial position $r_{in}$, and of the return time-of-flight $\tau_{out}$ of the ultrasonic wave between the output transducer $(TV_{out})$ of spatial position $r_{out}$ and the transducers of the reception basis u, and of the additional delay $\delta t$, as explained by the following formula:

$$\tau(r_{in}, r_{out}, u_{out}, i_{in}, \delta t) = \tau_{in}(r_{in}, i_{in}) + \tau_{out}(r_{out}, u_{out}) + \delta t$$

13. **System (40) for ultrasonic characterization of a medium (20)** in order to locally determine a preferred direction of anisotropy of scatterers in the medium, the system comprising:

- an array (10) of transducers that are suitable for generating a series of incident ultrasonic waves in an area of the medium, and for recording the ultrasonic waves backscattered by said area as a function of time; and
- a calculation unit (42) connected to the array of transducers and suitable for implementing the method according to one of the preceding claims.

FIG. 1A (ART ANTÉRIEUR)

FIG. 1B (ART ANTÉRIEUR)

**FIG. 1C** (ART ANTÉRIEUR)

**FIG. 2** (ART ANTÉRIEUR)

FIG. 3

FIG. 4

FIG. 5

FIG. 6

FIG. 7

A1

$|R(\Delta r = 0, \delta t)|$

I

1

0

-3            0            3

A2            $\delta t$ (µs)

**FIG. 8A**

$|TF[R(\Delta r = 0, \delta t)]|$

J

1

0

3            6            9

S1

S2

$f$ (MHz)

**FIG. 8B**

FIG. 9A

FIG. 9B

FIG. 10

FIG. 11

**FIG. 12**

**FIG. 13**

**FIG. 14**

$$\gamma(\mathbf{r}) = -2(\Delta\phi^{max}(\mathbf{r}) - \hat{\theta}_{in}(\mathbf{r}))$$

**FIG. 15**

FIG. 16

**FIG. 17**

**FIG. 18**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- Reflection matrix approach for quantitative imaging of scattering media. **W. LAMBERT et al.** ARXIV.ORG. CORNELL UNIVERSITY LIBRARY, 04 Juin 2020
- **G. MONTALDO et al.** Cohérent plane-wave compounding for very high frame rate ultrasonography and transient elastography''. *IEEE Trans. Ultrason., Ferroelect. Freq. Control,* 2009, vol. 56, 489-506
- **RAOUL MALLART ; MATHIAS FINK.** The van Cittert-Zernike theorem in pulse écho measurements. *J. Acoust. Soc. Am.,* Novembre 1991, vol. 90 (5
- **ALFONSO RODRIGUEZ-MOLARES et al.** Specular Beamforming''. *IEEE Transactions on Ultrasonics, Ferroelectrics, and Frequency Control,* Septembre 2017, vol. 64 (9